# EUROPEAN PATENT APPLICATION

(11) **EP 4 835 099 A1**
(43) Date of publication of application: **04.11.2026**
(21) Application number: 24911005.7
(22) Date of filing: 23.12.2024
(51) Int. Cl.: C12Q 1/689, C12Q 1/6895, C12Q 1/686, C12Q 1/70, C12N 15/11, C12R 1/01, C12R 1/45, C12R 1/46, C12R 1/445, C12R 1/22, C12R 1/385, C12R 1/19, C12R 1/44, C12R 1/43, C12R 1/21, C12R 1/74, C12R 1/72, C12R 1/725

(54) **COMBINATION FOR DETECTING TARGET GENES AND USE THEREOF**

(30) Priority: 26.12.2023 CN 202311805997; 20.08.2024 CN 202411143306
(71) Applicant: Suzhou Precigenome Ltd, Co, Suzhou City, Jiangsu 215000 (CN)
(72) Inventor: ZHANG, Xuan, Suzhou, Jiangsu 215000 (CN); WANG, Yaqi, Suzhou, Jiangsu 215000 (CN); LING, Yunfeng, Suzhou, Jiangsu 215000 (CN); HE, Jing, Suzhou, Jiangsu 215000 (CN); DONG, Jia, Suzhou, Jiangsu 215000 (CN)
(74) Representative: Lavoix
(86) International application number: PCT/CN2024/141311
(87) International publication number: WO 2025/140077

(57) **Abstract**

Provided is a combination for specifically detecting P target genes. The combination comprises P pairs of primers used for amplifying P genes and P probes used for hybridizing the P genes. The P probes are respectively labelled with M different fluorescent groups having N concentrations; the types or concentrations of fluorescent groups carried by each probe among the P probes are different; and the P target genes and the fluorescent groups meet the following formula: 2≤P≤(M^{N}+M)-X, M being a positive integer greater than or equal to 2, N ranging from 1 to 3, and X ranging from 0 to 19.

## Description

### TECHNICAL FIELD

This application relates to the field of biotechnology, particularly to a combination for detecting target genes and use thereof.

### BACKGROUND ART

Sepsis is a life-threatening organ dysfunction caused by a host dysregulation resulting from infection. It causes an inflammatory response syndrome in the primary infection site and throughout the body, and in severe cases, it can even lead to septic shock. Infections in any part of the body can develop into sepsis. Common sites of infection include: the digestive system (such as the gastrointestinal tract, gallbladder, etc.), lungs, kidneys and other parts of the urinary system, central nervous system, bloodstream, duct-related sites, and sites of severe trauma and burns. Timely and accurate detection of the types of pathogens causing sepsis is essential for early identification, diagnosis, and timely initiation of effective treatment. At the same time, it is of great significance for the accurate use of antibiotics, improving treatment effects, and saving patients' lives.

Traditional detection methods for bacteria and fungi, such as culture, smear microscopy, and antigen detection, generally suffer from low sensitivity and high false negative rates. In addition, the culture method is time-consuming. If traditional culture plus drug sensitivity test is used, it takes an average of about 57.4 hours for diagnosis, which is far from meeting the needs of early diagnosis and treatment of sepsis patients. In addition, the detection rate of culture method is only about 10%, and it is difficult to accurately detect pathogens that are difficult to culture or cannot be cultured.

Methods based on PCR detection after blood culture (such as BioFire's FilmArray^{®} Blood Culture Test and Luminex's Verigene^{®} Blood Culture Test) have shown good performance and have been shown to shorten the time required to optimize antibiotic regimens. Matrix-assisted laser desorption/ionization time-of-flight mass spectrometry (MALDI-TOF) can also be used for bacterial identification after blood culture (such as Biomerieux Bruker's Vitekms Biotyper), showing good concordance (over 90%) for Gram-negative bacteria, but only 80% concordance for Gram-positive bacteria. The above methods still require the pathogen to be cultured for a period of time (more than 4 hours), and cannot provide test results in a shorter time. In addition, these methods rely on bacterial identification after positive blood culture, leaving a gap in the detection of some unculturable pathogens.

Methods that can directly detect pathogens and drug-resistance markers in blood samples by fluorescence quantitative PCR (such as Roche LightCycler^{®} SeptiFast, SeeGene MagicPlex^{®} Sepsis Test, and Abbott Iridica) without prior blood culture have been introduced, but they have not been widely adopted because their sensitivity and specificity do not meet clinical requirements.

Although smear microscopy is quick, the identification of pathogens relies on the doctor's experience and judgment, and it is difficult to distinguish some morphologically similar microorganisms. Antigen testing is convenient and quick, but it is subject to many interfering factors, and false positives and false negatives are difficult to distinguish.

Digital PCR (dPCR) is known as the third generation of PCR technology after conventional PCR and fluorescent PCR. Its principle is to divide a PCR system into multiple smaller units by limiting dilution method, so that each unit comprises at most one target template. The presence or absence of endpoint fluorescence is used as the judgment criterion for each partition after PCR, and then Poisson distribution is used for data analysis to absolutely quantify the target gene in the sample. Digital PCR, with its advantages of simple operation, absolute quantification, high sensitivity, and high specificity, is widely used in various fields such as quantitative detection of rare tumor mutations in in vitro diagnosis, quantitative detection of infectious pathogens, quantitative detection of foodborne pathogens in food safety control, and determination of genetically modified abundance in imported agricultural products by customs inspection and quarantine.

Digital PCR takes only 3-4 hours from extracting nucleic acid from a sample to reading the test results. Its high sensitivity, high specificity, and absolute quantification enable it to accurately identify infectious pathogens and help clinicians make timely early diagnoses. At the same time, its absolute quantification feature helps to accurately monitor the efficacy of treatment, and has great application value in optimizing the types and dosages of drugs. It can be used for the prevention and diagnosis of sepsis. The testing targets comprise high-risk groups for sepsis who need prevention, patients with infections in key areas, patients highly suspected of having sepsis, and patients at all stages of diagnosis of sepsis.

### SUMMARY

The object of this application is to provide PCR primer pairs, probes, microfluidic cartridges, kits, detection systems, and detection methods for specifically detecting target genes. The PCR primer pairs, probes, microfluidic cartridges, kits, detection systems, and detection methods can be used in any PCR detection system, including but not limited to conventional PCR, RL-PCR, RNA-PCR, fluorescence quantitative PCR, digital PCR, PCR-ELISA, nested PCR-high resolution melting analysis (nPCR-HRM), etc., preferably for digital PCR. For example, the object of this application is to provide PCR primer pairs, probes, microfluidic cartridges, kits, detection systems, and detection methods for specifically detecting multiple pathogens in bloodstream infections. The object of this application is to provide PCR primer pairs, probes, microfluidic cartridges, kits, detection systems, and detection methods for specifically detecting a variety of different drug-resistance genes.

To achieve the above objects, this application adopts the following technical solutions:
This application relates to a combination for specifically detecting P target genes, wherein the combination comprises P pairs of primers for amplifying the P genes and P probes for hybridizing the P genes, and the P probes are labeled with N concentrations of M different fluorescent groups, and the types and/or concentrations of fluorescent groups carried by each of the P probes are different; the P target genes and the fluorescent group meet the following formula: 2≤P≤(M^{N+}M)-X, wherein M is a positive integer greater than or equal to 2; N ranges from 1 to 3; and X ranges from 0 to 19.

In one particular embodiment, M is 2, 3, 4, 5, 6, 7 or 8, N is 1, 2 or 3, and X is 0, 1, 2, 3, 4, 5, 6, 7, 8, 9, 10, 11, 12, 13, 14, 15 or 16.

In one particular embodiment, the N concentrations are achieved by using any two different fluorescent groups in a specific ratio, and the ratio of any two different fluorescent groups A and B is in the range of A:B = 1:(0.20-10).

In one particular embodiment, the target gene is either a pathogen gene that causes bloodstream infection or a drug-resistance gene.

In one particular embodiment, the pathogen is one or more pathogens selected from the group consisting of: *Bacteroides fragilis, Staphylococcus epidermidis, Enterococcus faecium, Streptococcus pneumoniae, Acinetobacter baumannii, Enterobacter cloacae, Enterococcus faecalis, Staphylococcus aureus, Klebsiella pneumoniae, Pseudomonas aeruginosa, Escherichia coli, Staphylococcus capitis, Stenotrophomonas maltophilia, Haemophilus influenzae, Serratia marcescens, Candida tropicalis, Cryptococcus neoformans, Candida krusei, Candida parapsilosis, Candida glabrata, Candida albicans,* human herpesvirus 1, human alpha herpesvirus 1, varicella-zoster virus, *Listeria monocytogenes, Mycobacterium tuberculosis,* human cytomegalovirus, human herpesvirus 5, human herpesvirus 2, human herpesvirus 6, Epstein-Barr virus, human herpesvirus 4, parvovirus B19, primate erythrocytic parvovirus 1, human herpesvirus 7, JC virus, JC polyomavirus, human polyomavirus type 2, pseudorabies virus, porcine herpesvirus type 1, pseudorabies virus (porcine herpesvirus 1), *Neisseria meningitidis*, *Streptococcus agalactiae*, *Brucella, Brucella melitensis, Brucella melitensis, Cryptococcus gattii, Cryptococcus gattii, Aspergillus fumigatus*, *Angiostrongylus cantonensis, Taenia solium, Toxoplasma gondii, Balamuthia mandrillaris*, adenovirus subgroup C type 1, adenovirus subgroup B type 4, human mammary gland Adenovirus B, human polyomavirus Type 1, Merkel cell polyomavirus, human polyomavirus 5, torque teno virus, *Streptococcus intermedius*, *Fusobacterium*, *Porphyromonas gingivalis*, *Klebsiella oxytoca, Streptococcus suis, Campylobacter coli, Streptococcus constellatus, Proteus, Salmonella, Prevotella, Rhizopus oryzae, Mucor, Histoplasma capsulatum, Cryptococcus laurentii, Cryptococcus albidus*, *Aspergillus flavus*, *Treponema pallidum*, and *Orientia tsutsugamushi*; or
the drug-resistance gene is any one or more selected from the group consisting of: *mecA*, *mecC*, *vanA, vanB, MCR-1, blaKPC, blaCTX-M, blaOXA-23, blaNDM-1, blaTEM, blaSHV, blaVIM-1, blaVIM-2, blaIMP-1, blaIMP-4, SmeDEF, Sull, QnrA, QnrB, gyrA, gyrB*, *Aac(6')-Ib-cr, ermB, MefA, aac(3')-VI, aph(3 ')-VI, ant(2")-Ia, DfrA1, catA1, TetA, TetB,* and *cfr.*

In one particular embodiment, the 5' end of the sequence of the probe is labeled with a fluorescent reporter group and the 3' end of the sequence of the probe is labeled with a fluorescence quenching group; when the probes are in the same reaction system, the fluorescent reporter groups for screening different types of pathogens or drug-resistance genes have different spectral ranges.

In one particular embodiment, the fluorescent reporter group is selected from FAM, VIC, ROX, Cy5 and/or HEX; and the fluorescence quenching group is selected from BHQ1, BHQ2, BHQ3 and/or Eclipse.

The combination described above for specifically detecting P target genes according to this application is a combination for digital PCR (dPCR) reaction.

This application further relates to a digital PCR reaction premix for specifically detecting a target gene, wherein the digital PCR reaction premix comprises the combination described above for specifically detecting P target genes according to this application.

In one particular embodiment, the digital PCR reaction premix further comprises one, two, three, four, five, six, seven, or eight components selected from the group consisting of: hot-start Taq enzyme, UDG enzyme, dNTPs, bovine serum albumin, glycerol, betaine, NH₄⁺, and Mg²⁺ ions.

This application further relates to a digital PCR microfluidic cartridge, wherein the digital PCR microfluidic cartridge comprises the digital PCR reaction premix for specifically detecting target genes described above according to this application.

In one particular embodiment, the microfluidic cartridge comprises at least one or two wells.

This application further relates to a kit for detecting a target gene, wherein the kit comprises the digital PCR reaction premix for specifically detecting the target gene described above according to this application.

In one particular embodiment, the kit further comprises positive quality control, negative quality control, and/or microdroplet-generating oil.

In one particular embodiment, the kit further comprises the digital PCR microfluidic cartridge described above according to this application.

This application further relates to a method for detecting a target gene for non-disease diagnosis purposes, the method comprises the following steps:
(1) releasing the nucleic acids from the sample to be tested;
(2) preparing a digital PCR amplification mixture, wherein the mixture comprises: the nucleic acids of the sample to be tested in step (1), the combination of the present application for specifically detecting P target genes, and/or the digital PCR premix for specifically detecting target genes of the present application and distilled water;
(3) preparing microdroplets in a digital PCR microfluidic cartridge and performing PCR amplification reaction;
(4) reading the fluorescence signal after the microdroplet PCR amplification reaction by a digital PCR microfluidic cartridge.

In one particular embodiment, the types of samples involved in the detection comprise whole blood, serum, plasma, throat swabs, sputum, urine, feces, cerebrospinal fluid, body fluids, or ascites.

It should be understood that, within the scope of this application, the above-mentioned technical features of this application and the technical features particularly described below can be combined with each other to form new or preferred technical solutions. For brevity, further details are omitted.

The beneficial effect of this application is that it provides sets of primers and probes for detecting multiple target genes, wherein the 5' end of the probe sequence of this application is labeled with fluorescent groups of different colors and concentrations, and the target sequence of each target gene is efficiently amplified by digital PCR, so as to achieve absolute quantification of each target gene to be detected, and directly give the copy number per microliter (CFU/µL) of each target gene in the reaction system. In addition, this application further provides a method for detecting target genes for non-disease diagnosis purposes by using the sets of primers and probes according to the method of this application. For example, the combination according to this application can be used to detect a variety of pathogens in bloodstream infections. The sensitivity and specificity of the method according to this application are 92.88% and 100%, respectively, which exceed the prior art. Moreover, the limit of detection is 500 cps/ml, which is far lower than the limit of detection of bloodstream infection samples by existing qPCR technology.

Particularly, 1) the digital PCR detection kit is used to directly detect nucleic acids of the pathogenic microbes in 2-10 mL of human peripheral whole blood without requiring a 7-8 h blood culture enrichment step; 2) the primer-probe set fluorescence mixed colors encoding technology for digital PCR enables single-well detection of 11-300 pathogens; 3) the detection targets include, but are not limited to: Gram-positive bacteria, Gram-negative bacteria, fungi, viruses, yeasts, and drug-resistance genes; 4) the detection kit comprises an internal reference gene to prevent false negatives caused by insufficient mass of sample extraction or PCR amplification failure.

### BRIEF DESCRIPTION OF THE DRAWINGS

To more clearly illustrate the technical solutions in the examples of this application or the prior art, the accompanying drawings used in the description of the examples or the prior art will be briefly introduced below.
Figs. 1A and 1B are schematic diagrams of the detection results of simultaneous 10-fold digital PCR amplification in one well. Numbers 1-10 represent the 10 pathogens to be tested. When multiple infections are present in a bloodstream infection sample, corresponding positive droplets will appear at double, triple, and quadruple positions.

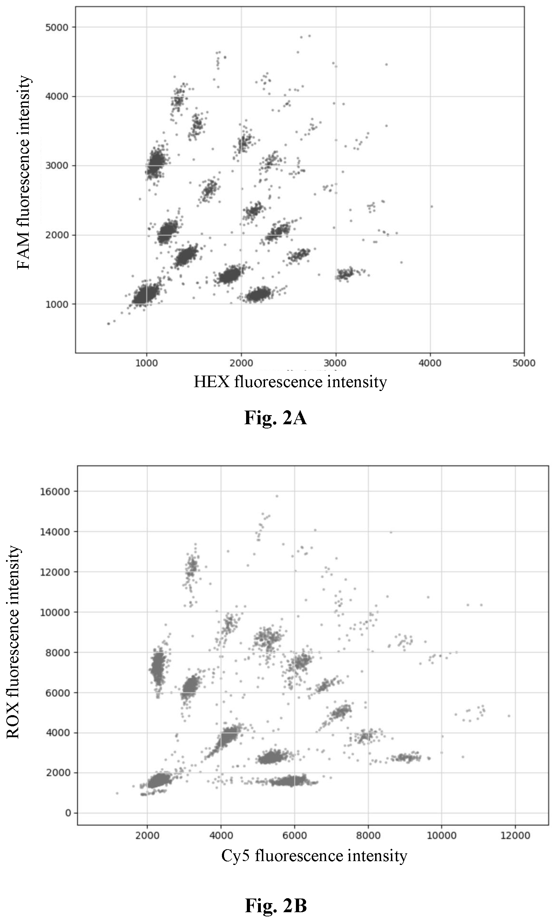

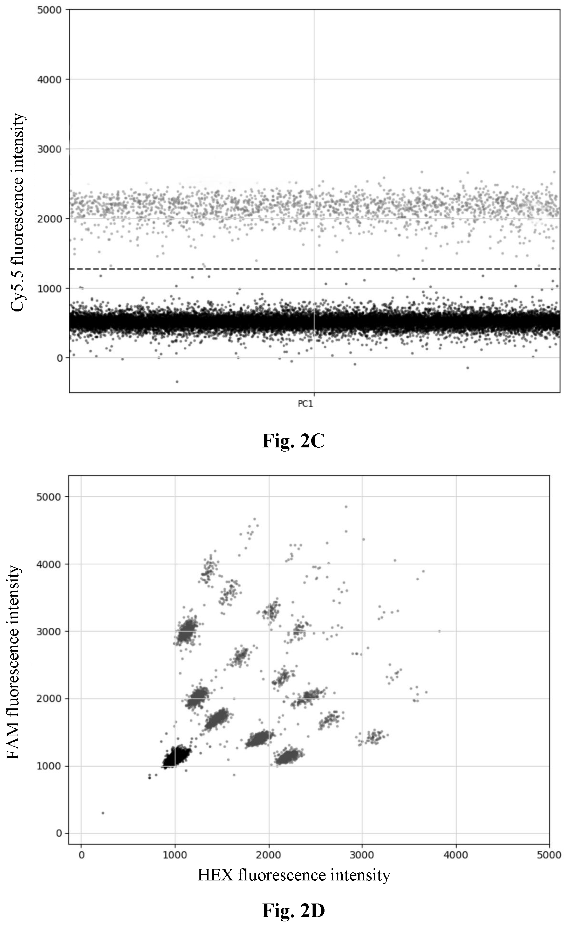

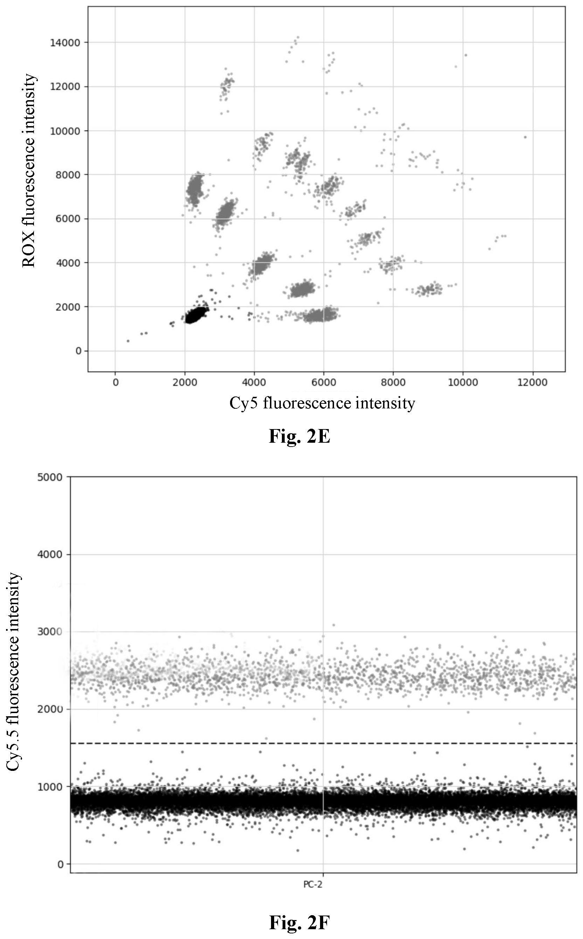

Figs. 2A-2F show the results of simultaneous detection of 21 pathogens plus one internal reference gene in two wells by using the primer-probe sets of this application in Example 1. Fig. 2A is a two-dimensional graph of the positive control FAM & HEX in well 1; Fig. 2B is a two-dimensional graph of the positive control ROX & Cy5 in well 1; Fig. 2C is a one-dimensional graph of the positive control Cy5.5 in well 1; Fig. 2D is a two-dimensional graph of the positive control FAM & HEX in well 2; Fig. 2E is a two-dimensional graph of the positive control ROX & Cy5 in well 2; Fig. 2F is a one-dimensional graph of the positive control Cy5.5 in well 1. Note: In this design, since the Cy5.5 fluorescence channel does not form a two-dimensional graph with other channels, only its one-dimensional graph is shown. The horizontal axis represents the droplet number that is not shown, and the vertical axis represents the fluorescence intensity.

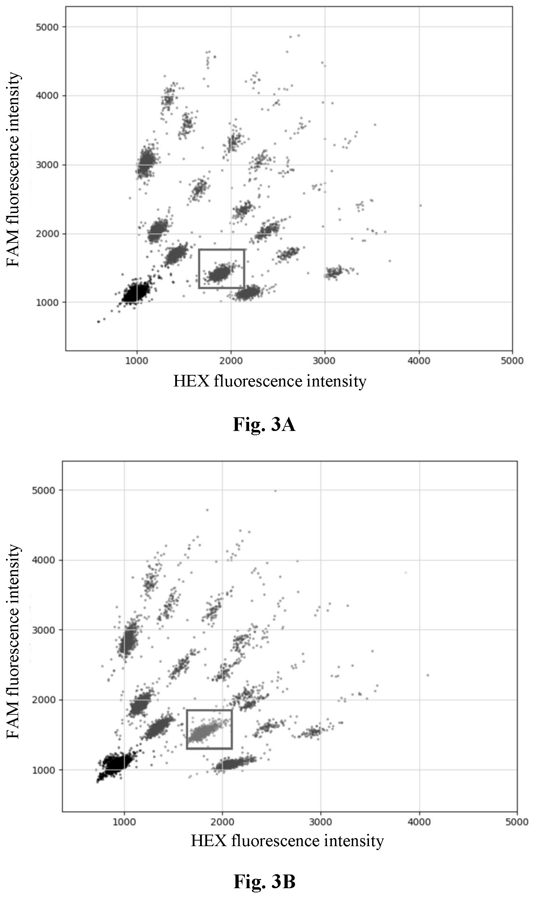

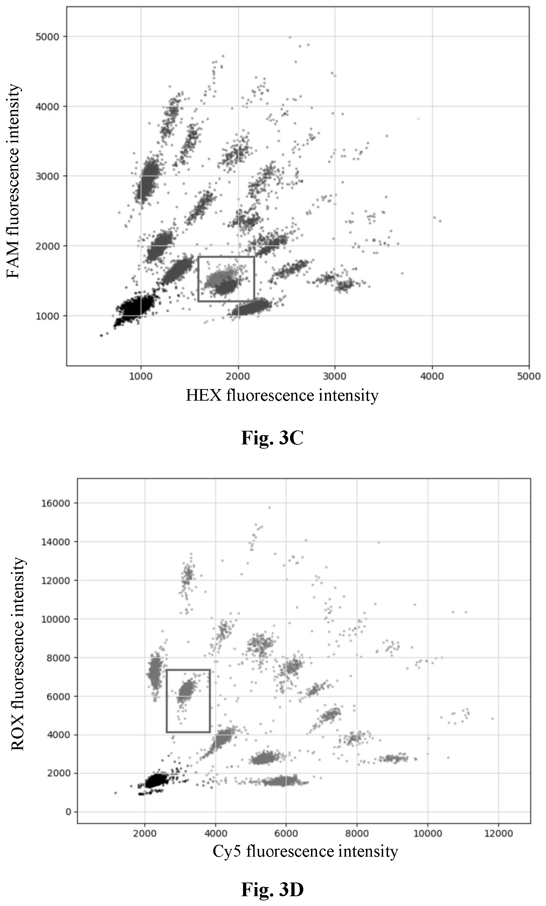

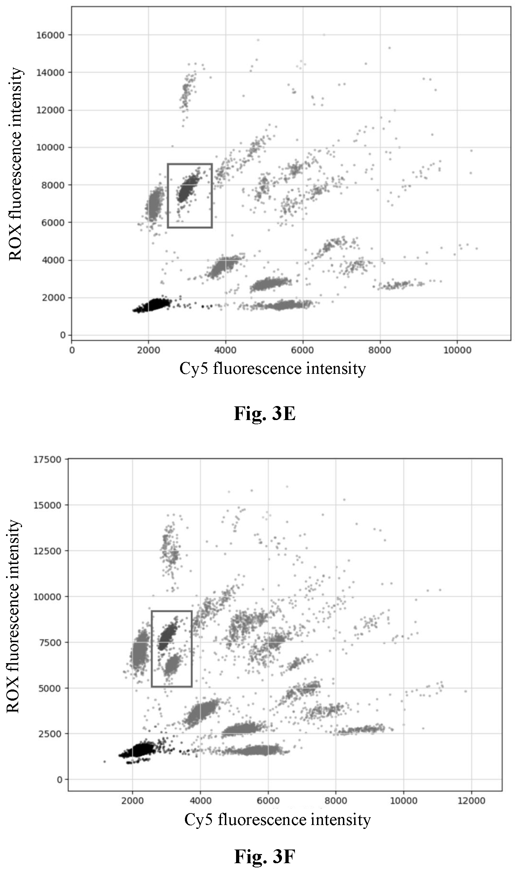

Figs. 3A-3F are examples of moving the position of the two-dimensional graph of positive droplet clusters by adjusting the proportion of different color probes, which are used to illustrate the principle of achieving ultra-multiple target detection by fluorescence mixed colors encoding of different colors. Fig. 3A shows the droplet cluster indicated by the box with a FAM:HEX ratio of 1:3; Fig. 3B shows the droplet cluster indicated by the box with a FAM:HEX ratio of 1.8:3; Fig. 3C shows the superimposed diagram of Figs. 3A and 3B, which can be used to compare the movement of the droplet clusters; Fig. 3D shows the droplet cluster indicated by the box with a ROX:Cy5 ratio of 4:1; Fig. 3E shows the droplet cluster indicated by the box with a ROX:Cy5 ratio of 5.6:1; Fig. 3F shows the superimposed diagram of Figs. 3D and 3E, which can be used to compare the movement of the droplet clusters.
Fig. 4 shows the detection limit range results of 21 bacterial suspension samples by using the kit according to this application (*Staphylococcus aureus* is shown as an example).
Fig. 5 shows the results of the specificity test. The kit for detecting 21 pathogens was reacted with 115 non-target bacteria in 3 replicate wells, and no positive droplet clusters were produced in any of the 3 wells. The results show that the kit for detecting 21 pathogens does not exhibit non-specific cross-reactions with 115 non-target bacteria.
Figs. 6A-6C show the detection results of a real pathogen-positive sample by using the kit according to this application.
Fig. 7 shows the detection results of *Staphylococcus epidermidis* in sputum samples of patients by using the kit according to this application.
Fig. 8 shows the detection results of cerebrospinal fluid samples of patients by using the kit according to this application.
Figs. 9A-9D show the detection results of *Acinetobacter baumannii, Stenotrophomonas maltophilia, Enterobacter cloacae,* and *Candida albicans* in sputum samples of patients by using the kit according to this application.
Figs. 10A-10D show the detection results of throat swab samples of patients by using the kit according to this application; Figs. 10E-10H show the detection results of anal swab samples of patients by using the kit according to this application.
Fig. 11 shows the detection results of urine samples of patients by using the kit according to this application.
Figs. 12A-12I show the results of simultaneous detection of 32 drug-resistance genes plus 1 internal reference gene in 3 wells by using the primer-probe set according to this application in Example 1.

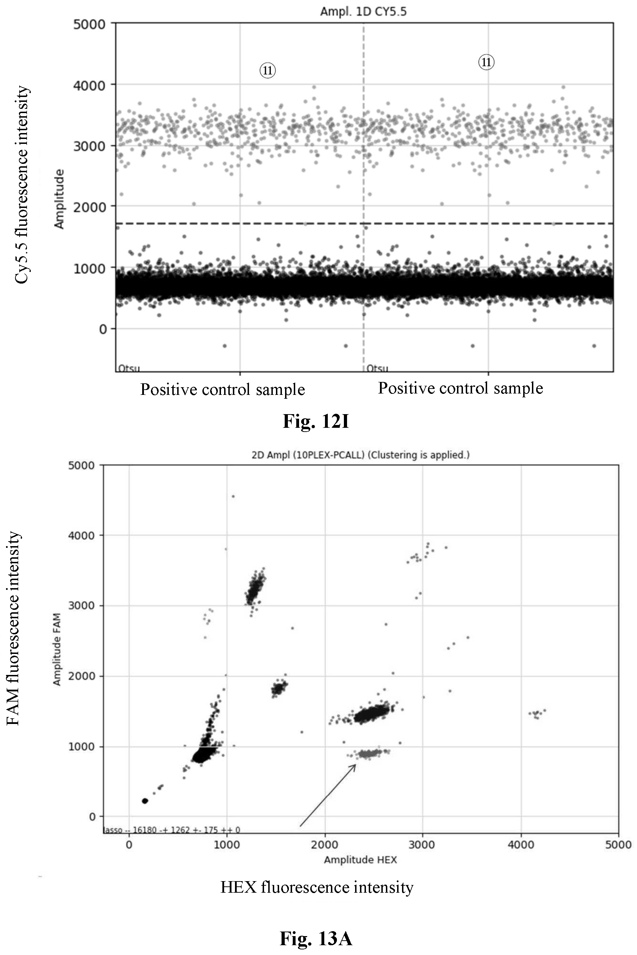

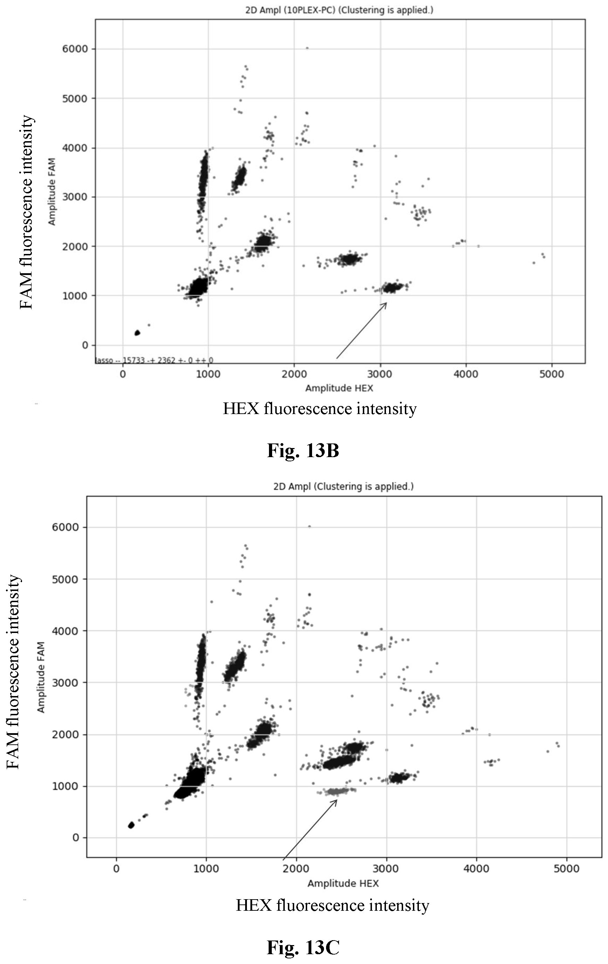

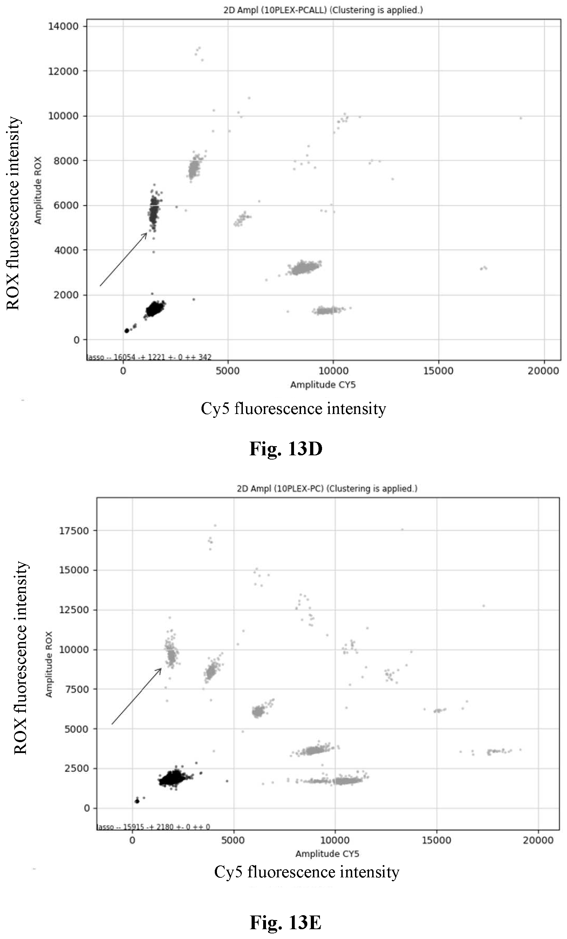

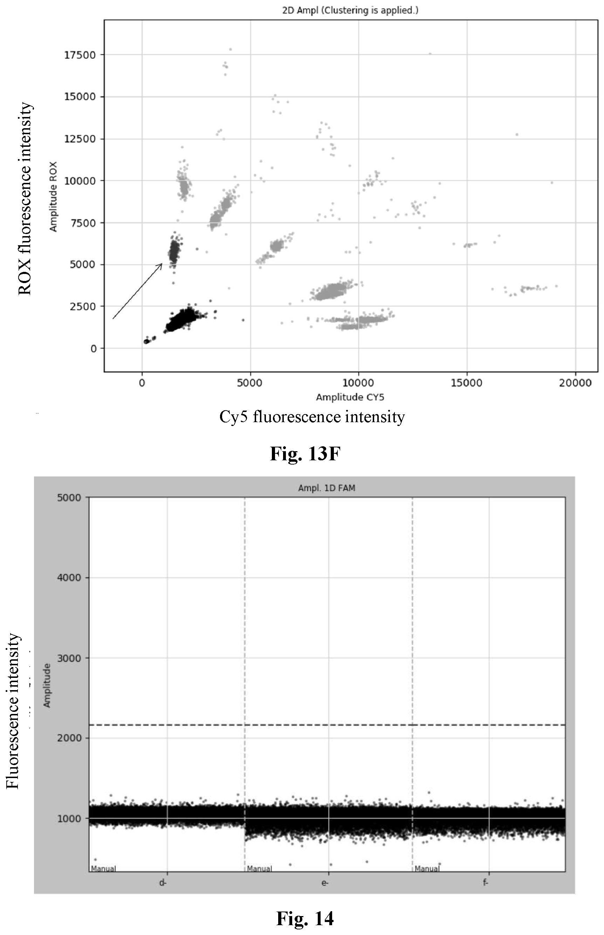

Figs. 13A-13F are examples of moving the position of the two-dimensional graph of positive droplet clusters by adjusting the proportion of probes with different color, which are used to illustrate the principle of achieving ultra-multiple target detection by fluorescence mixed colors coding of different colors. Fig. 13A shows the droplet cluster indicated by the arrow with a FAM:HEX ratio of 1:2.5, Fig. 13B shows the droplet cluster indicated by the arrow with a FAM:HEX ratio of 1:3.2, and Fig. 13C shows the superposition of *mecA* resistance gene positive droplets with a FAM:HEX probe concentration of 1:2.5 and a *mecA* resistance gene positive droplet cluster with a FAM:HEX probe concentration of 1:3.2. It can be clearly seen that by adjusting the concentration ratio of the two probes, FAM and HEX, the position of the positive droplet cluster in the 2D graph of digital PCR detection can be moved. Fig. 13D shows the droplet cluster indicated by the arrow with a ROX:Cy5 ratio of 4:1, Fig. 13E shows the droplet cluster indicated by the arrow with a ROX:Cy5 ratio of 5.5:1, and Fig. 13F shows the superposition of two positive droplets with different ROX:Cy5 ratios. This illustrates that by adjusting the ROX:Cy5 ratio, the position of the *mecC* drug-resistance gene positive droplet cluster in the 2D graph of digital PCR detection can be moved.
Fig. 14 shows the results of the specificity test. The kit for detecting 32 drug-resistance genes was reacted with 115 strains without carrying drug-resistance genes. The reaction was performed in 3 replicate wells, and no positive droplet clusters were generated in any of the 3 wells. It is demonstrated that the kit for detecting 32 drug-resistance genes shows no nonspecific cross-reaction with 115 strains without carrying drug-resistance genes.

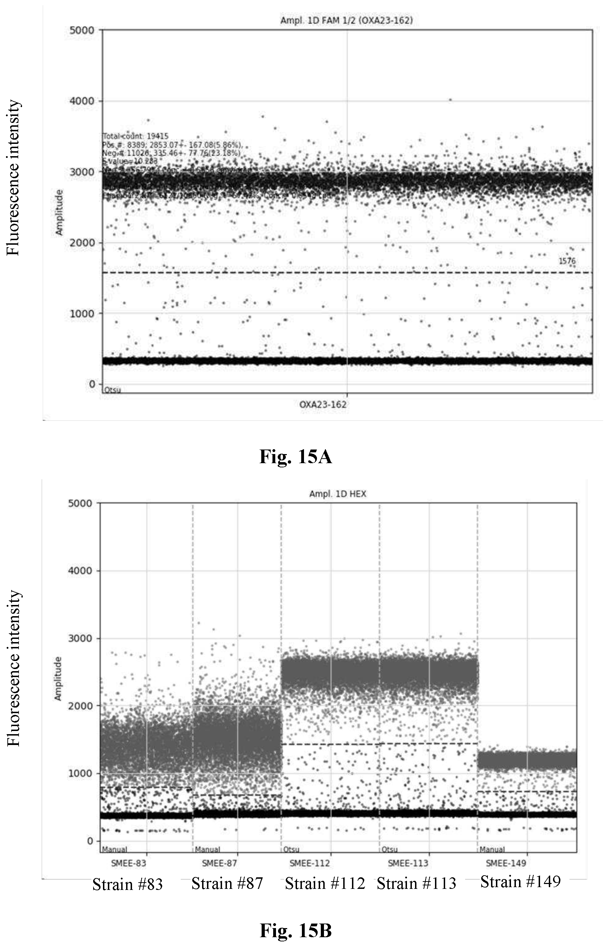

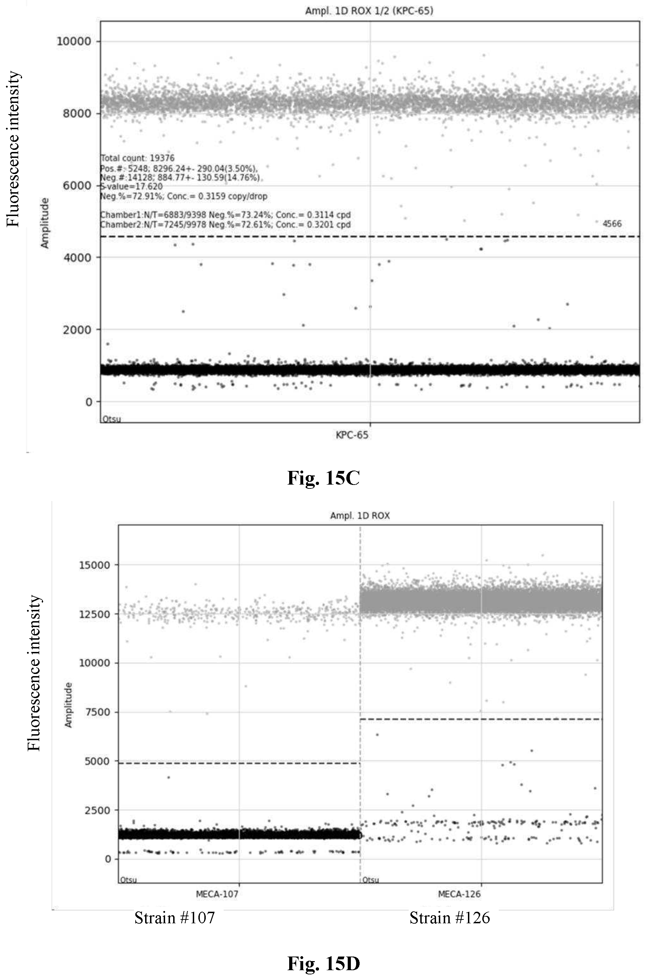

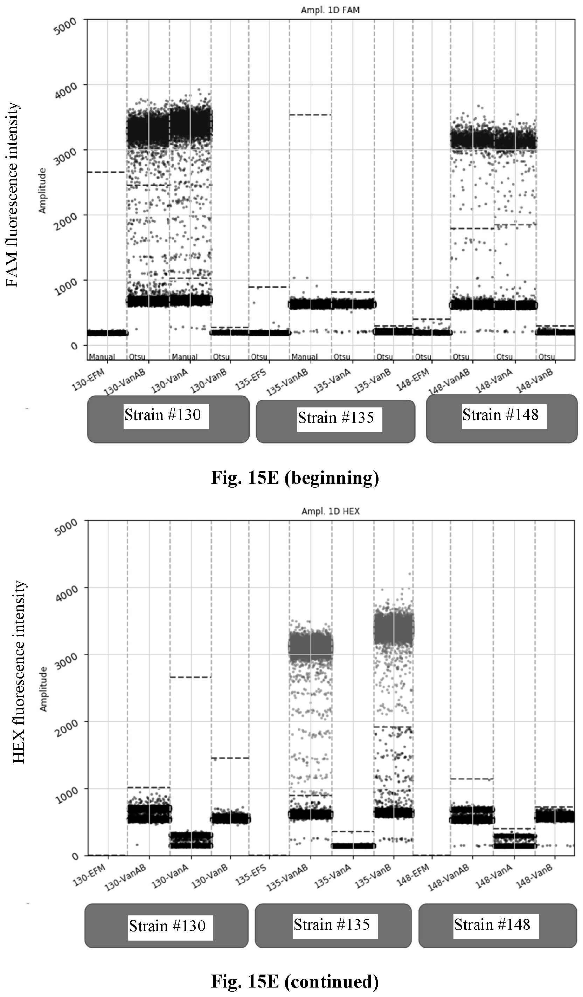

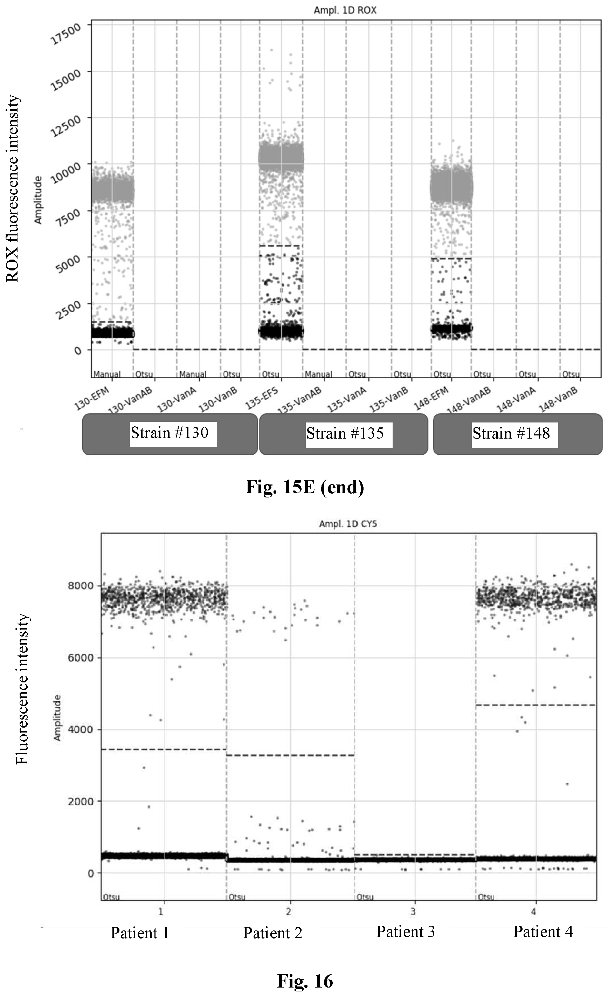

Figs. 15A-15E show the detection results of samples of strains carrying drug-resistance genes by using the kit according to this application. Fig. 15A shows the detection of the *blaOXA-48* resistance gene in *Escherichia coli* strains carrying resistance genes; Fig. 15B shows the detection of the *SmeDEF* resistance gene in 5 strains of *Stenotrophomonas maltophilia* carrying resistance genes; Fig. 15C shows the detection of the *SmeDEF* resistance gene in *Klebsiella pneumoniae* carrying resistance genes; Fig. 15D shows the detection of the *mecA* resistance gene in 2 strains of *Staphylococcus aureus* carrying resistance genes; Fig. 15E shows the detection of *vanA* or *vanB* resistance genes in 3 strains carrying *VanA* and *blaCTX-M* genes.
Fig. 16 shows the detection results of sputum samples of patients by using the kit according to this application.

### DETAILED DESCRIPTION

The particular embodiments of this application are described in detail below, but it should be understood that these examples are only for illustrating this application and are not intended to limit the scope of this application. For the experimental methods in the examples below that do not specify specific experimental conditions, they are usually performed under standard conditions or as recommended by the manufacturer.

Unless otherwise expressly stated, throughout the specification and claims, the term "comprise/include" or its variations such as "comprising/including" or "comprises/includes" shall be understood to comprise the stated ingredients or components without excluding other ingredients or other components.

Digital PCR (dPCR) is a novel absolute quantitative PCR technique that involves limiting dilution of PCR reactants, followed by PCR amplification in different reaction chambers, and finally determining the initial copy number or concentration of the target molecule based on the Poisson distribution principle and the number and proportion of positive droplets. Because multiplex digital PCR reaction systems comprise multiple primers and probes, the requirements for primer-probe combinations in the detection system are very high.

To date, there have been no reports on multiplex PCR techniques for the efficient detection of various mixed nucleic acid samples by digital PCR methods.

In some embodiments, P pairs of primers are used to amplify P types of genes, and P probes are used to hybridize the P types of genes. The P probes are labeled with N concentrations of M different fluorescent groups. The type or concentration of the fluorescent group carried by each of the P probes is different. The P target genes and the fluorescent groups meet the following formula: 2≤P≤(M^{N} +M)-X, wherein M is a positive integer greater than or equal to 2; N ranges from 1 to 3; and X ranges from 0 to 19.

Preferably, M is any value of 2, 3, 4, 5, 6, 7 or 8; more preferably, M is 2 or 3.

N can be 1, 2 or 3.

X can be 0, 1, 2, 3, 4, 5, 6, 7, 8, 9, 10, 11, 12, 13, 14, 15, 16, 17, 18 or 19. Preferably, X is 0, 1, 2, 3, 4, 5, 6, 7, 8, 9, 10, 11, 12, 13, 14, 15 or 16. More preferably, X is 0, 1, 2, 3, 4, 5, 6, 7, 8, 9 or 10.

Particularly, the ratio range of any two fluorescent groups A and B is 1:(0.20-10), for example, 1:0.2, 1:0.25, 1:0.3, 1:0.33, 1:0.4, 1:0.75, 1:1, 1:1.2, 1:1.4, 1:1.6, 1:1.7, 1:1.8, 1:2, 1:2.2, 1:2.4, 1:2.5, 1:2.6, 1:2.8, 1:3, 1:3.2, 1:3.4, 1:3.6, 1:3.8, 1:4, 1:4.2, 1:4.4, 1:4.6, 1:4.8, 1:5, 1:5.2, 1:5.4, 1:5.5, 1:5.6, 1:5.8, 1:6, 1:6.2, 1:6.4, 1:6.6, 1:6.8, 1:7, 1:7.2, 1:7.4, 1:7.6, 1:7.8, 1:8, 1:8.2, 1:8.4, 1:8.6, 1:8.8, 1:9, 1:9.2, 1:9.4, 1:9.6, 1:9.8, or 1:10, etc.

Preferably, the ratio of any two fluorescent groups A and B is 1:0.25, 1:0.33, 1:0.75, 1:1, 1:2, 1:1.4, 1:1.6, 1:1.7, 1:1.8, 1:2, 1:2.2, 1:2.4, 1:2.6, 1:2.8, 1:3, 1:3.2, 1:3.4, 1:3.6, 1:3.8, 1:4, 1:4.2, 1:4.4, 1:4.6, 1:4.8, 1:5, 1:5.2, 1:5.4, 1:5.6, 1:5.8, or 1:6.

In this application, A and B represent any fluorescent group. For example, A can represent any one of FAM, VIC, ROX, Cy5, Cy5.5 and/or HEX, and B represents a fluorescent group different from A, which can also be any one of FAM, VIC, ROX, Cy5, Cy5.5 and/or HEX.

More preferably, the ratio of the two fluorescent groups is 1:0.2, 1:0.25, 1:0.3, 1:0.33, 1:0.4, 1:0.75, 1:1, 1:1.7, 1:2, 1:2.5, 1:3, 1:3.2, 1:4, or 1:5.5.

In one embodiment of this application, when M is 4, N is 2, and X is 8, the P of the identified pathogens is 12.

In one embodiment of this application, when M is 5, N is 2, and X is 8, the P of the identified pathogens is 22.

In another embodiment of this application, when M is 6, N is 2, and X is 8, the P of the identified pathogens is 34.

In one particular embodiment, P probes are labeled by using N concentrations of M different fluorescent groups. The difference in the types or concentrations of fluorescent groups carried by each of the P probes means that each of the P probes carries different types of fluorescent groups, or different concentrations of fluorescent groups, or that each of the P probes carries different types or concentrations of fluorescent groups. Those skilled in the art will understand that the core of this application lies in the need to distinguish each probe by using different types of fluorescent groups or fluorescence intensities, as long as the P probes can be distinguished and detected by detection methods.

In one particular embodiment, two fluorescent groups, A and B, are labeled on a first probe at a ratio of 1:0.33, on a second probe at a ratio of 1:0.75, on a third probe at a ratio of 1:1, on a fourth probe at a ratio of 1:2, and on a fifth probe at a ratio of 1:4. By adjusting the ratio of two fluorescent groups, A and B, the detection of different pathogens can be achieved.

For example, as shown in Example 2 of this application, by adjusting the ratio of fluorescent probes FAM and HEX and the ratio of fluorescent probes ROX and Cy5, positive droplet clusters at different positions can be detected. Adjusting the ratio of FAM probe to HEX probe from 1:3 to 1.8:3, and the ratio of ROX probe to Cy5 probe from 4:1 to 5.6:1, can both achieve the identification of positive droplet clusters at different positions.

In some embodiments, the target gene can be any type of gene, including but not limited to animal genes, plant genes, and/or microbial genes.

Preferably, the microbial genes comprise pathogen microbial genes. The pathogenic microorganisms comprise bacteria, fungi, viruses, spirochetes, mycoplasma, rickettsia, chlamydia, and parasites (protozoa, worms, medical insects), etc.

In some embodiments, the target genes are a variety of different drug-resistance genes.

Preferably, the drug-resistance genes comprise drug-resistance genes of 32 pathogenic microorganisms.

The combination of this application can effectively control the number of multiple probes and utilize various concentrations of multiple probes to specifically and efficiently detect drug-resistance genes of multiple pathogens.

In one embodiment of this application, the above-mentioned primer-probe set is used to achieve simultaneous detection of 11 pathogens in one well with 5 fluorescence colors, and simultaneous detection of 21 pathogens and internal reference in two wells with 5 fluorescence colors by digital PCR, covering the most common pathogens in clinical bloodstream infections. One well with 7 fluorescence colors can simultaneously detect 16 targets, and 3 fluorescence mixed colors coding is used to achieve the effect of detecting more than 30 targets in a single well.

In one embodiment of this application, the above-mentioned primer-probe set is used to achieve simultaneous detection of 11 drug-resistance genes in one well with 5 fluorescence colors and simultaneous detection of 32 drug-resistance genes and internal reference in 3 wells with 5 fluorescence colors by digital PCR, covering the most common drug-resistance genes in clinical bacterial infections.

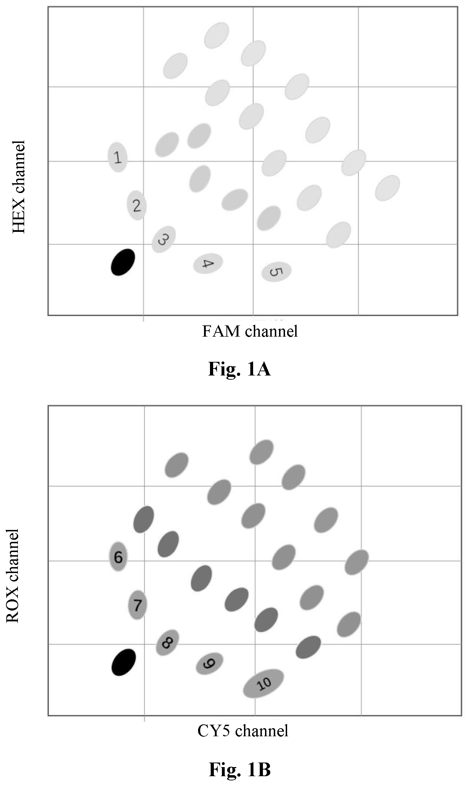

In some embodiments, this application identifies multiple target genes by the relative positional relationships of negative clusters, single positive clusters, double positive clusters, and diversity clusters. The definitions and classifications of negative clusters, single positive clusters, double positive clusters, and diverse clusters are determined as follows: the position of each droplet cluster in the two-dimensional graph is determined by two factors: ①the type and combination of fluorescence in the droplet cluster; ②the intensity of fluorescence in the droplet cluster. (a) Negative cluster: a cluster of droplets that does not comprise any target DNA that can be detected by primers or probes, i.e., a negative droplet cluster. In this cluster, the primers or probes do not undergo amplification, and do not produce a strong positive fluorescence signal. Only a background signal intensity is emitted (the intensity detected by each fluorescence channel is not 0). Therefore, this cluster is usually the cluster with the weakest fluorescence intensity. In the two-dimensional graphs generated by multiplex PCR positive controls (as shown in Figs. 1A-1B), the negative clusters are usually located in the bottom left corner of the two-dimensional graph. In addition, another method to determine the position of the negative cluster is: to mark its position by the unique droplet cluster (i.e., the negative cluster) produced by the negative control reaction; (b) single positive cluster: this droplet cluster comprises only a single type of target DNA, so it can only trigger the corresponding primers and probes to react. By controlling the type of fluorescein labeled on the probes and the probe concentrations, the positive cluster can be placed in the expected position of the two-dimensional graph, that is, it meets the expected (X, Y) coordinate values. In Figs. 1A and 1B, the distance between the five single positive clusters and the negative clusters is the shortest compared to the other clusters (referring to double positive clusters and multiple positive clusters). Each single positive cluster has a different (X, Y) value because of the different types (and combinations) and concentrations of fluorescein labeled by its corresponding probes. However, the position of a single positive cluster induced by a specific type of DNA is fixed; (c) double positive cluster: a cluster consisting of droplets comprising two specified target DNAs after reaction. Within these droplets, each of the two target DNAs may trigger a reaction of its corresponding primers and probes, thereby activating the corresponding fluorescein to emit the expected intensity. Therefore, the fluorescence of this droplet cluster is actually a superposition of the fluorescence produced by the two target DNAs under the background fluorescence (i.e., the intensity of the negative cluster). If we start from the negative cluster and end with the single positive cluster of target 1, we can draw a vector pointing to the single positive cluster of target 1. Similarly, we can draw a vector arrow from the negative cluster to target 2. By using the "parallelogram rule" or "triangle rule" of vector composition, we can add vectors 1 and 2 together to obtain the coordinates of the double positive cluster. If the coordinates of the single positive cluster of target 1 are (X1, Y1), the coordinates of the single positive cluster of target 2 are (X2, Y2), and the negative cluster is (X0, Y0), then the coordinates of the double positive cluster comprising targets 1 and 2 are ((X1-X0)+(X2-X0)+X0), ((Y1-Y0)+(Y2-Y0)+Y0)), that is, (X1+X2-X0, Y1+Y2-Y0). Therefore, within a two-dimensional graph, for any two target DNAs, their negative clusters, two single positive clusters, and corresponding double positive clusters must be located at the four vertices of the parallelogram; (d) triple positive clusters and multiple positive clusters: droplet clusters consisting of droplets comprising three or more specified target DNAs after reaction. The coordinates of these clusters also conform to the principle of vector composition. For example, a three-positive cluster comprising targets 1, 2, and 3 has coordinates (X1+X2+X3-2X0, Y1+Y2+Y3-2Y0). The coordinates of the multiple (n) positive clusters are (X1+X2+...... Xn-(n-1)X0, Y1+Y2+......Yn-(n-1)Y0).

As described above, this application controls the concentration ratio of different fluorescent reporter genes by designing and using different numbers of fluorescent reporter genes, that is, the allocation of N concentrations can achieve a reasonable configuration of negative clusters, single positive clusters, double positive clusters, and diverse clusters, thereby effectively realizing the rapid detection of multiple target genes simultaneously.

The primers and probes of this application can be applied to any PCR detection system for detecting target genes, including but not limited to: conventional PCR, RL-PCR, RNA-PCR, fluorescence quantitative PCR, digital PCR, PCR-ELISA, nested PCR-HRM, etc. Preferably, the primers and probes involved in this application are used for fluorescence quantitative PCR or digital PCR. More preferably, the primers and probes identified in this application are used for digital PCR.

The digital PCR reaction premix in this application comprises primers, probes, enzymes (hot-start Taq enzyme and/or UDG enzyme), dNTPs, glycerol, betaine, NH₄⁺, Mg²⁺, bovine serum albumin, etc.

Compared with the digital PCR reaction premix, the digital PCR amplification mixture in this application further comprises an amplification template (such as a sample template, positive control, or negative control) and distilled water.

In some embodiments, this application provides a digital PCR reaction premix for detecting a target gene, the PCR reaction premix comprises the primer pairs described above, any combination of the primer pairs and probes described above, or a combination of the primer pairs and any probes described above.

In one particular embodiment, this application targets specific nucleotide sequences of the following 21 pathogens: *Bacteroides fragilis*, *Staphylococcus epidermidis*, *Enterococcus faecium*, *Streptococcus pneumoniae, Acinetobacter baumannii, Enterobacter cloacae, Enterococcus faecalis, Staphylococcus aureus, Klebsiella pneumoniae, Pseudomonas aeruginosa, Escherichia coli, Staphylococcus capitis, Stenotrophomonas maltophilia, Serratia marcescens, Haemophilus influenzae, Candida tropicalis, Cryptococcus neoformans*, *Candida krusei, Candida parapsilosis, Candida glabrata,* and *Candida albicans*; through comparison and analysis of multiple sequences, the primers and probes of multiplex digital PCR for detecting the above pathogens are designed, and up to 11-12 multiplex digital PCR can be detected in one well by using different concentrations of probes. In addition, internal reference primers and internal reference probes are designed to monitor the sample collection and extraction process, thereby avoiding false negative results.

In one particular embodiment, this application targets specific nucleotide sequences of the follosing 32 drug-resistance genes: *mecA*, *mecC*, *vanA*, *vanB*, *MCR-1*, *blaKPC*, *blaCTX-M*, *blaOXA-23, blaNDM-1, blaTEM, blaSHV, blaVIM-1, blaVIM-2, blaIMP-1, blaIMP-4, SmeDEF, Sull, QnrA, QnrB, gyrA, gyrB*, *Aac(6')-Ib-cr, ermB, MefA, aac(3')-VI, aph(3')-VI, ant(2")-Ia*, *DfrA1, catA1, TetA, TetB,* and *cfr*; through comparison and analysis of multiple sequences, the primers and probes of multiplex digital PCR for detecting the above drug-resistance genes are designed, and up to 11-12 multiplex digital PCR can be detected in one well by using different concentrations of probes. In addition, internal reference primers and internal reference probes are designed to monitor the sample collection and extraction process, thereby avoiding false negative results.

To improve the efficiency of screening for bloodstream infection pathogens, in one aspect, this application provides a method for efficiently screening bloodstream infection pathogens by PCR. Particularly, as for *Bacteroides fragilis*, an example of the upstream primer is represented by SEQ ID NO: 1, and an example of the downstream primer is represented by SEQ ID NO: 2; as for *Staphylococcus epidermidis,* an example of the upstream primer is represented by SEQ ID NO: 4, and an example of the downstream primer is represented by SEQ ID NO: 5; as for *Enterococcus faecalis*, an example of the upstream primer is represented by SEQ ID NO: 7, and an example of the downstream primer is represented by SEQ ID NO: 8; as for *Streptococcus pneumoniae*, an example of the upstream primer is represented by SEQ ID NO: 10, and an example of the downstream primer is represented by SEQ ID NO: 11; as for *Acinetobacter baumannii,* an example of the upstream primer is represented by SEQ ID NO: 13, and an example of the downstream primer is represented by SEQ ID NO: 14; as for *Enterobacter cloacae*, an example of the upstream primer is represented by SEQ ID NO: 16, and an example of the downstream primer is represented by SEQ ID NO: 17; as for *Enterococcus faecium*, an example of the upstream primer is represented by SEQ ID NO: 19, and an example of the downstream primer is represented by SEQ ID NO: 20; as for *Staphylococcus aureus,* an example of the upstream primer is represented by SEQ ID NO: 22, and an example of the downstream primer is represented by SEQ ID NO: 23; as for *Klebsiella pneumoniae,* an example of the upstream primer is represented by SEQ ID NO: 25, and an example of the downstream primer is represented by SEQ ID NO: 26; as for *Pseudomonas aeruginosa,* an example of the upstream primer is represented by SEQ ID NO: 28, and an example of the downstream primer is represented by SEQ ID NO: 29; as for *Escherichia coli,* an example of the upstream primer is represented by SEQ ID NO: 31, and an example of the downstream primer is represented by SEQ ID NO: 32; as for *Staphylococcus capitis,* an example of the upstream primer is represented by SEQ ID NO: 34, and an example of the downstream primer is represented by SEQ ID NO: 35; as for *Stenotrophomonas maltophilia,* an example of the upstream primer is represented by SEQ ID NO: 37, and an example of the downstream primer is represented by SEQ ID NO: 38; as for *Haemophilus influenzae,* an example of the upstream primer is represented by SEQ ID NO: 40, and an example of the downstream primer is represented by SEQ ID NO: 41; as for *Serratia marcescens,* an example of the upstream primer is represented by SEQ ID NO: 43, and an example of the downstream primer is represented by SEQ ID NO: 44; as for *Candida tropicalis,* an example of the upstream primer is represented by SEQ ID NO: 46, and an example of the downstream primer is represented by SEQ ID NO: 47; as for *Candida krusei,* an example of the upstream primer is represented by SEQ ID NO: 49, and an example of the downstream primer is represented by SEQ ID NO: 50; as for *Candida parapsilosis,* an example of the upstream primer is represented by SEQ ID NO: 52, and an example of the downstream primer is represented by SEQ ID NO: 53; as for *Candida glabrata,* an example of the upstream primer is represented by SEQ ID NO: 55, and an example of the downstream primer is represented by SEQ ID NO: 56; as for *Candida albicans,* an example of the upstream primer is represented by SEQ ID NO: 58, and an example of the downstream primer is represented by SEQ ID NO: 59; as for *Cryptococcus neoformans*, an example of the upstream primer is represented by SEQ ID NO: 61, and an example of the downstream primer is represented by SEQ ID NO: 62; and as for an internal reference, an example of the upstream primer is represented by SEQ ID NO: 64, and an example of the downstream primer is represented by SEQ ID NO: 65.

In another aspect, this application provides probes for screening *Bacteroides fragilis*, one example of the probes is represented by SEQ ID NO: 3: 5'-CAGTTGTCCAAGTGGCGACG-3'; probes for screening *Staphylococcus epidermidis,* one example of the probes is represented by SEQ ID NO: 6: 5'-TGTGTATCATTATGCCATGAGCTTG-3'; probes for screening *Enterococcus faecalis*, one example of the probes is represented by SEQ ID NO: 9: 5'-GGCTTCATCCTAATCTTCAAGACAA-3'; probes for screening *Streptococcus pneumoniae*, one example of the probes is represented by SEQ ID NO: 12: 5'-TTAGAAAACGTGGGCAGGGAA-3'; probes for screening *Acinetobacter baumannii,* one example of the probes is represented by SEQ ID NO: 15: 5'-TCACAACCCGACAACGGTGAGCAA-3'; and probes for screening *Enterobacter cloacae*, one example of the probes is represented by SEQ ID NO: 18: 5'-AACCTTTGCCGACGTTGTCGGTCA-3'; probes for screening *Enterococcus faecium*, one example of the probes is represented by SEQ ID NO: 21: 5'-TTCATCCATTTTGGACTGATGCA-3'; probes for screening *Staphylococcus aureus,* one example of the probes is represented by SEQ ID NO: 24: 5'-TGGCTGAGATGAACTGTTCAGACCC-3'; probes for screening *Klebsiella pneumoniae*, one example of the probes is represented by SEQ ID NO: 27: 5'-CCGATTGAAAAACGCTCCGGGC-3'; probes for screening *Pseudomonas aeruginosa,* one example of the probes is represented by SEQ ID NO: 30: 5'-AACACAAACGCACTCGGAAAAATCG-3'; probes for screening *Escherichia coli,* one example of the probes is represented by SEQ ID NO: 33: 5'-GCCTGCAGCTTCCATACGCT-3'; probes for screening *Staphylococcus capitis,* one example of the probes is represented by SEQ ID NO: 36: 5'-ACCTCTTGCGAATAGTTCAGTACTTTC-3'; probes for screening *Stenotrophomonas maltophilia,* one example of the probes is represented by SEQ ID NO: 39: 5'-TCTTGCCTTCGTTCATCAGCTCGT-3'; probes for screening *Haemophilus influenzae,* one example of the probes is represented by SEQ ID NO: 42: 5'-CAAATATTGAAATTGGGCTTTGACG-3'; probes for screening *Serratia marcescens*, one example of the probes is represented by SEQ ID NO: 45: 5'-GCGCGCTGAACTACACCACT-3'; probes for screening *Candida tropicalis,* one example of the probes is represented by SEQ ID NO: 48: 5'-GGATCATACGTTCCATTTGCTTTA-3'; probes for screening *Candida krusei,* one example of the probes is represented by SEQ ID NO: 51: 5'-AGCATCTGGCCCTGGCTATAACAC-3'; probes for screening *Candida parapsilosis,* one example of the probes is represented by SEQ ID NO: 54: 5'-TAACGTATCTGCAGACGTGGCTGC-3'; probes for screening *Candida glabrata,* one example of the probes is represented by SEQ ID NO: 57: 5'-CTGCCGCAAGTCATGGGTTCTTG-3'; probes for screening *Candida albicans,* one example of the probes is represented by SEQ ID NO: 60: 5'-AACAAACTTGCTTTGGCGGTGGG-3'; probes for screening *Cryptococcus neoformans*, one example of the probes is represented by SEQ ID NO: 63: 5'-CCTGTCAGCCCGGCGTAATAAGTT-3'; an internal reference (human) probe, one example of the probes is represented by SEQ ID NO: 66: 5'-TGCCTGCCGTGTGAACCATGTGACT-3'.

In one aspect, this application provides combinations of primers and probes capable of simultaneously screening for 21 pathogens, the combinations of primers and probes comprises any one or more of the following sequences: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65 and SEQ ID NO: 66.

To improve the efficiency of screening for drug-resistance genes of bloodstream infection pathogens, in one aspect, this application provides a method for efficiently screening for drug-resistance genes of bloodstream infection pathogens by PCR. Particularly, as for *mecA,* an example of the upstream primer is represented by SEQ ID NO: 67, and an example of the downstream primer is represented by SEQ ID NO: 68; as for *mecC,* an example of the upstream primer is represented by SEQ ID NO: 70, and an example of the downstream primer is represented by SEQ ID NO: 71; as for *vanA,* an example of the upstream primer is represented by SEQ ID NO: 73, and an example of the downstream primer is represented by SEQ ID NO: 74; as for *vanB,* an example of the upstream primer is represented by SEQ ID NO: 76, and an example of the downstream primer is represented by SEQ ID NO: 77; as for *MCR-1,* an example of the upstream primer is represented by SEQ ID NO: 79, and an example of the downstream primer is represented by SEQ ID NO: 80; as for *blaKPC,* an example of the upstream primer is represented by SEQ ID NO: 82, and an example of the downstream primer is represented by SEQ ID NO: 83; as for *blaCTX-M,* an example of the upstream primer is represented by SEQ ID NO: 85, and an example of the downstream primer is represented by SEQ ID NO: 86; as for *blaOXA-23,* an example of the upstream primer is represented by SEQ ID NO: 88, and an example of the downstream primer is represented by SEQ ID NO: 89; as for *blaNDM-1,* an example of the upstream primer is represented by SEQ ID NO: 91, and an example of the downstream primer is represented by SEQ ID NO: 92; as for *blaTEM,* an example of the upstream primer is represented by SEQ ID NO: 94, and an example of the downstream primer is represented by SEQ ID NO: 95; as for *blaSHV,* an example of the upstream primer is represented by SEQ ID NO: 97, and an example of the downstream primer is represented by SEQ ID NO: 98; as for *blaVIM-1*, an example of the upstream primer is represented by SEQ ID NO: 100, and an example of the downstream primer is represented by SEQ ID NO: 101; as for *blaVIM-2,* an example of the upstream primer is represented by SEQ ID NO: 103, and an example of the downstream primer is represented by SEQ ID NO: 104; as for *blaIMP-1,* an example of the upstream primer is represented by SEQ ID NO: 106, and an example of the downstream primer is represented by SEQ ID NO: 107; as for *blaIMP-4,* an example of the upstream primer is represented by SEQ ID NO: 109, and an example of the downstream primer is represented by SEQ ID NO: 110; as for *SmeDEF,* an example of the upstream primer is represented by SEQ ID NO: 112, and an example of the downstream primer is represented by SEQ ID NO: 113; as for *Sull,* an example of the upstream primer is represented by SEQ ID NO: 115, and an example of the downstream primer is represented by SEQ ID NO: 116; as for *QnrA,* an example of the upstream primer is represented by SEQ ID NO: 118, and an example of the downstream primer is represented by SEQ ID NO: 119; as for *QnrB*, an example of the upstream primer is represented by SEQ ID NO: 121, and an example of the downstream primer is represented by SEQ ID NO: 122; as for *gyrB*, an example of the upstream primer is represented by SEQ ID NO: 124, and an example of the downstream primer is represented by SEQ ID NO: 125; as for *gyrA*, an example of the upstream primer is represented by SEQ ID NO: 127, and an example of the downstream primer is represented by SEQ ID NO: 128; as for *Aac(6')-Ib-cr,* an example of the upstream primer is represented by SEQ ID NO: 130, and an example of the downstream primer is represented by SEQ ID NO: 131; as for *ermB,* an example of the upstream primer is represented by SEQ ID NO: 133, and an example of the downstream primer is represented by SEQ ID NO: 134; as for *MefA,* an example of the upstream primer is represented by SEQ ID NO: 136, and an example of the downstream primer is represented by SEQ ID NO: 137; as for *cfr*, an example of the upstream primer is represented by SEQ ID NO: 139, and an example of the downstream primer is represented by SEQ ID NO: 140; as for *aac(3 ')-VI*, an example of the upstream primer is represented by SEQ ID NO: 142, and an example of the downstream primer is represented by SEQ ID NO: 143; as for *aph(3')-VI,* an example of the upstream primer is represented by SEQ ID NO: 145, and an example of the downstream primer is represented by SEQ ID NO: 146; as for *ant(2")-Ia*, an example of the upstream primer is represented by SEQ ID NO: 148, and an example of the downstream primer is represented by SEQ ID NO: 149; as for *DfrA1,* an example of the upstream primer is represented by SEQ ID NO: 151, and an example of the downstream primer is represented by SEQ ID NO: 152; as for *catA1,* an example of the upstream primer is represented by SEQ ID NO: 154, and an example of the downstream primer is represented by SEQ ID NO: 155; as for *TetA*, an example of the upstream primer is represented by SEQ ID NO: 157, and an example of the downstream primer is represented by SEQ ID NO: 158; as for *TetB*, an example of the upstream primer is represented by SEQ ID NO: 160, and an example of the downstream primer is represented by SEQ ID NO: 161; as for the internal reference *B2M* gene, an example of the upstream primer is represented by SEQ ID NO: 163, and an example of the downstream primer is represented by SEQ ID NO: 164.

In another aspect, this application provides probes for screening the *mecA* gene, one example of the probes is represented by SEQ ID NO: 69: 5'-AGCACTTGTAAGCACACCTTCATATGACG-3'; probes for screening the *mecC* gene, one example of the probes is represented by SEQ ID NO: 72: 5'-TCACTACATCACCAGGTTCAACCCA-3'; probes for screening the *VanA* gene, one example of the probes is represented by SEQ ID NO: 75: 5'-TCAGGCTCATCCTTCGGTGTGAAA-3'; probes for screening the *VanB* gene, one example of the probes is represented by SEQ ID NO: 78: 5'-TTACGCCAAAGGACGAACCTGACC-3'; probes for screening the *MCR-1* gene, one example of the probes is represented by SEQ ID NO: 81: 5'-AGTTTCTTTCGCGTGCATAAGCCG-3'; probes for screening the *blaKPC* gene, one example of the probes is represented by SEQ ID NO: 83: 5'-AAACCGGAACCTGCGGAGTGTATG-3'; probes for screening the *blaCTX-M* gene, one example of the probes is represented by SEQ ID NO: 87: 5'-TTCATCCATTTTGGACTGATGCA-3'; probes for screening the *blaOXA-23* gene, one example of the probes is represented by SEQ ID NO: 90: 5'-TGGCTGAGATGAACTGTTCAGACCC-3'; probes for screening the *blaNDM-1* gene, one example of the probes is represented by SEQ ID NO: 93: 5'-CAGGACAAGATGGGCGGTATGGAC-3'; probes for screening the *blaTEM* gene, one example of the probes is represented by SEQ ID NO: 96: 5'-TCACCCAGAAACGCTGGTGAAAGT-3'; probes for screening the *blaSHV* gene, one example of the probes is represented by SEQ ID NO: 99: 5'-AGGTGCTCATCATGGGAAAGCGTT-3'; probes for screening the *blaVIM-1* gene, one example of the probes is represented by SEQ ID NO: 102: 5'-CAAACGACTGCGTTGCGATATGCG-3'; probes for screening the *blaVIM-2* gene, one example of the probes is represented by SEQ ID NO: 105: 5'-CTGCGAGTGTGCTCTATGGTGGTT-3'; probes for screening the *blaIMP-1* gene, one example of the probes is represented by SEQ ID NO: 108: 5'-AGTGGTTTGGTTGCCTGAAAGGAA-3'; probes for screening the *blaIMP-4* gene, one example of the probes is represented by SEQ ID NO: 111: 5'-AGAAGCTTGGCCAAAGTCCGCTAA-3'; probes for screening the *SmeDEF* gene, one example of the probes is represented by SEQ ID NO: 114: 5'-TCAGCGTGGCGGAAGAAGAGTTT-3'; probes for screening the *Sull* gene, one example of the probes is represented by SEQ ID NO: 117: 5'-TCAATCGACAGCTTCCAACCGGAA-3'; probes for screening the *QnrA* gene, one example of the probes is represented by SEQ ID NO: 120: 5'-TTTCAAGGCCTGCCGTCTGTCTTT-3'; probes for screening the *OnrB* gene, one example of the probes is represented by SEQ ID NO: 123: 5'-ATTGCACCCTTTCTGGCTTTCACG-3'; probes for screening the *gyrB* gene, one example of the probes is represented by SEQ ID NO: 126: 5'-ATCATGACCGATGCTGACGTCGAC-3'; probes for screening the *gyrA* gene, one example of the probes is represented by SEQ ID NO: 129: 5'-CGCATCGCGCTTCACTTCAATCAC-3'; probes for screening the *Aac(6')-Ib-cr* gene, one example of the probes is represented by SEQ ID NO: 132: 5'-CATCACAACTGGGCAAAGGCTTGG-3'; probes for screening the *ermB* gene, one example of the probes is represented by SEQ ID NO: 135: 5'-TGCCAGCGGAATGCTTTCATCCTA-3'; probes for screening the *MefA* gene, one example of the probes is represented by SEQ ID NO: 138: 5'-CCCAGCACTCAATTCGGTTACACCA-3'; probes for screening the *cfr* gene, one example of the probes is represented by SEQ ID NO: 141: 5'-TACCTGCCCTTCGTTTGCTTCTCC-3'; probes for screening the *aac(3')-VI* gene, one example of the probes is represented by SEQ ID NO: NO: 144: 5'-TTCTGGCGTCTGGAGAATGTGACG-3'; probes for screening the *aph(3')-VI* gene, one example of the probes is represented by SEQ ID NO: 147: 5'-TTTAGATCTTGGCCGTGCTGGGTT-3'; probes for screening the *ant(2")-Ia* gene, one example of the probes is represented by SEQ ID NO: 150: 5'-TACTTGACTGCGAACCTGCTTGGT-3'; probes for screening the *DfrA1* gene, one example of the probes is represented by SEQ ID NO: 153: 5'-AATGGCTGTTGGTTGGACGCAAG-3'; probes for screening the *catA1* gene, one example of the probes is represented by SEQ ID NO: 156: 5'-ACGGTGTAACAAGGGTGAACACTATCC-3'; probes for screening the *TetA* gene, one example of the probes is represented by SEQ ID NO: 159: 5'-ATATCACTGATGGCGATGAGCGCG-3'; probes for screening the *TetB* gene, one example of the probes is represented by SEQ ID NO: 162: 5'-TGGATGGAATAGCATGATGGTTGGCT-3'; probes for an internal reference (human *B2M* gene), one example of the probes is represented by SEQ ID NO: 165: 5'-TGCCTGCCGTGTGAACCATGTGACT-3'.

In one aspect, this application provides combinations of primers and probes capable of simultaneously screening for drug-resistance genes of 32 pathogenic microorganisms, wherein the combinations of primers and probes comprises any one or more of the following sequences: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 91, SEQ ID NO: 92, SEQ ID NO: 93, SEQ ID NO: 94, SEQ ID NO: 95 and SEQ ID NO: 96.

In some embodiments, the 5' end of the probe sequence used in this application is labeled with a fluorescent group, and the 3' end of the probe sequence used in this application is labeled with a fluorescence quenching group. When the above probes are in the same reaction system, the fluorescent groups for screening different types of bloodstream pathogens and drug-resistance genes of microorganisms have different spectral ranges. The fluorescent groups of the probe sequences used in this application can be any fluorescent groups commonly used in the art, including but not limited to: FAM, VIC, ROX, Cy5, and/or HEX; the fluorescence quenching groups of the probe sequences used in this application can be any fluorescence quenching groups commonly used in the art, including but not limited to: BHQ1, BHQ2, BHQ3, and/or Eclipse.

In one aspect, the probes used in this application and their 5' end-labeled fluorescent reporter groups and 3' end-labeled fluorescence quenching groups are as follows: the probe for screening *Bacteroides fragilis* is represented by SEQ ID NO: 3, its 5' end fluorescently is labeled with FAM, and its 3' end is labeled with BHQ1, i.e., 5'-FAM-CAGTTGTCCAAGTGGCGACG-3'-BHQ1; the probe for screening *Staphylococcus epidermidis* is represented by SEQ ID NO: 6, its 5' end is fluorescently labeled with HEX, and its 3' end is fluorescently labeled with BHQ1, i.e., 5'-HEX-TGTGTATCATTATGCCATGAGCTTG-3'-BHQ1; the probe for screening *Enterococcus faecalis* is represented by SEQ ID NO: 9, its 5' end is fluorescently labeled with ROX, and its 3' end is fluorescently labeled with BHQ2, i.e., 5'-ROX-GGCTTCATCCTAATCTTCAAGACAA-3'-BHQ2; the probe for screening *Streptococcus pneumoniae* is represented by SEQ ID NO: 12, its 5' end is fluorescently labeled with Cy5, and its 3' end is fluorescently labeled with BHQ2, i.e., 5'-Cy5-TTAGAAAACGTGGGCAGGGAA-3'-BHQ2; the probe for screening *Acinetobacter baumannii* is represented by SEQ ID NO: 15, its 5' end is fluorescently labeled with FAM/HEX, and its 3' end is fluorescently labeled with BHQ1/BHQ2, i.e., 5'-FAM-TCACAACCCGACAACGGTGAGCAA-3'-BHQ1, 5'-HEX-TCACAACCCGACAACGGTGAGCAA-3'-BHQ2; the probe for screening *Enterobacter cloacae* is represented by SEQ ID NO: 18, its 5' end is fluorescently labeled with HEX, and its 3' end is fluorescently labeled with BHQ1, i.e., 5'-HEX-AACCTTTGCCGACGTTGTCGGTCA-3'-BHQ1; the probe for screening *Enterococcus faecium* is represented by SEQ ID NO: 21, its 5' end is fluorescently labeled is ROX, and its 3' end is fluorescently labeled with BHQ2, i.e., 5'-ROX-TTCATCCATTTTGGACTGATGCA-3'-BHQ2; the probe for screening *Staphylococcus aureus* is represented by SEQ ID NO: 24, its 5' end is fluorescently labeled with ROX/Cy5, and its 3' end is fluorescently labeled with BHQ2, i.e., 5'-ROX-TGGCTGAGATGAACTGTTCAGACCC-3'-BHQ2, 5'-Cy5-TGGCTGAGATGAACTGTTCAGACCC-3'-BHQ2; the probe for screening *Klebsiella pneumoniae* is represented by SEQ ID NO: 27, its 5' end is fluorescently labeled with ROX/Cy5, and its 3' end is fluorescently labeled with BHQ2, i.e., 5'-ROX-CCGATTGAAAAACGCTCCGGGC-3'-BHQ2, and 5'-Cy5-CCGATTGAAAAACGCTCCGGGC-3'-BHQ2; the probe for screening *Pseudomonas aeruginosa* is represented by SEQ ID NO: 30, its 5' end is fluorescently labeled with ROX/Cy5, and its 3' end is fluorescently labeled with BHQ2, i.e., 5'-ROX-AACACAAACGCACTCGGAAAAATCG-3'-BHQ2, and 5'-Cy5-AACACAAACGCACTCGGAAAAATCG-3'-BHQ2; the probe for screening *Escherichia coli* is represented by SEQ ID NO: 33, its 5' end is fluorescently labeled with FAM/HEX, and its 3' end is fluorescently labeled with BHQ1, i.e., 5'-FAM-GCCTGCAGCTTCCATACGCT-3'-BHQ1, and 5'-HEX-GCCTGCAGCTTCCATACGCT-3'-BHQ1; the probe for screening *Staphylococcus capitis* is represented by SEQ ID NO: 36, its 5' end is fluorescently labeled with FAM, and its 3' end is fluorescently labeled with BHQ1, i.e., 5'-FAM-ACCTCTTGCGAATAGTTCAGTACTTTC-3'-BHQ1; the probe for screening *Stenotrophomonas maltophilia* is represented by SEQ ID NO: 39, its 5' end is fluorescently labeled with ROX/Cy5, and its 3' end is fluorescently labeled with BHQ2, i.e., 5'-ROX-TCTTGCCTTCGTTCATCAGCTCGT-3'-BHQ2, and 5'-Cy5-TCTTGCCTTCGTTCATCAGCTCGT-3'-BHQ2; the probe for screening *Haemophilus influenzae* is represented by SEQ ID NO: 42, its 5' end is fluorescently labeled with ROX/Cy5, and its 3' end is fluorescently labeled with BHQ2, i.e., 5'-ROX-CAAATATTGAAATTGGGCTTTGACG-3'-BHQ2, and 5'-Cy5-CAAATATTGAAATTGGGCTTTGACG-3'-BHQ2; the probe for screening *Serratia marcescens* is represented by SEQ ID NO: 45, its 5' end is fluorescently labeled with Cy5.5, and its 3' end is fluorescently labeled with BHQ3, i.e., 5'-Cy5.5-GCGCGCTGAACTACACCACT-3'-BHQ3; the probe for screening *Candida tropicalis* is represented by SEQ ID NO: 48, its 5' end is fluorescently labeled with FAM/HEX, and its 3' end is fluorescently labeled with BHQ1, i.e., 5'-FAM-GGATCATACGTTCCATTTGCTTTA-3'-BHQ1, and 5'-HEX-GGATCATACGTTCCATTTGCTTTA-3'-BHQ1; the probe for screening *Candida krusei* is represented by SEQ ID NO: 51, its 5' end is fluorescently labeled with FAM/HEX, and its 3' end is fluorescently labeled with BHQ1, i.e., 5'-FAM-AGCATCTGGCCCTGGCTATAACAC-3'-BHQ1, and 5'-HEX-AGCATCTGGCCCTGGCTATAACAC-3'-BHQ1; the probe for screening *Candida parapsilosis* is represented by SEQ ID NO: 54, its 5' end is fluorescently labeled with FAM/HEX, and its 3' end is fluorescently labeled with BHQ1, i.e., 5'-FAM-TAACGTATCTGCAGACGTGGCTGC-3'-BHQ1, and 5'-HEX-TAACGTATCTGCAGACGTGGCTGC-3'-BHQ1; the probe for screening *Candida glabrata* is represented by SEQ ID NO: 57, its 5' end is fluorescently labeled with FAM/HEX, and its 3' end is fluorescently labeled with BHQ1, i.e., 5'-FAM-CTGCCGCAAGTCATGGGTTCTTG-3'-BHQ1, and 5'-HEX-CTGCCGCAAGTCATGGGTTCTTG-3'-BHQ1; the probe for screening *Candida albicans* is represented by SEQ ID NO: 60, its 5' end is fluorescently labeled with ROX/Cy5, and its 3' end is fluorescently labeled with BHQ2, i.e., 5'-ROX-AACAAACTTGCTTTGGCGGTGGG-3'-BHQ2, and 5'-Cy5-AACAAACTTGCTTTGGCGGTGGG-3'-BHQ2; the probe for screening *Cryptococcus neoformans* is represented by SEQ ID NO: 63, its 5' end is fluorescently labeled with ROX/Cy5, and its 3' end is fluorescently labeled with BHQ2, i.e., 5'-ROX-CCTGTCAGCCCGGCGTAATAAGTT-3'-BHQ2, and 5'-Cy5-CCTGTCAGCCCGGCGTAATAAGTT-3'-BHQ2; the probe for the internal reference (human) is represented by SEQ ID NO: 66, its 5' end is fluorescently labeled with Cy5.5, and its 3' end is fluorescently labeled with BHQ3, i.e., 5'-Cy5.5-TGCCTGCCGTGTGAACCATGTGACT-3'-BHQ3.

In another aspect, the probes used in this application and their 5' end-labeled fluorescent reporter groups and 3' end-labeled fluorescence quenching groups are as follows: the probe for screening the *mecA* gene is represented by SEQ ID NO: 69, its 5' end is fluorescently labeled with FAM, and its 3' end is fluorescently labeled with BHQ1, i.e., 5'-FAM-AGCACTTGTAAGCACACCTTCATATGACG-3'-BHQ1; the probe for screening the *mecC* gene is represented by SEQ ID NO: 72, its 5' end is fluorescently labeled with FAM, and its 3' end is fluorescently labeled with BHQ1, i.e., 5'-FAM-TCACTACATCACCAGGTTCAACCCA-3'-BHQ1; the probe for screening the *VanA* gene is represented by SEQ ID NO: 75, its 5' end is fluorescently labeled with FAM/HEX, and its 3' end is fluorescently labeled with BHQ1, i.e., 5'-FAM-TCAGGCTCATCCTTCGGTGTGAAA-3'-BHQ1, and 5'-HEX-TCAGGCTCATCCTTCGGTGTGAAA-3'-BHQ1; the probe for screening the *VanB* gene is represented by SEQ ID NO: 78, its 5' end is fluorescently labeled with FAM, and its 3' end is fluorescently labeled with BHQ1, i.e., 5'-FAM-TTACGCCAAAGGACGAACCTGACC-3'-BHQ1; the probe for screening the *MCR-1* gene is represented by SEQ ID NO: 81, its 5' end is fluorescently labeled with FAM/HEX, and its 3' end is fluorescently labeled with BHQ1, i.e., 5'-FAM-AGTTTCTTTCGCGTGCATAAGCCG-3'-BHQ1, and 5'-HEX-AGTTTCTTTCGCGTGCATAAGCCG-3'-BHQ1; the probe for screening the *blaKPC* gene is represented by SEQ ID NO: 84, its the 5' end is fluorescently labeled with FAM/HEX, and its 3' end is fluorescently labeled with BHQ1, i.e., 5'-FAM-AAACCGGAACCTGCGGAGTGTATG-3'-BHQ1, and 5'-HEX-AAACCGGAACCTGCGGAGTGTATG-3'-BHQ1; the probe for screening the *blaCTX-M* gene is represented by SEQ ID NO: 87, its 5' end is fluorescently labeled with FAM/HEX, and its 3' end is fluorescently labeled with BHQ1, i.e., 5'-FAM-TTCATCCATTTTGGACTGATGCA-3'-BHQ1, and 5'-HEX-TTCATCCATTTTGGACTGATGCA-3'-BHQ1; the probe for screening the *blaOXA-23* gene is represented by SEQ ID NO: 90, its 5' end is fluorescently labeled with FAM/HEX, and its 3' end is fluorescently labeled with BHQ1, i.e., 5'-FAM-TGGCTGAGATGAACTGTTCAGACCC-3'-BHQ1, and 5'-HEX-TGGCTGAGATGAACTGTTCAGACCC-3'-BHQ1; the probe for screening the *blaNDM-1* gene is represented by SEQ ID NO: 93, its 5' end is fluorescently labeled with HEX, and its 3' end is fluorescently labeled with BHQ1, i.e., 5'-HEX-CAGGACAAGATGGGCGGTATGGAC-3'-BHQ1; the probe for screening the *blaTEM* gene is represented by SEQ ID NO: 96, its 5' end is fluorescently labeled with FAM/HEX, and its 3' end is fluorescently labeled with BHQ1, i.e., 5'-FAM-TCACCCAGAAACGCTGGTGAAAGT-3'-BHQ1, and 5'-HEX-TCACCCAGAAACGCTGGTGAAAGT-3'-BHQ1; the probe for screening the *blaSHV* gene is represented by SEQ ID NO: 99, the 5' end is fluorescently labeled with FAM/HEX, and its 3' end is fluorescently labeled with BHQ1, i.e., 5'-FAM-AGGTGCTCATCATGGGAAAGCGTT-3'-BHQ1, and 5'-HEX-AGGTGCTCATCATGGGAAAGCGTT-3'-BHQ1; the probe for screening the *blaVIM-1* gene is represented by SEQ ID NO: 102, its 5' end is fluorescently labeled with HEX, and its 3' end is fluorescently labeled with BHQ1, i.e., 5'-HEX-CAAACGACTGCGTTGCGATATGCG-3'-BHQ1; the probe for screening the *blaVIM-2* gene is represented by SEQ ID NO: 105, its 5' end is fluorescently labeled with FAM/HEX, and its 3' end is fluorescently labeled with BHQ1, i.e., 5'-FAM-CTGCGAGTGTGCTCTATGGTGGTT-3'-BHQ1, and 5'-HEX-CTGCGAGTGTGCTCTATGGTGGTT-3'-BHQ1; the probe for screening the *blaIMP-1* gene is represented by SEQ ID NO: 108, its 5' end is fluorescently labeled with ROX, and its 3' end is fluorescently labeled with BHQ2, i.e., 5'-ROX-AGTGGTTTGGTTGCCTGAAAGGAA-3'-BHQ2; the probe for screening the *blaIMP-4* gene is represented by SEQ ID NO: 111, its 5' end is fluorescently labeled with FAM/HEX, and its 3' end is fluorescently labeled with BHQ1, i.e., 5'-FAM-AGAAGCTTGGCCAAAGTCCGCTAA-3'-BHQ1 and 5'-HEX-AGAAGCTTGGCCAAAGTCCGCTAA-3'-BHQ1; the probe for screening the *SmeDEF* gene is represented by SEQ ID NO: 114, its 5' end is fluorescently labeled with ROX/Cy5, and its 3' end is fluorescently labeled with BHQ2, i.e., 5'-ROX-TCAGCGTGGCGGAAGAAGAGTTT-3'-BHQ2, and 5'-CY5-TCAGCGTGGCGGAAGAAGAGTTT-3'-BHQ2; the probe for screening the *Sul1* gene is represented by SEQ ID NO: 117, its 5' end is fluorescently labeled with ROX/CY5, and its 3' end is fluorescently labeled with BHQ2, i.e., 5'-ROX-TCAATCGACAGCTTCCAACCGGAA-3'-BHQ2, and 5'-CY5-TCAATCGACAGCTTCCAACCGGAA-3'-BHQ2; the probe for screening the *QnrA* gene is represented by SEQ ID NO: 120, its 5' end is fluorescently labeled with HEX, and its 3' end is fluorescently labeled with BHQ1, i.e., 5'-HEX-TTTCAAGGCCTGCCGTCTGTCTTT-3'-BHQ1; the probe for screening the *OnrB* gene is represented by SEQ ID NO: 123, its 5' end is fluorescently labeled with ROX/CY5, and its 3' end is fluorescently labeled with BHQ2, i.e., 5'-ROX-ATTGCACCCTTTCTGGCTTTCACG-3'-BHQ2, and 5'-CY5-ATTGCACCCTTTCTGGCTTTCACG-3'-BHQ2; the probe for screening the *gyrB* gene is represented by SEQ ID NO: 126, its 5' end is fluorescently labeled with Cy5, and its 3' end fluorescently is labeled with BHQ2, i.e., 5'-Cy5-ATCATGACCGATGCTGACGTCGAC-3'-BHQ2; the probe for screening the *gyrA* gene is represented by SEQ ID NO: 129, its 5' end is fluorescently labeled with ROX, and its 3' end is fluorescently labeled with BHQ2, i.e., 5'-ROX-CGCATCGCGCTTCACTTCAATCAC-3'-BHQ2; the probe for screening the *Aac(6')-Ib-cr* gene is represented by SEQ ID NO: 132, its 5' end is fluorescently labeled with CY5.5, and its 3' end is fluorescently labeled with BHQ3, i.e., 5'-CY5.5-CATCACAACTGGGCAAAGGCTTGG-3'-BHQ3; the probe for screening the *ermB* gene is represented by SEQ ID NO: 135, its 5' end is fluorescently labeled with ROX/CY5, and its 3' end is fluorescently labeled with BHQ2, i.e., 5'-ROX-TGCCAGCGGAATGCTTTCATCCTA-3'-BHQ2, and 5'-CY5-TGCCAGCGGAATGCTTTCATCCTA-3'-BHQ2; the probe for screening the *MefA* gene is represented by SEQ ID NO: 138, its 5' end is fluorescently labeled with CY5, and its 3' end is labeled with BHQ2, i.e., 5'-CY5-CCCAGCACTCAATTCGGTTACACCA-3'-BHQ2; the probe for screening the *cfr* gene is represented by SEQ ID NO: 141, its 5' end is fluorescently labeled with ROX/Cy5, and its 3' end is fluorescently labeled with BHQ2, i.e., 5'-ROX-TACCTGCCCTTCGTTTGCTTCTCC-3'-BHQ2, and 5'-CY5-TACCTGCCCTTCGTTTGCTTCTCC-3'-BHQ2; the probe for screening the *aac(3')-VI* gene is represented by SEQ ID NO: 144, its 5' end is fluorescently labeled with ROX/CY5, and its 3' end is fluorescently labeled with BHQ2, i.e., 5'-ROX-TTCTGGCGTCTGGAGAATGTGACG-3'-BHQ2, and 5'-CY5-TTCTGGCGTCTGGAGAATGTGACG-3'-BHQ2; the probe for screening the *aph(3')-VI* gene is represented by SEQ ID NO: 147, its 5' end is fluorescently labeled with ROX/CY5, and its 3' end is fluorescently labeled with BHQ2, i.e., 5'-ROX-TTTAGATCTTGGCCGTGCTGGGTT-3'-BHQ2, and 5'-CYS-TTTAGATCTTGGCCGTGCTGGGTT-3'-BHQ2; the probe for screening the *ant(2")-Ia* gene is represented by SEQ ID NO: 150, its 5' end is fluorescently labeled with ROX/Cy5, and its 3' end is fluorescently labeled with BHQ2, i.e., 5'-ROX-TACTTGACTGCGAACCTGCTTGGT-3'-BHQ2, and 5'-CY5-TACTTGACTGCGAACCTGCTTGGT-3'-BHQ2; the probe for screening the *DfrA1* gene is represented by SEQ ID NO: 153, its 5' end is fluorescently labeled with ROX/CY5, and its 3' end is fluorescently labeled with BHQ2, i.e., 5'-ROX-AATGGCTGTTGGTTGGACGCAAG-3'-BHQ2, and 5'-CY5-AATGGCTGTTGGTTGGACGCAAG-3'-BHQ2; the probe for screening the *catA1* gene is represented by SEQ ID NO: 156, its 5' end is fluorescently labeled with CY5, and its 3' end is fluorescently labeled with BHQ2, i.e., 5'-CY5-ACGGTGTAACAAGGGTGAACACTATCC-3'-BHQ2; the probe for screening the *tetA* gene is represented by SEQ ID NO: 159, its 5' end is fluorescently labeled with ROX, and its 3' end is fluorescently labeled with BHQ2, i.e., 5'-ROX-ATATCACTGATGGCGATGAGCGCG-3'-BHQ2; the probe for screening the *tetB* gene is represented by SEQ ID NO: 162, its 5' end is fluorescently labeled with CY5.5, and its 3' end is fluorescently labeled with BHQ3, i.e., 5'-CY5.5-TGGATGGAATAGCATGATGGTTGGCT-3'-BHQ3; the probe for the internal reference (human) is represented by SEQ ID NO: 99, its 5' end is fluorescently labeled with Cy5.5, and its 3' end is fluorescently labeled with BHQ3, i.e., 5'-Cy5.5-TGCCTGCCGTGTGAACCATGTGACT-3'-BHQ3.

In some embodiments, the digital PCR reaction premix provided in this application may comprise, in addition to the above primer pairs or any combination of the primer pairs and probes described above, any one, two, three, four, five, six, seven or eight components selected from: hot-start Taq enzyme, UDG enzyme, dNTPs, bovine serum albumin, glycerol, betaine, NH₄⁺ and/or Mg²⁺ ions.

This application provides a kit for detecting a target gene, wherein the kit comprises the primer pairs mentioned above, or any combination of primer pairs and probes mentioned above, or a combination of the primer pairs mentioned above and any probes. The kit according to this application may further comprise any one, two, three, four, five, six, seven or eight components selected from the group consisting of: hot-start Taq enzyme, UDG enzyme, dNTPs, bovine serum albumin, glycerol, betaine, NH₄⁺ and/or Mg²⁺ ions. The kits according to this application may further comprise positive control and/or negative control.

In some embodiments, this application provides a method for detecting a target gene for non-disease diagnosis purposes, wherein the method comprises following the steps.

### (1) Treating the samples to be tested

The samples to be tested have a volume of 2-10 mL, preferably 5-10 mL, 6-10 mL, 7-10 mL, 8-10 mL, or 9-10 mL, most preferably 10 mL to improve the detection rate. The samples are centrifuged at 800 rpm for 10 min, then collecting the supernatant. The separated samples can be stored at -20 °C±5 °C for 3 years. It is recommended to use commercially available kits to extract DNA from the samples to be tested. After DNA extraction, it is recommended to perform testing immediately; otherwise, it is best to store it at -20°C or below for no more than 3 months.

(2) Preparing the digital PCR amplification mixture according to Table 1, which comprises the sample to be tested provided in step (1), and the digital PCR reaction premix solution as below. Particularly, the final concentrations of the forward and reverse primers in the digital PCR reaction premix solution are 50-900 nM, preferably 50-500 nM, 200-500 nM, and most preferably 450 nM, respectively; the final concentration of each probe are 50-800 nM, preferably 50-500 nM, 100-400 nM, and most preferably 250 nM; the final concentration of Mg²⁺ ions is 0.5-5 mM, preferably 1-4.5 mM, 1.5-4 mM, 2-3.5 mM, and most preferably 3 mM; the final concentration of bovine serum albumin is 0.01-1 wt%, preferably 0.02-0.5 wt%, 0.03-0.1 wt%, 0.04-0.08 wt%, most preferably 0.05%; the final concentration of hot-start Taq enzyme (which can be antibody-modified hot-start Taq enzyme, chemically modified hot-start Taq enzyme, or aptamer-modified hot-start Taq enzyme) is 0.5-10 U/reaction, preferably 1-5 U/reaction, 1.5-4 U/reaction, 2-3.5 U/reaction, and most preferably 2.5 U/reaction; the final concentration of dNTP is 20-300 µM, preferably 50-250 µM, 100-230 µM, 150-220 µM, and most preferably 200 µM. The digital PCR reaction premix may further comprise UNG enzyme, with a final concentration of 0.1-10 U/reaction, 0.5-5 U/reaction, 1-2.5 U/reaction, 1-2 U/reaction, and most preferably 1.5 U/reaction. The pH value of the digital PCR reaction premix is 7-9, preferably 7.4-8.5, more preferably 7.8-8.2, and most preferably 8.0.

Those skilled in the art will understand that the substances and concentrations listed in the above PCR amplification mixture are illustrative, and they can be selected based on the actual system.

**Table 1: Digital PCR Amplification Mixture**

| Reagent | Volume (µL) |
|---|---|
| Digital PCR reaction premix | 5 |
| Sample (template), or positive quality control, or negative quality control | Variable |
| NTC (distilled water without DNase and/or RNase) | Adding to 20µL |
| Total volume | 20 |

### (3) Preparing microdroplets and performing PCR amplification reaction;

Step 1: Preparation of microdroplets. 50-100 µl of droplet-generating oil, preferably 70-100 µl, and most preferably 75 µl, was added into the square oil-filling well at the top of the microfluidic cartridge.
Step 2: 13-25 µl, preferably 15-25 µl, and most preferably 20 µl of digital PCR amplification mixture (comprising template for amplifying nucleic acid, primers, and probes, etc.) was added into the circular sample well at the top of the microfluidic cartridge;
Step 3: 5-10 µl, preferably 10 µl of sealing oil was added to the top of the digital PCR amplification mixture by pipette;
Step 4: The top sample loading port and oil filling port of the microfluidic cartridge, and the bottom waste liquid port with were covered with two plastic sealing caps respectively; the microfluidic cartridge comprising the PCR amplification mixture was placed on a PCR amplification instrument, preferably an SG-2000 PCR amplification instrument. The amplification reaction can be performed according to the amplification procedure recommended in Table 2:

**Table 2: An example of PCR amplification reaction procedure.**

| Reaction steps | Reaction temperature | Reaction time | Heating rate | Number of cycles |
|---|---|---|---|---|
| Pre-denaturation | 95°C | 10 min | | 1 |
| Denaturation | 94°C | 30 s | | 40 |
| Annealing, extension | 60°C | 60 s | 2°C /s | |
| Inactivation | 98°C | 10 min | | 1 |
| Cooling | 20°C | 2 min | | 1 |

### (4) Signal collection

On a biochip reader (e.g., Dscanner4-1000, registration certificate number: Su Medical Device Registration 20202220818), MUT mode was selected, and the fluorescence channels were set to FAM, HEX, ROX, and Cy5. Then the high-throughput digital PCR cartridge (e.g., microfluidic cartridge) was removed from the PCR amplification instrument, preferably the SG-2000 PCR amplification instrument (registration certificate number: Su Medical Device Registration 20202220887), smoothly transferring it to the biochip reader for signal collection.

### (5) Results analysis

"Analyze" button on the biochip reader was clicked to analyze the experimental data, firstly checking if "Accepted Droplets" ≥ 10000 before proceeding with the analysis. "2D Amplitude" button was clicked to view the 2D scatter plots for Channel 1, Channel 2, Channel 3 and Channel 4. Based on the four positive quality controls, the 2D graph was clicked to divide each region. In each diagram, the darkest black dots at the bottom represent droplets without amplification template, green dots represent droplets comprising FAM-labeled probes, blue dots represent droplets comprising HEX-labeled probes, orange dots represent droplets comprising ROX-labeled probes, magenta dots represent droplets comprising Cy5 probes, and red dots represent droplets comprising both sets of probe markers.

The primers, probes, digital PCR reaction premix comprising primers and probes, kits, and microfluidic cartridges provided in this application can all be used to prepare quantitative detection kits for detecting multiple pathogens in bloodstream infections. They can also be used alone or in combination with other reagents for the detection of multiple pathogens in bloodstream infections. The processing steps of plasma samples are as follows: collecting 2-10 mL of peripheral blood sample, preferably 5-10 mL, 6-10 mL, 7-10 mL, 8-10 mL, or 9-10 mL, most preferably 10 mL to improve the detection rate. EDTA anticoagulant is preferred; heparin anticoagulant should not be used. Plasma should be separated within 2 hours after blood is removed from the body, and generally should not exceed 4 hours; the plasma volume should be 0.5-4 mL, preferably 1-4 mL, 2-4 mL, or 3-4 mL, and most preferably 4 mL. The plasma was centrifuged at 800 rpm for 10 min to collect the supernatant. If plasma cannot be separated in time, vacuum blood collection tubes can be used for room temperature storage, or special room temperature blood collection tubes (Streck tubes) comprising free DNA protectant and protectant for preventing cell lysis can be used, with a storage time not exceeding 3 days. The separated plasma can be stored at -20°C±5°C for 3 years. It is recommended to use commercially available kits to extract microbial DNA from plasma samples. After DNA extraction, it is recommended to perform testing immediately; otherwise, it is best to store it at -20°C or below for no more than 3 months.

In some embodiments, the types of samples involved in the detection further comprise whole blood (also known as blood), plasma, serum, throat swabs, sputum, urine, feces, cerebrospinal fluid, body fluids, or ascites.

A whole blood test involves drawing peripheral blood from a patient and examining all blood cells and plasma components as a whole. In addition, plasma or serum from the blood can be used as samples for testing as needed.

A throat swab test is a medical testing method that involves using a medical cotton swab to collect a small amount of secretions from the human throat, inoculating it into a specially designed culture dish, and then placing it in a temperature-controlled device for incubation. The throat swab samples in this application can be samples obtained directly from clinical settings by those skilled in the art, or throat swab samples that have been cultured for a period of time, depending on the items that need to be tested.

Sputum is the secretion from the alveoli, bronchi, and trachea. Healthy people produce very little sputum. Under normal circumstances, the bronchial mucosal glands and goblet cells can secrete a small amount of mucus to keep the respiratory mucosa moist. Under pathological conditions, the respiratory mucosa and alveoli become congested and edematous after being stimulated, with serous exudation and increased mucus secretion. Sputum is formed by the mixture of various cells (red blood cells, white blood cells, phagocytes, etc.), exudates such as fibrin, mucus, inhaled dust, and necrotic tissue. The sputum sample used in this application is one that can be obtained by those skilled in the art.

Urine and fecal samples are also samples that can be obtained by those skilled in the art through conventional means.

Cerebrospinal fluid (CSF) is a colorless and transparent liquid that fills the ventricles, subarachnoid space, and central canal of the spinal cord. Cerebrospinal fluid is produced by the choroid plexus in the ventricles of the brain, and is similar in nature to blood plasma and lymph, being slightly viscous. Cerebrospinal fluid is an extracellular fluid. The cerebrospinal fluid sample used in this application is one that can be obtained by those skilled in the art.

In this application, body fluids refer to various body fluids such as blood, urine, feces, serous cavity fluid, cerebrospinal fluid, and ascites.

The ascites mentioned in this application refers to the fluid inside the abdominal cavity of a human body, which can be obtained through conventional means.

For convenience, the sequences of primers and probes used in this application are summarized in the table below:

**Table 3: Sequences of primers and probes for 21 pathogens in this application**

| SEQ ID NO: | Sequence name | Specific sequence (5'-3') |
|---|---|---|
| SEQ ID NO:1 | Upstream primer of *Bacteroides fragilis* | TCGGTTTATGCAGTTCATGG |
| SEQ ID NO:2 | Downstream primer of *Bacteroides fragilis* | AAGCCTTCCAGTCCCTCTTT |
| SEQ ID NO:3 | Probe of *Bacteroides fragilis* | CAGTTGTCCAAGTGGCGACG |
| SEQ ID NO:4 | Upstream primer of *Staphylococcus epidermidis* | CCAAGATTTCACGGACCATT |
| SEQ ID NO:5 | Downstream primer of *Staphylococcus epidermidis* | AGATCGTGACCATGCACAAC |
| SEQ ID NO:6 | Probe of *Staphylococcus epidermidis* | TGTGTATCATTATGCCATGAGCTTG |
| SEQ ID NO:7 | Upstream primer of *Enterococcus faecalis* | GCTCTAATTGACTCGGGGATT |
| SEQ ID NO:8 | Downstream primer of *Enterococcus faecalis* | GCAACTTGTGTACCATGACCA |
| SEQ ID NO:9 | Probe of *Enterococcus faecalis* | GGCTTCATCCTAATCTTCAAGACAA |
| SEQ ID NO:10 | Upstream primer of *Streptococcus pneumoniae* | TTGCCTGTAGTCGAATGACG |
| SEQ ID NO:11 | Downstream primer of *Streptococcus pneumoniae* | GCCACGAGAGAGAACAAAGG |
| SEQ ID NO:12 | Probe of *Streptococcus pneumoniae* | TTAGAAAACGTGGGCAGGGAA |
| SEQ ID NO:13 | Upstream primer of *Acinetobacter baumannii* | ACGCCCTAGACCCTCAATA |
| SEQ ID NO:14 | Downstream primer of *Acinetobacter baumannii* | GGACAAGCATGTCAGCAATTT |
| SEQ ID NO:15 | Probe of *Acinetobacter baumannii* | TCACAACCCGACAACGGTGAGCAA |
| SEQ ID NO:16 | Upstream primer of *Enterobacter cloacae* | TCAGGTGTTAGCCCGTAAATG |
| SEQ ID NO:17 | Downstream primer of *Enterobacter cloacae* | AGGGCCGTCAGCACATG |
| SEQ ID NO:18 | Probe of *Enterobacter cloacae* | AACCTTTGCCGACGTTGTCGGTCA |
| SEQ ID NO:19 | Upstream primer of *Enterococcus faecium* | TCAATGAATGGTGGCGTTTA |
| SEQ ID NO:20 | Downstream primer of *Enterococcus faecium* | TTGCTGCCCCATAAAATACA |
| SEQ ID NO:21 | Probe of *Enterococcus faecium* | TTCATCCATTTTGGACTGATGCA |
| SEQ ID NO:22 | Upstream primer of *Staphylococcus aureus* | AACTACTAGGGGAGCCTAATAAT |
| SEQ ID NO:23 | Downstream primer of *Staphylococcus aureus* | GGTACTAACCAAATCAGGTCATAA |
| SEQ ID NO:24 | Probe of *Staphylococcus aureus* | TGGCTGAGATGAACTGTTCAGACCC |
| SEQ ID NO:25 | Upstream primer of *Klebsiella pneumoniae* | CGATGCTACTTATCCCGACA |
| SEQ ID NO:26 | Downstream primer of *Klebsiella pneumoniae* | AGCCGGTTGAGACGTAAAC |
| SEQ ID NO:27 | Probe of *Klebsiella pneumoniae* | CCGATTGAAAAACGCTCCGGGC |
| SEQ ID NO:28 | Upstream primer of *Pseudomonas aeruginosa* | GCTTCATCGACAGCATCGG |
| SEQ ID NO:29 | Downstream primer of *Pseudomonas aeruginosa* | CGGCTTTTTCTCTGGTCTGTG |
| SEQ ID NO:30 | Probe of *Pseudomonas aeruginosa* | AACACAAACGCACTCGGAAAAATCG |
| SEQ ID NO:31 | Upstream primer of *Escherichia coli* | TTATGCCAGCAGATTTGCAC |
| SEQ ID NO:32 | Downstream primer of *Escherichia coli* | TCTTCCGAGGCTTGTATGCT |
| SEQ ID NO:33 | Probe of *Escherichia coli* | GCCTGCAGCTTCCATACGCT |
| SEQ ID NO:34 | Upstream primer of *Staphylococcus capitis* | TGGCGCCACATTGAGAATAA |
| SEQ ID NO:35 | Downstream primer of *Staphylococcus capitis* | CTACATCGGTAGAATCGCCAAC |
| SEQ ID NO:36 | Probe of *Staphylococcus capitis* | ACCTCTTGCGAATAGTTCAGTACTTTC |
| SEQ ID NO:37 | Upstream primer of *Stenotrophomonas maltophilia* | GCTTGAAGCCCTGACAGATGTA |
| SEQ ID NO:38 | Downstream primer of *Stenotrophomonas maltophilia* | TCGACAAGCCGTACGACAT |
| SEQ ID NO:39 | Probe of *Stenotrophomonas maltophilia* | TCTTGCCTTCGTTCATCAGCTCGT |
| SEQ ID NO:40 | Upstream primer of *Haemophilus influenzae* | AGAGCTGCTTGAGAATGAGAGC |
| SEQ ID NO:41 | Downstream primer of *Haemophilus influenzae* | ACCTGCATCGTGTTCCAC |
| SEQ ID NO:42 | Probe of *Haemophilus influenzae* | CAAATATTGAAATTGGGCTTTGACG |
| SEQ ID NO:43 | Upstream primer of *Serratia marcescens* | AAACTGCCTGCGACCATC |
| SEQ ID NO:44 | Downstream primer of *Serratia marcescens* | CGAAGACGATCTTGTCAAAGAA |
| SEQ ID NO:45 | Probe of *Serratia marcescens* | GCGCGCTGAACTACACCACT |
| SEQ ID NO:46 | Upstream primer of *Candida tropicalis* | GGATCCTCTGATTTATGGGTAGTT |
| SEQ ID NO:47 | Downstream primer of *Candida tropicalis* | TGCCACGTGTGTTCGTAGATTTGAACA |
| SEQ ID NO:48 | Probe of *Candida tropicalis* | GGATCATACGTTCCATTTGCTTTA |
| SEQ ID NO:49 | Upstream primer of *Candida krusei* | GTTCTGGAACGTGGCTCTT |
| SEQ ID NO:50 | Downstream primer of *Candida krusei* | CAGCATCCGTGACCTACAC |
| SEQ ID NO:51 | Probe of *Candida krusei* | AGCATCTGGCCCTGGCTATAACAC |
| SEQ ID NO:52 | Upstream primer of *Candida parapsilosis* | CCACGTAACTTGTCTCGTATCC |
| SEQ ID NO:53 | Downstream primer of *Candida parapsilosis* | GGTGACACTTGAGCCATCTT |
| SEQ ID NO:54 | Probe of *Candida parapsilosis* | TAACGTATCTGCAGACGTGGCTGC |
| SEQ ID NO:55 | Upstream primer of *Candida glabrata* | GCTGCGGAGAGGAATAAGAAA |
| SEQ ID NO:56 | Downstream primer of *Candida glabrata* | GCTCTTTGCAGTGAGACAGTA |
| SEQ ID NO:57 | Probe of *Candida glabrata* | CTGCCGCAAGTCATGGGTTCTTG |
| SEQ ID NO:58 | Upstream primer of *Candida albicans* | GAACCTGCGGAAGGATCAATA |
| SEQ ID NO:59 | Downstream primer of *Candida albicans* | TGGTTGTAAGTTTAGACCTCTGG |
| SEQ ID NO:60 | Probe of *Candida albicans* | AACAAACTTGCTTTGGCGGTGGG |
| SEQ ID NO:61 | Upstream primer of *Cryptococcus neoformans* | GCTCGCCTTAAATGTGTTAGTG |
| SEQ ID NO:62 | Downstream primer of *Cryptococcus neoformans* | GTTGTTATCAGCAAGCCGAAG |
| SEQ ID NO:63 | Probe of *Cryptococcus neoformans* | CCTGTCAGCCCGGCGTAATAAGTT |
| SEQ ID NO:64 | Upstream primer of internal reference (human) | GCAAGGACTGGTCTTTCTATCTCT |
| SEQ ID NO:65 | Downstream primer of internal reference (human) | ACCCCACTTAACTATCTTGGGC |
| SEQ ID NO:66 | Probe of internal reference (human) | TGCCTGCCGTGTGAACCATGTGACT |

**Table 4: Examples of fluorescently labeled probes used in this application.**

| Name of Bacterium | Probe | 5' end labelling | 3' end labelling |
|---|---|---|---|
| *Bacteroides fragilis* | SEQ ID NO:3 | FAM | BHQ1 |
| *Staphylococcus epidermidis* | SEQ ID NO:6 | HEX | BHQ1 |
| *Enterococcus faecalis* | SEQ ID NO:9 | ROX | BHQ2 |
| *Streptococcus pneumoniae* | SEQ ID NO:12 | Cy5 | BHQ2 |
| *Acinetobacter baumannii* | SEQ ID NO:15 | FAM | BHQ1 |
| | | HEX | BHQ2 |
| *Enterobacter cloacae* | SEQ ID NO:18 | HEX | BHQ1 |
| *Enterococcus faecium* | SEQ ID NO:21 | ROX | BHQ2 |
| *Staphylococcus aureus* | SEQ ID NO:24 | ROX | BHQ2 |
| | | Cy5 | BHQ2 |
| *Klebsiella pneumoniae* | SEQ ID NO:27 | ROX | BHQ2 |
| | | Cy5 | BHQ2 |
| *Pseudomonas aeruginosa* | SEQ ID NO:30 | ROX | BHQ2 |
| | | Cy5 | BHQ2 |
| *Escherichia coli* | SEQ ID NO:33 | FAM | BHQ1 |
| | | HEX | BHQ1 |
| *Staphylococcus capitis* | SEQ ID NO:36 | FAM | BHQ1 |
| *Stenotrophomonas maltophilia* | SEQ ID NO:39 | ROX | BHQ2 |
| | | Cy5 | BHQ2 |
| *Haemophilus influenzae* | SEQ ID NO:42 | ROX | BHQ2 |
| | | Cy5 | BHQ2 |
| *Serratia marcescens* | SEQ ID NO:45 | Cy5.5 | BHQ3 |
| *Candida tropicalis* | SEQ ID NO:48 | FAM | BHQ1 |
| | | HEX | BHQ1 |
| *Candida krusei* | SEQ ID NO:51 | FAM | BHQ1 |
| | | HEX | BHQ1 |
| *Candida parapsilosis* | SEQ ID NO:54 | FAM | BHQ1 |
| | | HEX | BHQ1 |
| *Candida glabrata* | SEQ ID NO:57 | FAM | BHQ1 |
| | | HEX | BHQ1 |
| *Candida albicans* | SEQ ID NO:60 | ROX | BHQ2 |
| | | Cy5 | BHQ2 |
| *Cryptococcus neoformans* | SEQ ID NO:63 | ROX | BHQ2 |
| | | Cy5 | BHQ2 |
| Internal reference (human) | SEQ ID NO:66 | Cy5.5 | BHQ3 |

**Table 5: Sequences of primers and probes of this application targeting 32 microbial resistance genes.**

| SEQ ID NO: | Sequence name | Specific sequence (5'-3') | Genbank No. of a Gene |
|---|---|---|---|
| SEQ ID NO:67 | Upstream primer of *mecA* gene | GTACTGCTATCCACCCTCAAAC | NC_016941.1 (39646-41655) Pp449373 |
| SEQ ID NO:68 | Downstream primer of *mecA* gene | TTCTTCGTTACTCATGCCATACA | |
| SEQ ID NO:69 | Probe of *mecA* gene | AGCACTTGTAAGCACACCTTCA TATGACG | |
| SEQ ID NO:70 | Upstream primer of *mecC* gene | AGCCTTTGCTCAACAAATTTCA | NG_047955.1 |
| SEQ ID NO:71 | Downstream primer of *mecC* gene | TGCCAACCCTTACCATAAATAT CA | |
| SEQ ID NO:72 | Probe of *mecC* gene | TCACTACATCACCAGGTTCAAC CCA | |
| SEQ ID NO:73 | Upstream primer of *VanA* gene | GGCAGCTACGTTTACCTATCC | NG_048323.1 |
| SEQ ID NO:74 | Downstream primer of *VanA* gene | AATTCGTCCGCGCTATTGA | |
| SEQ ID NO:75 | Probe of *VanA* gene | TCAGGCTCATCCTTCGGTGTGA AA | |
| SEQ ID NO:76 | Upstream primer of *VanB* gene | CGCTTACCTACCCTGTCTTTG | NG_048334.1 |
| SEQ ID NO:77 | Downstream primer of *VanB* gene | ATTGTCCTGCCGCTTCTATC | |
| SEQ ID NO:78 | Probe of *VanB* gene | TTACGCCAAAGGACGAACCTGA CC | |
| SEQ ID NO:79 | Upstream primer of *MCR-1* gene | TGGCGTTCAGCAGTCATTAT | NZ_CP041926 |
| SEQ ID NO:80 | Downstream primer of *MCR-1* gene | AGCTTACCCACCGAGTAGAT | |
| SEQ ID NO:81 | Probe of *MCR-1* gene | AGTTTCTTTCGCGTGCATAAGC CG | |
| SEQ ID NO:82 | Upstream primer of *blaKPC* gene | CGGCAGCAGTTTGTTGATTG | NG_078054.1 |
| SEQ ID NO:83 | Downstream primer of *blaKPC* gene | CAGACGACGGCATAGTCATTT | |
| SEQ ID NO:84 | Probe of *blaKPC* gene | AAACCGGAACCTGCGGAGTGT ATG | |
| SEQ ID NO:85 | Upstream primer of *blaCTX-M* gene | CGTGGACTGTGGGTGATAAG | NZ_CP035775.1(3 1113-31988) |
| SEQ ID NO:86 | Downstream primer of *blaCTX-M* gene | TGTTGCGGCTGGGTAAA | |
| SEQ ID NO:87 | Probe of *blaCTX-M* gene | TATCATTGGTGGTGCCGTAGTC GC | |
| SEQ ID NO:88 | Upstream primer of *blaOXA-23* gene | TGTATTAGCCTTATCGGCTGTG | PP296992.1 |
| SEQ ID NO:89 | Downstream primer of *blaOXA-23* gene | ACAACTACGCCCTGTGATTT | |
| SEQ ID NO:90 | Probe of *blaOXA-23* gene | ATTATCGGAATGCCTGCGGTAG CA | |
| SEQ ID NO:91 | Upstream primer of *blaNDM-1* gene | CAACTGGATCAAGCAGGAGAT | NZ_CP009115.1(3 0287-31099) |
| SEQ ID NO:92 | Downstream primer of *blaNDM-1* gene | CGACAACGCATTGGCATAAG | |
| SEQ ID NO:93 | Probe of *blaNDM-1* gene | CAGGACAAGATGGGCGGTATG GAC | |
| SEQ ID NO:94 | Upstream primer of *blaTEM* gene | TTTCCGTGTCGCCCTTATTC | NZ_CP026359.1(4 643191-4644048) |
| SEQ ID NO:95 | Downstream primer of *blaTEM* gene | CCCAACTGATCTTCAGCATCTT | |
| SEQ ID NO:96 | Probe of *blaTEM* gene | TCACCCAGAAACGCTGGTGAAA GT | |
| SEQ ID NO:97 | Upstream primer of *blaSHV* gene | GCCGCAGAGCACTACTTTA | NZ_JAPDUA0100 00015.1(begin-451 ) |
| SEQ ID NO:98 | Downstream primer of *blaSHV* gene | CCGCGTAGGCATGATAGAAA | |
| SEQ ID NO:99 | Probe of *blaSHV* gene | AGGTGCTCATCATGGGAAAGCG TT | |
| SEQ ID NO: 100 | Upstream primer of *blaVIM-1* gene | ATCACCATCACGGACAATGAG | NZ_CP032176.1(6 5291-66091) |
| SEQ ID NO:101 | Downstream primer of *blaVIM-1* gene | GAGGTCCGACTTTACCAGATTG | |
| SEQ ID NO:102 | Probe of *bla VIM-1* gene | CAAACGACTGCGTTGCGATATG CG | |
| SEQ ID NO: 103 | Upstream primer of *blaVIM-2* gene | GAACTCTTCTATCCTGGTGCTG | NZ_CAADPS0100 01021.1(58-858) |
| SEQ ID NO:104 | Downstream primer of *blaVIM-2* gene | CGTGCGTGACAACTCATAAATC | |
| SEQ ID NO:105 | Probe of *blaVIM-2* gene | CTGCGAGTGTGCTCTATGGTGG TT | |
| SEQ ID NO: 106 | Upstream primer of *blaIMP-1* gene | CGGGACACACTCCAGATAAC | NZ_BFEO0100006 3.1(3398-4138) |
| SEQ ID NO:107 | Downstream primer of *blaIMP-1* gene | CGTCACCCAAATTGCCTAAAC | |
| SEQ ID NO: 108 | Probe of *blaIMP-1* gene | AGTGGTTTGGTTGCCTGAAAGG AA | |
| SEQ ID NO:109 | Upstream primer of *blaIMP-4* gene | CCGTACGGTCTAGGTAATTTGG | NZ_CAIZUM0100 00106.1(185-925) |
| SEQ ID NO:110 | Downstream primer of *blaIMP-4* gene | CAGCTTCACTGTGACTTGGA | |
| SEQ ID NO:111 | Probe of *blaIMP-4* gene | AGAAGCTTGGCCAAAGTCCGCT AA | |
| SEQ ID NO:112 | Upstream primer of *SmeDEF* gene | GGAAAGCGACTATACCCACTAC | NZ_JAGMRQ010 000085.1() |
| SEQ ID NO:113 | Downstream primer of *SmeDEF* gene | CCCTTCACTGCGGTTATCTT | |
| SEQ ID NO: 114 | Probe of *SmeDEF* gene | TCAGCGTGGCGGAAGAAGAGT TT | |
| SEQ ID NO:115 | Upstream primer of *Sull* gene | GCCGATGAGATCAGACGTATTG | NZ_BFEN0100003 0.1(14761-15600) |
| SEQ ID NO:116 | Downstream primer of *Sull* gene | CCTTGGATATCGTTCAGGTAGC | |
| SEQ ID NO:117 | Probe of *Sul1* gene | TCAATCGACAGCTTCCAACCGG AA | |
| SEQ ID NO:118 | Upstream primer of *QnrA* gene | GAAGGGTGTCACTTCAGCTATG | NZ_CABVNN010 000088.1(129-785 |
| SEQ ID NO:119 | Downstream primer of *QnrA* gene | TCGCACTCCCTGAACTCTAT | |
| SEQ ID NO:120 | Probe of *QnrA* gene | TTTCAAGGCCTGCCGTCTGTCTT T | |
| SEQ ID NO:121 | Upstream primer of *OnrB* gene | CTTTCAGCATCGCACGACTAA | NZ_JAOEBO0100 00088.1(825-1649) |
| SEQ ID NO:122 | Downstream primer of *OnrB* gene | CCTGAGCGGCACTGAATTTAT | |
| SEQ ID NO: 123 | Probe of *QnrB* gene | ATTGCACCCTTTCTGGCTTTCAC G | |
| SEQ ID NO:124 | Upstream primer of *gyrB* gene | CGAGGAGTACAACATCGACAA G | NZ_FRJY0100002 1.1(173901-17632 1) |
| SEQ ID NO:125 | Downstream primer of *gyrB* gene | GCGGAAGAAGAAGGTCAACA | |
| SEQ ID NO:126 | Probe of *gyrB* gene | ATCATGACCGATGCTGACGTCG AC | |
| SEQ ID NO:127 | Upstream primer of *gyrA* gene | CTGCGTGACGAGTCTGATAAA | NZ_FBRD010000 03.1(45550-48183) |
| SEQ ID NO:128 | Downstream primer of *gyrA* gene | AGCTGGGTCTGGGAATAGA | |
| SEQ ID NO:129 | Probe of *gyrA* gene | CGCATCGCGCTTCACTTCAATC AC | |
| SEQ ID NO: 130 | Upstream primer of *Aac(6')-Ib-cr* gene | ATCCAGGAGTACGCGGAATA | DAGUQY0100000 94.1(begin-296) |
| SEQ ID NO:131 | Downstream primer of *Aac(6')-Ib-cr* gene | TGAACAGCAACTCAACCAGAG | |
| SEQ ID NO:132 | Probe of *Aac(6')-Ib-cr* gene | CATCACAACTGGGCAAAGGCTT GG | |
| SEQ ID NO:133 | Upstream primer of *ermB* gene | GCACACTCAAGTCTCGATTCA | NZ_LR135484.1(4 2511-43248) |
| SEQ ID NO: 134 | Downstream primer of *ermB* gene | GTGGTATGGCGGGTAAGTTT | |
| SEQ ID NO:135 | Probe of *ermB* gene | TGCCAGCGGAATGCTTTCATCC TA | |
| SEQ ID NO:136 | Upstream primer of *MefA* gene | GCTACCTGTCTGGATGGTTATG | NC_023287.1 (65292-66509) |
| SEQ ID NO:137 | Downstream primer of *MefA* gene | GCATTTCGTTAGCTGCTCTTC | |
| SEQ ID NO:138 | Probe of *MefA* gene | CCCAGCACTCAATTCGGTTACA CCA | |
| SEQ ID NO:139 | Upstream primer of *cfr* gene | CATGGATACCAGCAGACTTCA | AJ879565.1(1144.. 2193 ) |
| SEQ ID NO:140 | Downstream primer of *cfr* gene | CCTACAATAAGTGCACCTGAGA | |
| SEQ ID NO: 141 | Probe of *cfr* gene | TACCTGCCCTTCGTTTGCTTCTC C | |
| SEQ ID NO: 142 | Upstream primer of *aac(3')-VI* gene | CACACCCGACCTTGGAATAG | NC_020528.1(248 0002-2480820) |
| SEQ ID NO:143 | Downstream primer of *aac(3')- VI* gene | GCAGGCTGTCTGATGTGAT | |
| SEQ ID NO:144 | Probe of *aac(3')-VI* gene | TTCTGGCGTCTGGAGAATGTGA CG | |
| SEQ ID NO: 145 | Upstream primer of *aph(3 ')-VI* gene | CATGGCGATATCACGGATAGTA A | NC_019045.2(115 543-116322) |
| SEQ ID NO:146 | Downstream primer of *aph(3 ')-VI* gene | CTTAGGCAACGTTCAACAAAGG | |
| SEQ ID NO:147 | Probe of *aph(3')-VI* gene | TTTAGATCTTGGCCGTGCTGGG TT | |
| SEQ ID NO: 148 | Upstream primer of *ant(2'')-Ia* gene | GTCATGGAGGAGTTGGACTATG | NC_024975.1(120 19-12552) |
| SEQ ID NO: 149 | Downstream primer of *ant(2'')-Ia* gene | AGCCTCCGCGATTTCATAC | |
| SEQ ID NO:150 | Probe of *ant(2 '')-Ia* gene | TACTTGACTGCGAACCTGCTTG GT | |
| SEQ ID NO:151 | Upstream primer of *DfrA1* gene | CGGGAATGGCCCTGATATT | NC_032101.1(272 95-27758) |
| SEQ ID NO:152 | Downstream primer of *DfrA1* gene | GCATACTTTCGGTTGGGTAATG | |
| SEQ ID NO:153 | Probe of *DfrA1* gene | AATGGCTGTTGGTTGGACGCAA G | |
| SEQ ID NO:154 | Upstream primer of *catA1* gene | GACGGTGAGCTGGTGATATG | NC_023898.1(133 246-133905) |
| SEQ ID NO:155 | Downstream primer of *catA1* gene | CCGGAAATCGTCGTGGTATT | |
| SEQ ID NO:156 | Probe of *catA1* gene | ACGGTGTAACAAGGGTGAACA CTATCC | |
| SEQ ID NO: 157 | Upstream primer of *tetA* gene | GGTAGCCGGCGCTTATATT | AJ313332.1 (990-2 189) |
| SEQ ID NO:158 | Downstream primer of *tetA* gene | CATCCCGAACCCGAAACA | |
| SEQ ID NO:159 | Probe of *tetA* gene | ATATCACTGATGGCGATGAGCG CG | |
| SEQ ID NO:160 | Upstream primer of *tetB* gene | AACGGTGTGGGTGCTATTT | AB089588.1 |
| SEQ ID NO:161 | Downstream primer of *tetB* gene | AAGACCAAGACCCGCTAATG | |
| SEQ ID NO:162 | Probe of *tetB* gene | TGGATGGAATAGCATGATGGTT GGCT | |
| SEQ ID NO:163 | Upstream primer of *B2M* gene | GCAAGGACTGGTCTTTCTATCT CT | NC_000015.10 (44711517-44718 145) |
| SEQ ID NO:164 | Downstream primer of *B2M* gene | AYCCCACTTAACTATCTTGGGC | |
| SEQ ID NO: 165 | Probe of *B2M* gene | TGCCTGCCGTGTGAACCATGTG ACT | |

**Table 6: Examples of fluorescently labeled probes used in this application.**

| Name of Bacterium | Probe | 5' end labelling | 3' end labelling |
|---|---|---|---|
| *mecA* gene | SEQ ID NO:69 | FAM | BHQ1 |
| *mecC* gene | SEQ ID NO:72 | FAM | BHQ1 |
| *vanA* gene | SEQ ID NO:75 | HEX | BHQ1 |
| *vanB* gene | SEQ ID NO:78 | FAM | BHQ1 |
| *MCR-1* gene | SEQ ID NO:81 | FAM | BHQ1 |
| | | HEX | BHQ1 |
| *blaKPC* gene | SEQ ID NO:84 | HEX | BHQ1 |
| | | FAM | BHQ1 |
| *blaCTX-M* gene | SEQ ID NO:87 | FAM | BHQ1 |
| | | HEX | BHQ1 |
| *blaOXA-23* gene | SEQ ID NO:90 | FAM | BHQ1 |
| | | HEX | BHQ1 |
| *blaNDM-1* gene | SEQ ID NO:93 | HEX | BHQ1 |
| *blaTEM* gene | SEQ ID NO:96 | FAM | BHQ1 |
| | | HEX | BHQ1 |
| *blaSHV* gene | SEQ ID NO:99 | FAM | BHQ1 |
| | | HEX | BHQ1 |
| *blaVIM-1* gene | SEQ ID NO:102 | HEX | BHQ1 |
| *blaVIM-2* gene | SEQ ID NO:105 | FAM | BHQ1 |
| | | HEX | BHQ1 |
| *blaIMP-1* gene | SEQ ID NO:108 | ROX | BHQ2 |
| *blaIMP-4* gene | SEQ ID NO:111 | FAM | BHQ1 |
| | | HEX | BHQ1 |
| *SmeDEF* gene | SEQ ID NO:114 | ROX | BHQ2 |
| | | CY5 | BHQ2 |
| *Sull* gene | SEQ ID NO:117 | ROX | BHQ2 |
| | | CY5 | BHQ2 |
| *QnrA* gene | SEQ ID NO:120 | HEX | BHQ1 |
| *QnrB* gene | SEQ ID NO:123 | ROX | BHQ2 |
| | | CY5 | BHQ2 |
| *gyrB* gene | SEQ ID NO:126 | Cy5 | BHQ2 |
| *gyrA* gene | SEQ ID NO:129 | ROX | BHQ2 |
| *Aac(6')-Ib-cr* gene | SEQ ID NO:132 | CY5.5 | BHQ3 |
| *ermB* gene | SEQ ID NO:135 | ROX | BHQ2 |
| | | CY5 | BHQ2 |
| *MefA* gene | SEQ ID NO:138 | CY5 | BHQ2 |
| *cfr* gene | SEQ ID NO:141 | ROX | BHQ2 |
| | | CY5 | BHQ2 |
| *aac(3')-VI* gene | SEQ ID NO:144 | ROX | BHQ2 |
| | | CY5 | BHQ2 |
| *aph(3')-VI* gene | SEQ ID NO:147 | ROX | BHQ2 |
| | | CY5 | BHQ2 |
| *ant(2")-Ia* gene | SEQ ID NO:150 | ROX | BHQ2 |
| | | CY5 | BHQ2 |
| *DfrA1* gene | SEQ ID NO:153 | ROX | BHQ2 |
| | | CY5 | BHQ2 |
| *catA1* gene | SEQ ID NO:156 | CY5 | BHQ2 |
| *tetA* gene | SEQ ID NO:159 | ROX | BHQ2 |
| *tetB* gene | SEQ ID NO:162 | CY5.5 | BHQ3 |
| Internal reference (human) | SEQ ID NO: 165 | Cy5.5 | BHQ3 |

### EXAMPLES

### Example 1: Detection Kit for Multiple Pathogens and Drug-resistance Genes

### 1.1 Detection kits for multiple pathogens in bloodstream infections

This application develops a digital PCR detection kit for detecting 21 pathogens plus one internal reference gene in bloodstream infections. The above kit comprises primer-probe mixture 1, primer-probe mixture 2, digital PCR premix (digital PCR buffer, dNTPs, MgCl₂, Taq hot start enzyme mixture, glycerol, betaine), negative quality control and positive quality control.

Each sample needs to be tested twice. The primer-probe mixture and digital PCR premix in the kit are used to generate digital PCR droplets and perform PCR amplification for the sample to be tested. Particularly, the volume of primer-probe mixture is 2 µL, the volume of digital PCR premix is 10 µL, the amount of DNA added is 8 µL, and the total reaction volume is 20 µL. The reaction system for digital PCR amplification is shown in Tables 7-8:

**Table 7: Multiple digital reaction system of Group 1**

| Reagent | Volume for a single reaction (µL) |
|---|---|
| Digital PCR premix (Probe method) | 10 |
| Mixture of primers and probes (Group 1) | 2 |
| Sample DNA, or positive quality control, or negative quality control | 8 |
| Total | 20 |

**Table 8: Multiple digital reaction system of Group 2**

| Reagent | Volume for a single reaction (µL) |
|---|---|
| Digital PCR premix (Probe method) | 10 |
| Mixture of primers and probes (Group 2) | 2 |
| Sample DNA, or positive quality control, or negative quality control | 8 |
| Total | 20 |

The components of the kit in this application are shown in Table 9.

**Table 9: Components of the Kit**

| Name of component | Main components |
|---|---|
| Digital PCR premix | dNTP, Taq DNA polymerase, etc. |
| Mixture of primers and probes (Group 1) | Primers and probes for *Staphylococcus capitis,* primers and probes for *Acinetobacter baumannii,* primers and probes for *Candida tropicalis,* primers and probes for *Candida krusei,* primers and probes for *Staphylococcus epidermidis,* primers and probes for *Enterococcus faecium,* primers and probes for *Cryptococcus neoformans,* primers and probes for *Stenotrophomonas maltophilia,* primers and probes for *Klebsiella pneumoniae,* primers and probes for *Streptococcus pneumoniae,* primers and probes for *Serratia marcescens* |
| Mixture of primers and probes (Group 2) | Primers and probes for *Bacteroides fragilis,* Primers and probes for *Escherichia coli,* Primers and probes for *Candida parapsilosis,* Primers and probes for *Candida glabrata,* Primers and probes for *Enterobacter cloacae* complex, Primers and probes for *Enterococcus faecalis,* Primers and probes for *Candida albicans,* Primers and probes for *Pseudomonas aeruginosa,* Primers and probes for *Haemophilus influenzae,* Primers and probes for *Staphylococcus aureus,* Primers and probes for internal reference gene |
| Positive quality control | *Staphylococcus capitis* DNA, *Acinetobacter baumannii* DNA , *Candida tropicalis* DNA, *Candida krusei* DNA, *Staphylococcus epidermidis* DNA, *Enterococcus faecium* DNA, *Cryptococcus neoformans* DNA, *Stenotrophomonas maltophilia* DNA, *Klebsiella pneumoniae* DNA, *Streptococcus pneumoniae* DNA, *Serratia marcescens* DNA, *Bacteroides fragilis* DNA, *Escherichia coli* DNA, *Candida parapsilosis* DNA, *Candida glabrata* DNA, *Enterobacter cloacae* complex DNA, *Enterococcus faecalis* DNA, *Candida albicans* DNA, *Pseudomonas aeruginosa* DNA, *Haemophilus influenzae* DNA, *Staphylococcus aureus* DNA, Interal reference gene DNA |
| Negative quality control | *Citrobacter freundii* DNA, *Staphylococcus pasteuri* DNA, *Acinetobacter calcoaceticus* DNA, *Pseudomonas fluorescens* DNA, *Staphylococcus simulans* DNA, Enterobacter aerogenes DNA |

The kit according to this application incorporates an internal quality control system to monitor the sample collection and extraction process to avoid false negatives. The kit in this application comprises negative and positive quality controls for monitoring digital PCR amplification and microarray reading of samples, and to assist in the interpretation of the final results. The specific steps are as follows:
1) releasing the nucleic acid from the sample to be tested;
2) in one well of the microfluidic chip, using the nucleic acid obtained in step 1) as a template, adding a mixture of primers and fluorescently labeled probes (corresponding to Group 1) for 11 pathogens and digital PCR buffer;
3) in another well of the microfluidic chip, using the nucleic acid obtained in step 1) as a template, adding another mixture of primers and fluorescently labeled probes (corresponding to Group 2) for 10 pathogens, a mixture of internal reference primers and fluorescently labeled probes, and digital PCR buffer;
4) performing digital PCR droplet-generation and PCR amplification in a digital PCR amplification instrument;
75 µl of oil-phase coated reagent was added to the oil wells of the high-throughput digital PCR chip, and 20 µl of amplification mixture was added to the reagent wells, then sealing the chip, and placing it on a PCR amplification instrument to generate droplets and perform PCR amplification.

The amplification reaction was performed according to the following recommended amplification procedure:

| Reaction steps | Reaction temperature | Reaction time | Number of cycles |
|---|---|---|---|
| Pre-denaturation | 95°C | 10 min | 1 |
| Denaturation | 94°C | 30 s | 40 |
| Annealing, extension | 60°C | 60 s | |
| Inactivation | 98°C | 10 min | 1 |
| Cooling | 20°C | 2 min | 1 |

### 5) Signal collection

After amplification by using the nucleic acid obtained in step 4) as a template, the chip was used as the detection material. Fluorescence was detected by a biochip reader, and the results were interpreted by the presence and intensity of fluorescence in each microdroplet.

Result interpretation:
The results are shown in Figs. 2A-2F. In Figs. 2A-2F, the markers for the 11 bacteria detected in the first well and the 10 bacteria detected in the second well plus one internal reference are shown in Table 10.

**Table 10:**

| | Fluorescence channel | Position 1 | Position 2 | Position 3 | Position 4 | Position 5 |
|---|---|---|---|---|---|---|
| The first well | FAM+HEX | *Staphylococcus capitis* | *Acinetobacter baumannii* | *Candida tropicalis* | *Candida krusei* | *Staphylococcus epidermidis* |
| | ROX+Cy5 | *Enterococcus faecium* | *Cryptococcus neoformans* | *Stenotrophomon as maltophilia* | *Klebsiella pneumoniae* | *Streptococcus pneumoniae* |
| | Cy5.5 | *Serratia marcescens* | | | | |
| The second well | Fluorescence channel | Position 1 | Position 2 | Position 3 | Position 4 | Position 5 |
| | FAM+HEX | *Bacteroides fragilis* | *Escherichia coli* | *Candida parapsilosis* | *Candida glabrata* | *Enterobacter cloacae* complex |
| | ROX+Cy5 | *Enterococcus faecalis* | *Haemophilus influenzae* | *Pseudomonas aeruginosa* | *Staphylococcus aureus* | *Candida albicans* |
| | Cy5.5 | Internal reference | | | | |

If, in the 2D graph, there are positive droplets within the positive droplet region (excluding negative droplets), and the number of droplets is greater than the threshold, then the test result for the corresponding strain at that position is determined to be positive. The test results are shown in Table 11 below:

**Table 11**

| Fluorescence probe | | The first well | The second well |
|---|---|---|---|
| FAM&HEX | 1# positive cluster | *Staphylococcus capitis* | *Bacteroides fragilis* |
| | 2# positive cluster | *Acinetobacter baumannii* | *Escherichia coli* |
| | 3# positive cluster | *Candida tropicalis* | *Candida parapsilosis* |
| | 4# positive cluster | *Candida krusei* | *Candida glabrata* |
| | 5# positive cluster | *Staphylococcus epidermidis* | *Enterobacter cloacae* complex |
| ROX&Cy5 | 1# positive cluster | *Enterococcus faecium* | *Enterococcus faecalis* |
| | 2# positive cluster | *Cryptococcus neoformans* | *Haemophilus influenzae* |
| | 3# positive cluster | *Stenotrophomonas maltophilia* | *Pseudomonas aeruginosa* |
| | 4# positive cluster | *Klebsiella pneumoniae* | *Staphylococcus aureus* |
| | 5# positive cluster | *Streptococcus pneumoniae* | *Candida albicans* |
| Cy5.5 | | *Serratia marcescens* | Interal reference gene |

### 1.2 Detection kit for multiple drug-resistance genes in bacterial pathogen infections

This application develops a digital PCR detection kit for detecting drug-resistance genes of 32 pathogens plus one internal reference gene in bacterial infections. The above kit comprises primer-probe mixture 1, primer-probe mixture 2, primer-probe mixture 3, digital PCR premix (digital PCR buffer, dNTPs, MgCl₂, Taq hot start enzyme mixture, glycerol, betaine), negative quality control and positive quality control.

Each sample needs to be tested in 3 sets. The primer-probe mixture and digital PCR premix in the kit are used to generate digital PCR droplets and perform PCR amplification in the sample to be tested. The volume of primer-probe mixture is 2 µL, the volume of digital PCR premix is 10 µL, the amount of DNA added is 8 µL, and the total reaction volume is 20 µL. The reaction system for digital PCR amplification is shown in Tables 7-8 above.

The components of the kit in this application are shown in Table 12.

**Table 12 Components of the kit**

| Name of component | Main components |
|---|---|
| Digital PCR premix | dNTP, Taq DNA polymerase, etc. |
| Mixture of primers and probes (Group 1) | Primers and probes for *mecA,* primers and probes for *vanA,* primers and probes for *MCR-1,* primers and probes for *blaKPC,* primers and probes for *blaNDM-1,* primers and probes for *blaIMP-1,* primers and probes for *SmeDEF,* primers and probes for *Sull,* primers and probes for *OnrB,* primers and probes for *gyrB,* primers and probes for *Aac(6')-Ib-cr* |
| Mixture of primers and probes (Group 2) | Primers and probes for *mecC,* primers and probes for *blaCTX-M,* primers and probes for *blaTEM,* primers and probes for *blaVIM-2,* primers and probes for *blaVIM-1,* primers and probes for *gyrA,* primers and probes for *ermB,* primers and probes for *aac(3')-VI,* primers and probes for *cfr,* primers and probes for *catA1,* primers and probes for *TetB* |
| Mixture of primers and probes (Group 3) | Primers and probes for *vanB,* primers and probes for *blaOXA-23,* primers and probes for *blaSHV,* primers and probes for *blaIMP-4,* primers and probes for *QnrA,* primers and probes for *TetA,* primers and probes for *aph(3')-VI,* primers and probes for *MefA,* primers and probes for *ant(2")-Ia,* primers and probes for *DfrA1,* primers and probes for internal reference (*B2M* gene) |
| Positive quality control | mecA plasmid, mecC plasmid, vanA plasmid, vanB plasmid, MCR-1 plasmid, blaKPC plasmid, blaCTX-M plasmid, blaOXA-23 plasmid, blaNDM-1 plasmid, blaTEM plasmid, blaSHV plasmid, blaVIM-1 plasmid, blaVIM-2 plasmid, blaIMP-1 plasmid, blaIMP-4 plasmid, SmeDEF plasmid, *Sul1* plasmid, QnrA plasmid, QnrB plasmid, gyrA plasmid, gyrB plasmid, Aac(6')-Ib-cr plasmid, ermB plasmid, TetA plasmid, TetB plasmid, aac(3')-VI plasmid, aph(3')-VI plasmid, ant(2")-Ia plasmid, *DfrA1* plasmid, *catA1* plasmid, MefA plasmid, cfr plasmid |
| Negative quality control | Nuclease-free Water |

The kit according to this application incorporates an internal quality control system to monitor the sample collection and extraction process to avoid false negatives. The kit in this application comprises negative and positive quality controls for monitoring digital PCR amplification and microarray reading of samples, and to assist in the interpretation of the final results. The specific steps are as follows:
1) releasing the nucleic acid from the sample to be tested;
2) in one well of the microfluidic chip, using the nucleic acid obtained in step 1) as a template, adding a mixture of primers and fluorescently labeled probes for 11 pathogen drug-resistance genes (corresponding to Group 1) and digital PCR buffer;
3) in another well of the microfluidic chip, using the nucleic acid obtained in step 1) as a template, add another mixture of primers and fluorescently labeled probes for 11 other pathogen drug-resistance genes (corresponding to Group 2) and digital PCR buffer;
4) in the third well of the microfluidic chip, using the nucleic acid obtained in step 1) as a template, add another mixture of primers and fluorescently labeled probes for 10 other pathogen drug-resistance genes (corresponding to Group 3), a mixture of internal reference primers and fluorescently labeled probes, and digital PCR buffer.
5) performing digital PCR droplet-generation and PCR amplification in a digital PCR amplification instrument;
75 µl of oil-phase coated reagent was added to the oil wells of the high-throughput digital PCR chip, and 20 µl of amplification mixture was added to the reagent wells, then sealing the chip, and placing it on a PCR amplification instrument to generate droplets and perform PCR amplification.

The amplification reaction was performed according to the following recommended amplification procedure:

| Reaction steps | Reaction temperature | Reaction time | Number of cycles |
|---|---|---|---|
| Pre-denaturation | 95°C | 10 min | 1 |
| Denaturation | 94°C | 30 s | 40 |
| Annealing, extension | 60°C | 60 s | |
| Inactivation | 98°C | 10 min | 1 |
| Cooling | 20°C | 2 min | 1 |

### 6) Signal collection

After amplification by using the nucleic acid obtained in step 4) as a template, the chip was used as the detection material. Fluorescence was detected by a biochip reader, and the results were interpreted by the presence and intensity of fluorescence in each microdroplet.

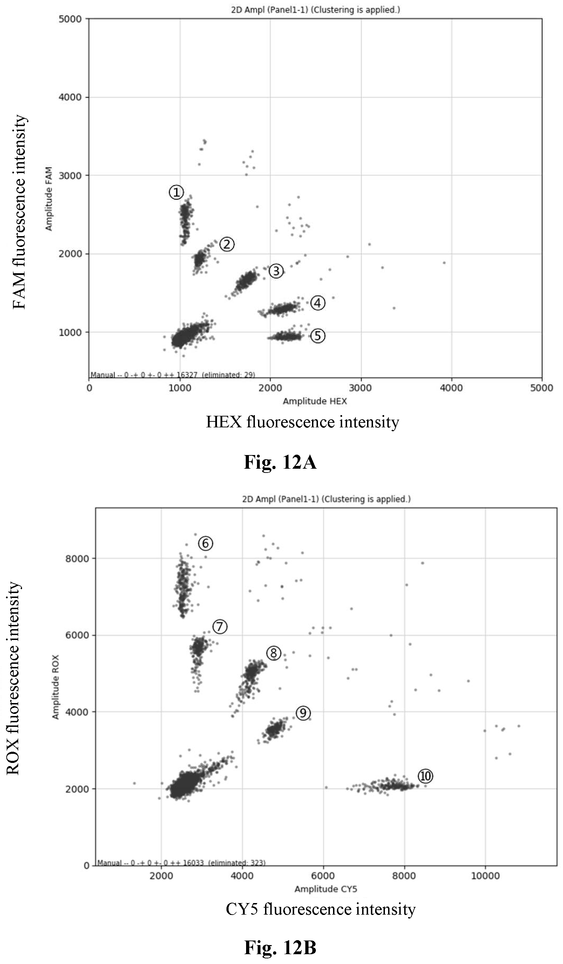

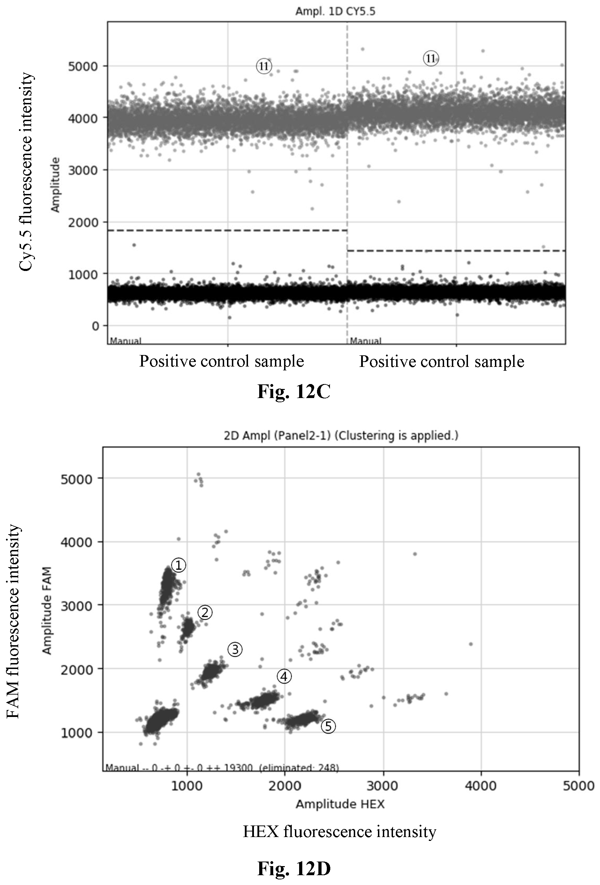

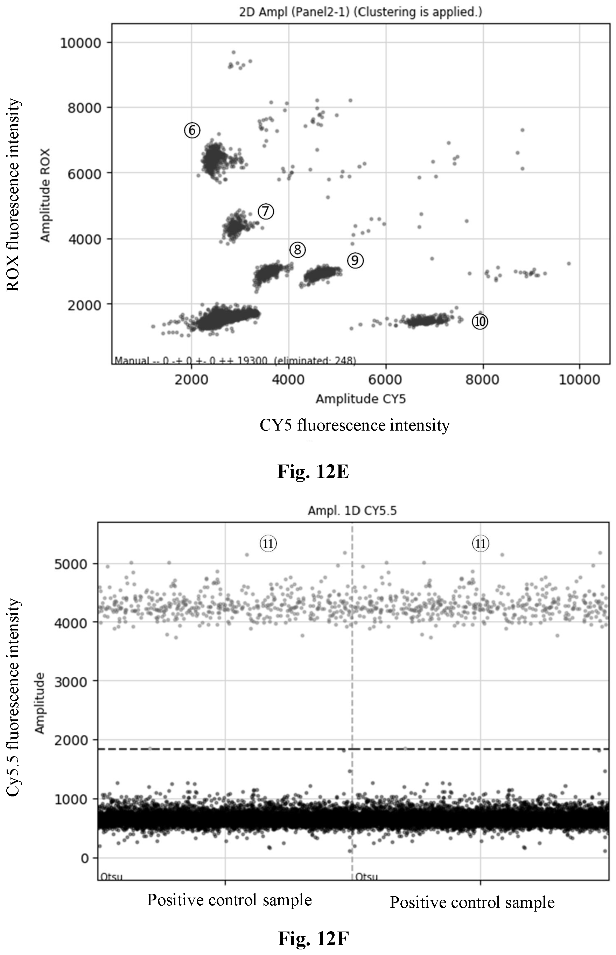

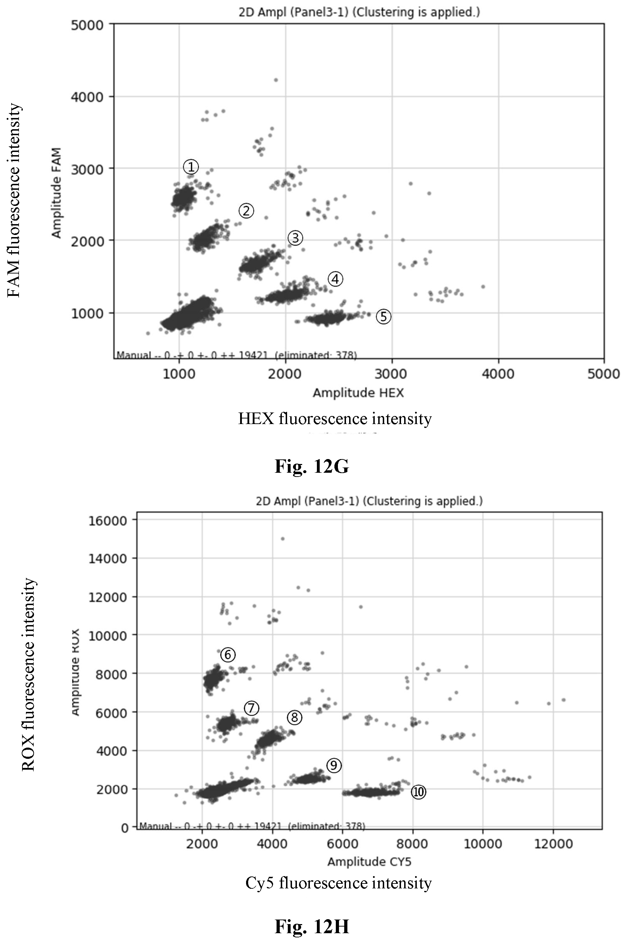

Result interpretation:
The results are shown in Fig. 12. Table 13 shows the markers for the 11 drug-resistance genes detected in the first well, the 11 drug-resistance genes detected in the second well, and the 10 drug-resistance genes detected in the third well, plus one internal reference.

**Table 13**

| | Fluorescence channel | Position 1 | Position 2 | Position 3 | Position 4 | Position 5 |
|---|---|---|---|---|---|---|
| The first well | FAM+HEX | *mecA* | *vanA* | *MCR-1* | *blaKPC* | *blaNDM-1* |
| | ROX+Cy5 | *blaIMP-1* | *SmeDEF* | *Sul1* | *QnrB* | *gyrB* |
| | Cy5.5 | *Aac(6')-Ib-cr* | | | | |

| | Fluorescence channel | Position 1 | Position 2 | Position 3 | Position 4 | Position 5 |
|---|---|---|---|---|---|---|
| The second well | FAM+HEX | *mecC* | *blaCTX-M* | *blaTEM* | *blaVIM-2* | blaVIM-1 |
| | ROX+Cy5 | *gyrA* | *ermB* | *aac(3')-VI* | *cfr* | *catA1* |
| | Cy5.5 | *TetB* | | | | |

| | Fluorescence channel | Position 1 | Position 2 | Position 3 | Position 4 | Position 5 |
|---|---|---|---|---|---|---|
| The third well | FAM+HEX | *vanB* | *blaOXA-23* | *blaSHV* | *blaIMP-4* | *QnrA* |
| | ROX+Cy5 | *TetA* | *aph(3 ')-VI* | *ant(2 ")-Ia* | *DfrA1* | *MefA* |
| | Cy5.5 | Internal reference | | | | |

If, in the 2D graph, there are positive droplets within the positive droplet region (excluding negative droplets), and the number of droplets is greater than the threshold, then the test result for the corresponding strain at that position is determined to be positive. The test results are shown in Table 14 below:

**Table 14**

| Fluorescence probe | | The first well | The second well | The third well |
|---|---|---|---|---|
| FAM&HEX | 1# positive cluster | *mecA* | *mecC* | *vanB* |
| | 2# positive cluster | *vanA* | *blaCTX-M* | *blaOXA-23* |
| | 3# positive cluster | *MCR-1* | *blaTEM* | *blaSHV* |
| | 4# positive cluster | *blaKPC* | *blaVIM-2* | *blaIMP-4* |
| | 5# positive cluster | *blaNDM-1* | *blaVIM-1* | *QnrA* |
| ROX&Cy5 | 1# positive cluster | *blaIMP-1* | *gyrA* | *TetA* |
| | 2# positive cluster | *SmeDEF* | *ermB* | *aph(3')- VI* |
| | 3# positive cluster | *Sull* | *aac(3')-VI* | *ant(2")-Ia* |
| | 4# positive cluster | *QnrB* | *cfr* | *DfrA1* |
| | 5# positive cluster | *gyrB* | *catA1* | *MefA* |
| Cy5.5 | Positive cluster | *Aac(6')-Ib-cr* | *TetB* | Internal reference |

| | | | | |
|---|---|---|---|---|
| Note: Schematic diagram of 32 drug-resistance genes and 1 internal reference detected in 3 wells. 11 drug-resistance genes were tested in the first well, another 11 drug-resistance genes were tested second well, and other 10 drug-resistance genes plus one internal reference were tested in the third well. | | | | |

### Example 2: Detection of Different Pathogens and Drug-Resistance Genes by Adjusting the Probe Ratio

### 2.1 Adjusting the probe ratio for the detection of different pathogens

Figs. 3A-3C show examples of how to adjust the position of *Candida tropicalis* positive droplet cluster by adjusting the ratio of FAM and HEX probes. In Fig. 3A, the ratio of FAM to HEX probes in the positive droplet clusters of *Candida tropicalis* is 1:3, the position is indicated by the box. Adjusting the ratio of FAM to HEX probes to 1.8:3 increases the concentration of FAM probes, and *Candida tropicalis* can be seen moving to the position indicated by the box in Fig. 3B. Fig. 3C shows the superposition of positive clusters of *Candida tropicalis* with a FAM:HEX probe concentration of 1:3 and a FAM:HEX probe concentration of 1.8:3, and it can be clearly seen that by adjusting the concentration ratio of FAM and HEX probes, the position of the positive droplet cluster in the 2D digital PCR detection graph can be moved. Figs. 3D-3F show examples of how to adjust the position of *Haemophilus influenzae* positive droplet clusters by adjusting the ratio of ROX and Cy5 probes. Fig. 3D shows a positive droplet image of *Haemophilus influenzae* with a ROX:Cy5 probe ratio of 4:1, the position is indicated by the box. Adjusting the ROX:Cy5 probe ratio to 5.6:1 increases the concentration of the ROX probe, as shown in Fig. 3E, the *Haemophilus influenzae* positive droplet clusters have moved to new positions, as indicated by the box. Fig. 3F shows the superposition of two positive droplet clusters with different ROX:Cy5 ratios, illustrating that by adjusting the ROX:Cy5 ratio, the effect of moving the *Haemophilus influenzae* positive droplet clusters in the 2D digital PCR detection graph can be achieved.

### 2.2 Adjusting the probe ratio for the detection of different drug-resistance genes

Figs. 13A-13C show examples of how to adjust the position of *mecA* resistance gene positive droplet cluster by adjusting the ratio of FAM and HEX probes. In Fig. 13A, the ratio of the *mecA* resistance gene positive droplet cluster FAM to the HEX probe is 1:2.5, as indicated by the arrow. Adjusting the ratio of FAM to HEX probes to 1:3.2 increases the concentration of FAM probes, it can be seen that the *mecA* resistance gene positive droplet cluster has moved to the position indicated by the arrow in Fig. 13B. Fig. 13C shows the superposition of *mecA* resistance gene positive droplet clusters with a FAM:HEX probe concentration of 1:2.5 and a FAM:HEX probe concentration of 1:3.2. It can be clearly seen that by adjusting the concentration ratio of FAM and HEX probes, the position of the positive droplet cluster in the 2D digital PCR detection graph can be moved (as shown by the arrows). Figs. 13D-13F show examples of how to move the position of *mecC* drug-resistance gene positive droplet cluster by adjusting the ratio of ROX and Cy5 probes. Fig. 13D shows a positive droplet cluster of *mecC* resistance gene with a ROX:Cy5 probe ratio of 4:1, as indicated by the arrow. Adjusting the ROX:Cy5 probe ratio to 5.5:1 increases the concentration of the ROX probe, as shown in Fig. 13E, the *mecC* drug-resistance gene positive droplet cluster has moved to a new position, as indicated by the arrow. Fig. 13F shows the superposition of two positive droplet clusters with different ROX:Cy5 ratios, illustrating that by adjusting the ROX:Cy5 ratio, the position of the *mecC* resistance gene positive droplet in the 2D digital PCR detection graph can be moved.

### Example 3: Testing the Detection Limit

This application tested the range of the detection limit of the primer-probe combination by diluting bacterial solutions of: *Bacteroides fragilis, Staphylococcus epidermidis, Enterococcus faecium, Streptococcus pneumoniae, Acinetobacter baumannii, Enterobacter cloacae, Enterococcus faecalis, Staphylococcus aureus, Klebsiella pneumoniae, Pseudomonas aeruginosa, Escherichia coli, Staphylococcus capitis, Stenotrophomonas maltophilia, Serratia marcescens, Haemophilus influenzae, Candida tropicalis, Cryptococcus neoformans, Candida krusei, Candida parapsilosis, Candida glabrata,* and *Candida albicans.*

### (1) Dilution of bacterial solutions

The above bacterial solutions were serially diluted to five concentrations (S9: 5×10⁶, S10: 5×10⁵, S11: 5×10⁴, S12: 5×10³, S13: 5×10², S14: 5.0, S15: 0.5, S16: 0 copies/mL), resulting in detection concentrations of 4×10⁴, 4×10³, 4×10², 4×10¹, 4, 0.4 and 0 copies/reaction, respectively. The formula for calculating the copy/reaction is: copies/mL x 8 µL. The loading volume for each digital PCR reaction is 8 microliters.

### (2) Formation of digital PCR droplets and amplification

Formation of digital PCR droplets and digital PCR amplification were performed by using the reaction system and procedure described in Example 1.

### (3) Signal collection

The digital PCR chip was removed from the digital PCR amplification instrument, then smoothly transferring to a biochip reader for signal collection.

### (4) Result interpretation and analysis:

Threshold lines are drawn based on the midpoint position between the negative and positive droplets of the positive quality control samples for each strain. If the number of positive droplets is greater than the threshold, the test result of the strain corresponding to the positive droplet is determined to be positive. The software provides the copy number for the positive droplet. A linear regression graph is plotted with dilution as the horizontal axis and logarithm of the measured value for each dilution as the vertical axis.

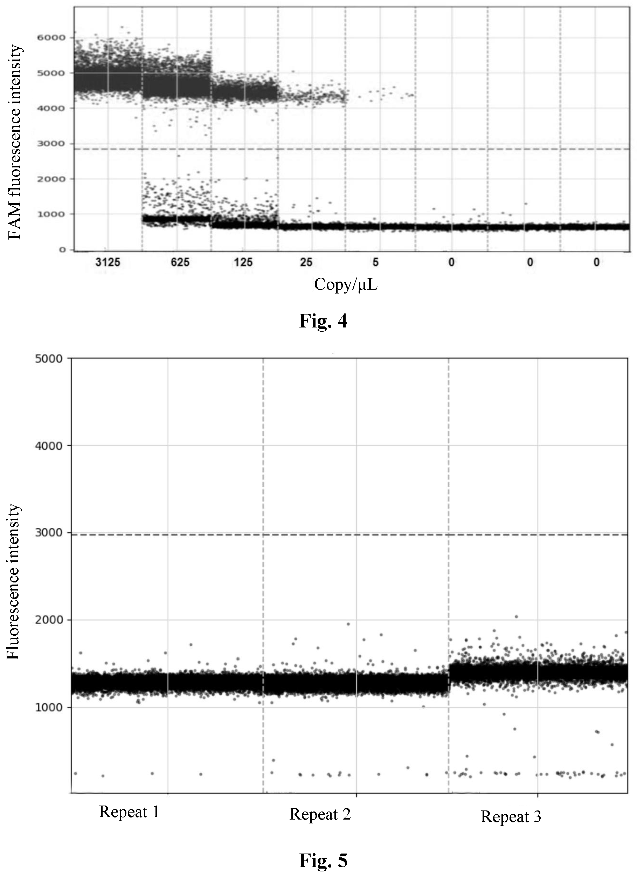

The linear range detection results of 21 bacterial solution samples after serial dilution are shown in Fig. 4. As can be seen from the results in Fig. 4, the detection limit of the detection kit according to this application is 4 copies/reaction, after calculating from the serial dilution results of the bacterial solutions. No positive droplet was detected in the detection well with 0 copies/mL. This demonstrates that the blank detection limit of the kit is 0 copies/mL.

### Example 4: Precision Test

This application utilizes plasmids comprising target fragments from *Bacteroides fragilis, Staphylococcus epidermidis, Enterococcus faecium, Streptococcus pneumoniae, Acinetobacter baumannii, Enterobacter cloacae, Enterococcus faecalis, Staphylococcus aureus, Staphylococcus hemolyticus, Klebsiella pneumoniae, Pseudomonas aeruginosa, Escherichia coli, Staphylococcus capitis, Stenotrophomonas maltophilia, Staphylococcus hominis, Candida tropicalis, Pneumocystis jirovecii, Cryptococcus neoformans, Candida krusei, Candida parapsilosis, Candida glabrata,* and *Candida albicans* to test the precision of the primer and probe combinations of this application.

Detection method: A high-concentration plasmid mixture (a synthetic plasmid mixture of 21 target pathogens, with a concentration of approximately 5×10⁶ copies/ml for each pathogen) was selected and diluted 10,000 times to approximately 5×10² copies/ml to prepare a low-concentration sample. Following the procedure in Example 3, the two concentration samples were measured continuously for 5 days, with four replicate tests conducted each day. The mean, standard deviation, and coefficient of variation (CV) of the calculated log values are given. The test results are as follows:

**Table 15: CV% of bacterial group**

| Strains and internal referen ce | *B. fragil is* | *S*. *epider midis* | *E. faeci um* | *S*. *pneumo niae* | *A. baum annii* | *E. cloaca e* | *E*. *faec alis* | *S*. *aure us* | Intern al refere nce | *K. pneumo niae* | *P*. *aerugi nosa* | *E. coli* | *S*. *capitis* | *S*. *malto philia* | *H. influe nzae* | *S*. *marce scens* |
|---|---|---|---|---|---|---|---|---|---|---|---|---|---|---|---|---|
| High concent ration | 0.31 | 0.30 | 0.30 | 0.30 | 0.27 | 0.27 | 0.28 | 0.30 | 0.30 | 0.30 | 0.32 | 0.32 | 0.43 | 0.43 | 0.42 | 0.42 |
| Low concent ration | 8.25 | 8.29 | 9.39 | 9.36 | 8.49 | 10.89 | 9.04 | 6.46 | 8.64 | 10.50 | 7.74 | 7.83 | 10.83 | 5.74 | 7.37 | 7.94 |

**Table 16: CV% of fungal group**

| Strains | *Candida tropicalis* | *Cryptococcus neoformans* | *Candida krusei* | *Candida parapsilosis* | *Candida glabrata* | *Candida albicans* |
|---|---|---|---|---|---|---|
| High concentration | 0.19 | 0.18 | 0.19 | 0.17 | 0.18 | 0.15 |
| Low concentration | 9.56 | 8.26 | 6.34 | 10.47 | 9.46 | 9.74 |

The CV values for high concentrations (5 × 10⁶ copies/ml) range from 0.15% to 0.43%, with an average CV of 0.275%.

The CV values for low concentrations (5 × 10² copies/ml) range from 5.74% to 10.89%, with an average CV of 8.87%.

### Example 5: Accuracy Test

This application utilizes plasmids comprising the target fragments of the following microorganisms: *Bacteroides fragilis*, *Staphylococcus epidermidis, Enterococcus faecalis*, *Streptococcus pneumoniae, Acinetobacter baumannii, Enterobacter cloacae, Enterococcus faecalis*, *Staphylococcus aureus, Klebsiella pneumoniae, Pseudomonas aeruginosa, Escherichia coli, Staphylococcus capitis, Stenotrophomonas maltophilia, Serratia marcescens, Haemophilus influenzae, Candida tropicalis, Cryptococcus neoformans*, *Candida krusei, Candida parapsilosis, Candida glabrata, Candida albicans,* and the internal reference gene, to test the accuracy of the primer-probe combinations in this application.

### (1) Plasmid dilution

The high-concentration standards of the above plasmids (5×10⁶ copies/ml) were diluted to 12 concentrations (2/5/10/20/50/100/200/500/1000/2000/5000/10000-fold), so that the detection concentrations were 2.5×10⁶, 1×10⁶, 5×10⁵, 2.5×10⁵, 1×10⁵, 5×10⁴, 2.5×10⁴, 1×10⁴, 5×10³, 2.5×10³, 1×10³, and 5×10² copies/reaction, respectively.

### (2) Formation of Digital PCR droplet and the amplification

Formation of Digital PCR droplet and the digital PCR amplification were performed by using the reaction system and procedure described in Example 1.

### (3) Signal collection

The digital PCR chips were removed from the digital PCR amplification instrument, and smoothly transferred to a biochip reader for signal collection.

Result interpretation and analysis:

**Table 17: Results of bacterial detection**

| Strains | Stock solution | 2X | 5X | 10X | 20X | 50X | 100X | 200X | 500X | 1000X | 2000X | 5000X | 10000X |
|---|---|---|---|---|---|---|---|---|---|---|---|---|---|
| *B. fragilis* | 0.013 | 0.013 | 0.018 | 0.013 | -0.045 | 0.021 | -0.023 | -0.023 | 0.018 | 0.002 | 0.023 | 0.33 | 0.351 |
| S. *epidermidis* | 0.014 | 0.004 | 0.026 | 0.023 | -0.024 | 0.044 | 0.011 | -0.065 | 0.203 | 0.066 | -0.086 | 0.389 | 0.051 |
| *E. faecium* | 0.016 | 0.015 | 0.018 | 0.035 | -0.015 | -0.014 | -0.017 | -0.098 | 0.038 | -0.014 | -0.02 | 0.534 | 0.059 |
| *S*. *pneumoniae* | -0.01 | -0.016 | -0.017 | -0.009 | -0.048 | -0.031 | -0.024 | -0.089 | 0.231 | -0.075 | 0.033 | -0.114 | -0.026 |
| *A. baumannii* | 0.057 | 0.061 | 0.042 | 0.037 | 0.002 | 0.016 | -0.075 | -0.131 | 0.068 | -0.009 | 0.01 | 0.021 | 0.033 |
| *E. cloacae* | 0.034 | 0.043 | 0.015 | 0.004 | -0.008 | 0.039 | -0.019 | -0.067 | -0.02 | 0.086 | -0.111 | 0.094 | 0.309 |
| *E. faecalis* | 0.055 | 0.053 | 0.011 | 0.022 | 0.005 | 0.051 | -0.026 | -0.021 | -0.049 | -0.075 | -0.162 | -0.122 | -0.287 |
| *S. aureus* | 0.051 | 0.053 | 0.039 | -0.006 | -0.023 | 0.03 | -0.092 | 0.024 | -0.028 | 0.003 | 0.147 | 0.189 | 0.024 |
| Internal reference | 0.035 | 0.051 | 0.008 | 0.022 | 0.007 | 0.039 | -0.02 | -0.104 | -0.052 | -0.018 | -0.211 | 0.1 | 0.187 |
| *K. pneumoniae* | 0.029 | 0.053 | 0.026 | 0.049 | 0.023 | 0.056 | 0.005 | -0.099 | -0.043 | -0.02 | 0.034 | 0.123 | -0.268 |
| *P*. *aeruginosa* | 0.047 | 0.038 | 0.018 | 0.026 | 0.036 | 0.067 | 0.007 | -0.115 | -0.09 | -0.534 | 0.204 | 0.266 | -0.028 |
| *E. coli* | 0.046 | 0.047 | 0.018 | 0.03 | 0.03 | 0.034 | -0.028 | -0.059 | 0.08 | 0.065 | -0.038 | -0.125 | 0.484 |
| *S. capitis* | 0.01 | 0.029 | 0.042 | 0.022 | 0.025 | 0.014 | -0.033 | -0.052 | -0.003 | 0.069 | 0.273 | 0.387 | -0.294 |
| *S*. *maltophilia* | 0.028 | 0.073 | 0.078 | 0.041 | 0.037 | 0.036 | 0.019 | 0.016 | 0.028 | -0.13 | -0.213 | 0.13 | 0.228 |
| *H. influenzae* | 0.011 | 0.033 | 0.041 | 0.03 | 0.033 | 0.024 | -0.004 | -0.027 | 0.105 | -0.045 | 0.102 | -0.158 | -0.316 |
| *S*. *marcescens* | 0.012 | 0.032 | 0.049 | 0.027 | 0.018 | -0.039 | 0.034 | 0.037 | 0.11 | 0.07 | 0.043 | 0.165 | 0.184 |

**Table 18 Results of Fungal Detection**

| Strains | Stock solution | 2X | 5X | 10X | 20X | 50X | 100X | 200X | 500X | 1000X | 2000X | 5000X | 10000X |
|---|---|---|---|---|---|---|---|---|---|---|---|---|---|
| *C. tropicalis* | 0.02 | -0 | -0 | -0.06 | -0.04 | -0.02 | -0 | -0.05 | 0.123 | -0.08 | 0.089 | -0.33 | -0 |
| *C. neoformans* | 0.01 | -0.01 | -0.02 | -0.06 | -0.06 | -0.04 | -0.03 | -0.19 | 0.019 | -0.04 | -0.08 | -0.64 | -0.31 |
| *C. krusei* | 0.01 | -0.01 | -0.01 | -0.06 | -0.03 | -0.02 | -0.04 | -0.1 | -0.11 | -0.04 | -0.14 | -0.04 | 0.156 |
| *C. parapsilosis* | 0 | -0.01 | -0.03 | -0.04 | -0.01 | -0.03 | 0.17 | 0.036 | -0.02 | -0.25 | -0.23 | -0.32 | -0.29 |
| *C. albicans* | -0.02 | -0.02 | -0.03 | -0.02 | -0.03 | -0.02 | -0.08 | -0.08 | 0.004 | -0.15 | -0.06 | 0.096 | -0.03 |

### Example 6: Specificity Detection

This application selects the following 115 strains for cross reaction: *Chromobacterium violaceum, Streptococcus intermedia, Bordetella bronchiseptica, Serratia marcescens, Streptococcus sobrinus, Listeria ivanovii, Burkholderia cepacia, Streptococcus anginosus, Peptostreptococcus anaerobius, Streptococcus sanguinis, Streptococcus constella subsp. constellatus, Veillonella Parvula, Streptococcus agalactiae, Listeria innocua, Corynebacterium striatum, Aeromonas sobria, Streptococcus salivarius, Aeromonas guinea, Raoultella terrigena (Klebsiella terrigena), Staphylococcus* capitis subsp. *capitis, Streptococcus dysgalactiae, Micrococcus luteus, Shigella sonnei, Staphylococcus sciuri, Listeria seeligeri, Rahnella aquatilis, Salmonella typhimurium, Lactobacillus acidophilus, Aeromonas hydrophila, Aggregatibacter aphrophilus, Legionella pneumophila subsp. pneumophila, Staphylococcus schleiferi subsp. schleiferi, Pseudomonas stutzeri, Enterobacter cancerogenus, Sphingomonas paucimobilis, Neisseria lactis, Staphylococcus carnosus, Pluralibacter gergoviae, Streptococcus canis, Enterococcus casseliflavus, Proteus mirabilis, Pseudomonas oryzihabitans, Bacteroides vulgatus, Acinetobacter pittii, Ralstonia pickettii, Streptococcus bovis, Eikenella corrodens, Streptococcus pyogenes, Neisseria meningitidis, Enterococcus durans, Staphylococcus xylose, Moraxella catarrhalis, Morganella morganii, Pseudomonas mendoza, Enterococcus cecum, Streptococcus equi subsp. Zooepidemicus, rhodococcus equi, Talaromyces marneffei, Gemella morbillorum, Bacteroides ovatus, Staphylococcus lugdunensis, Acinetobacter lwoffii*, *Haemophilus influenzae, Neisseria gonorrhoeae, Shigella dysenteriae, Plesiomonas shigelloides, Bacillus cereus, Bacillus subtilis, Streptococcus stomatitis, Acinetobacter radioresistens, Enterobacter kobei, Kingella kingae, Streptococcus gallolyticus, Staphylococcus pseudintermedius, Enterobacter hormaechei, Streptococcus mitis, Enterococcus hirae, Listeria grayi, Streptococcus gordonii, Neisseria sicca, streptococcus parasanguinis, Clostridium difficile, Bordetella parapertussis, Staphylococcus saprophyticus, Shigella flexneri, Yersinia frederiksenii*, *Hafnia alvei, Photorhabdus asymbiotica, Staphylococcus auricularis, Pseudomonas putida, Bacteroides thetaiotaomicron, Burkholderia multivorans, Pasteurella multocida, Parabacteroides distasonis, Bacillus licheniformis, Bacteroides uniformis*, *Listeria monocytogenes, Propionibacterium acnes, Vibrio vulnificus, Pantoea agglomerans, Salmonella enteritidis, Yersinia enterocolitica subsp. enterocolitica, Klebsiella oxytoca, Pseudomonas alcaligenes, Streptococcus mutans, Acinetobacter baylyi, Acinetobacter bereziniae, Shigella boydii*, *Actinobacillus actinomycetemcomitans, Cronobacter sakazakii, Bordetella pertussis, Enterococcus gallinarum, Clostridium perfringens, Staphylococcus warneri,* and *Haemophilus parainfluenzae.* These strains are pathogens with homology to the nucleic acid sequences of the target pathogens and are likely to cause the same or similar clinical symptoms. All of the above strains were confirmed not to carry drug-resistance genes.
(1) Releasing the nucleic acids of the test strains;
(2) Mixing the nucleic acids extracted from the above-mentioned strains;
(3) Formation of Digital PCR droplets and the amplification;
   Formation of Digital PCR droplets and digital PCR amplification were performed according to the reaction system and reaction procedure described in Example 1;
(4) Signal collection:
   The digital PCR chips were removed from the digital PCR amplification instrument, and smoothly transferred to a biochip reader for signal collection.
(5) Result interpretation and analysis:
   Threshold lines were drawn based on the midpoint between the negative and positive droplets of the positive quality control samples for each strain. If the number of positive droplets is greater than the threshold, the test result of the strain corresponding to the positive droplets is determined to be positive. The software provides the copy number corresponding to the positive droplets. A linear regression diagram was plotted with dilution as the horizontal axis and logarithm of the measured value for each dilution as the vertical axis.

The results of cross-reactivity detection for the 115 strains are shown in Figs. 5 and 14, respectively. No cross-reactivity was detected between these 115 strains and the 21 pathogen primers and probes in the kit. Figs. 5 and 14 comprise only negative droplet clusters; no positive droplet clusters were detected that cross-reacted with the 115 strains.

### Example 7: Detection of a Real Pathogen-Positive Sample with the Detection Kit

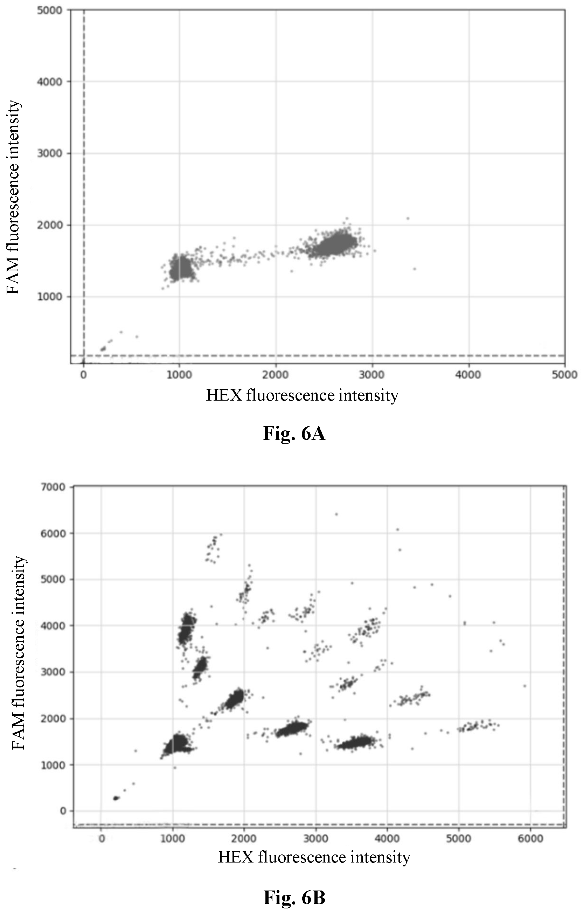

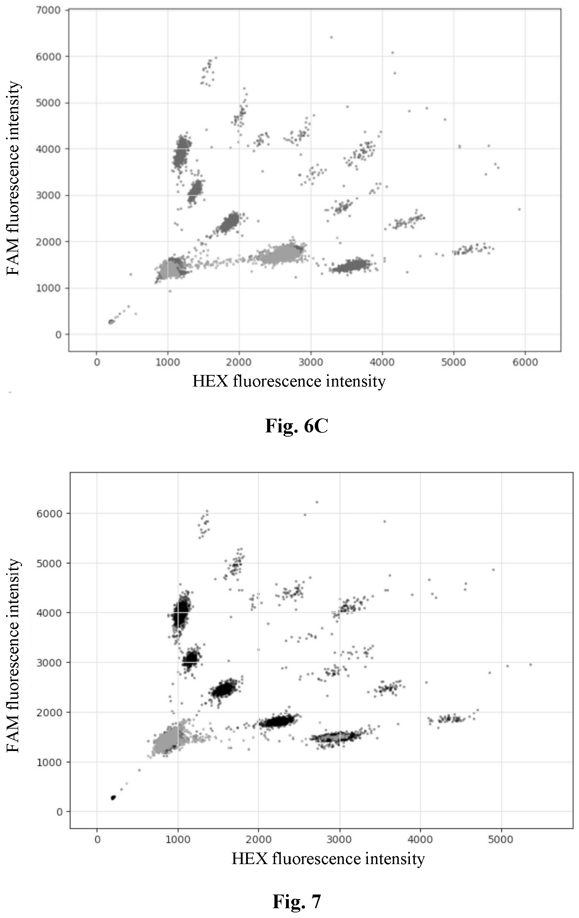

The clinical samples were tested according to the method in Example 1. Fig. 6A is a 2D graph of test results for the clinical samples, and Fig. 6B is a 2D graph of test results for the positive quality control samples. Fig. 6C shows the results of superimposing and comparing the 2D images of clinical samples and positive quality control samples. By superimposing and comparing, it can be seen that the position of the negative droplet cluster in the lower left corner of the clinical sample and the position of the negative droplet cluster in the positive quality control sample are superimposed. The position of the droplet cluster in the upper right corner of the negative droplet cluster in the 2D graph of the clinical sample and the position of *Klebsiella pneumoniae* in the positive quality control sample are superimposed, indicating that *Klebsiella pneumoniae* is detected in the clinical sample.

### Example 8: Detection of Whole Blood Samples from Patients with Bloodstream Infection by the Detection kit

Whole blood samples (1.8-2 mL each) were collected from 7 infected patients whose blood cultures were positive (confirmed bloodstream infection). After nucleic acid extraction, the detection kit for 21 pathogens was tested by digital PCR assay. The results of blood culture detection and the detection kit by digital PCR were compared, and the results are shown in Table 19. The blind test comparison of the two methods above shows that, the results of the detection kit for bloodstream infection by using digital PCR and those of the blood culture detection have high consistency. It can rapidly detect pathogens in a patient's blood within 5 hours by using 2mL of whole blood, avoiding the cumbersome steps of blood culture which requires 40-60ml (2-3 sets of blood culture bottles) and several days of culture time, thereby providing a rapid and effective method for detecting bloodstream infection pathogens. Meanwhile, due to factors such as sensitivity, culture medium selection, growth inhibition caused by competition between bacterial species, and inhibition caused by patients taking antibiotics in advance, blood culture detection can only detect one pathogen infection. Digital PCR can detect more pathogens than blood culture, indicating that the digital PCR method has higher detection sensitivity.

**Table 19: Comparison of results from blood culture detection and digital PCR detection kits**

| Sample No. | Results of blood culture | Results of digital PCR detection kit |
|---|---|---|
| 1 | *Streptococcus pneumoniae* | *Streptococcus pneumoniae* |
| 2 | *Enterococcus faecalis* | *Enterococcus faecalis* |
| 3 | *Escherichia coli* | *Escherichia coli* |
| 4 | *Staphylococcus aureus* | *Staphylococcus aureus, Pseudomonas aeruginosa , Pseudomonas maltophilia* |
| 5 | *Enterobacter cloacae* | *Enterobacter cloacae, Pseudomonas aeruginosa* |
| 6 | *Staphylococcus epidermidis* | *Klebsiella pneumoniae* |
| 7 | *Staphylococcus epidermidis* | *Staphylococcus epidermidis, Klebsiella pneumoniae , Pseudomonas aeruginosa* |

### Example 9: Detection of Other Samples from Infected Patients with the Detection Kit

Samples were collected from 6 patients with clinical symptoms of infection or suspected infection. The sample types included urine, ascites and cerebrospinal fluid with a collection volume of 3-5 ml, and 1 ml of sputum. Throat swabs and anal swabs were routinely stored in 2 ml of preservation solution. After nucleic acid extraction, 21 pathogens were detected with the pathogen detection kit described in Example 1 by digital PCR. Comparing the results of smear microscopy with those of the digital PCR detection kit, it can be seen that the detection results of bloodstream infection with digital PCR detection kit and those of the smear test have high consistency, and the kit can accurately identify specific bacterial species that cannot be identified by smear test.

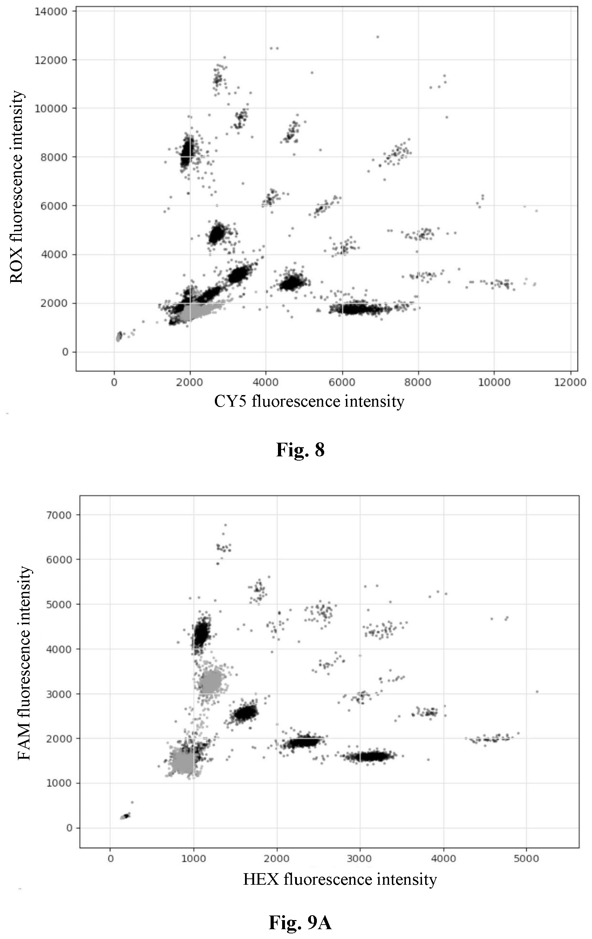

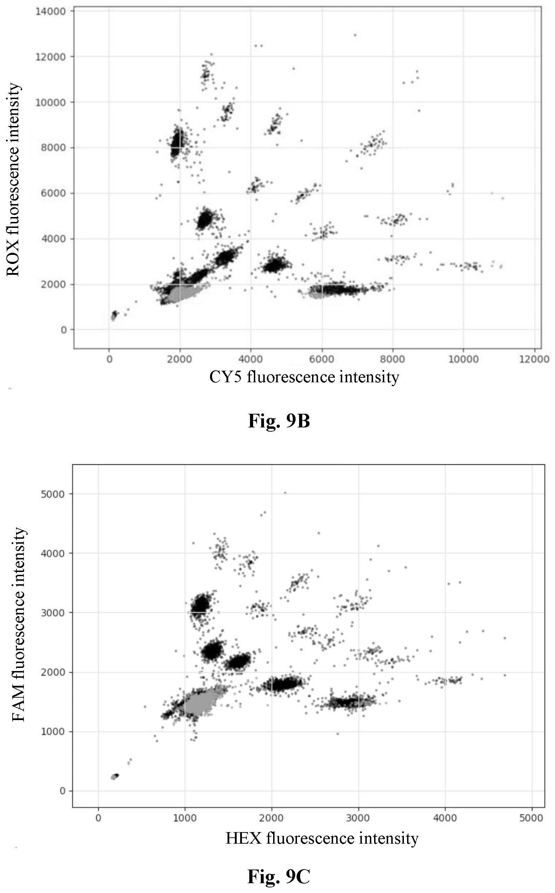

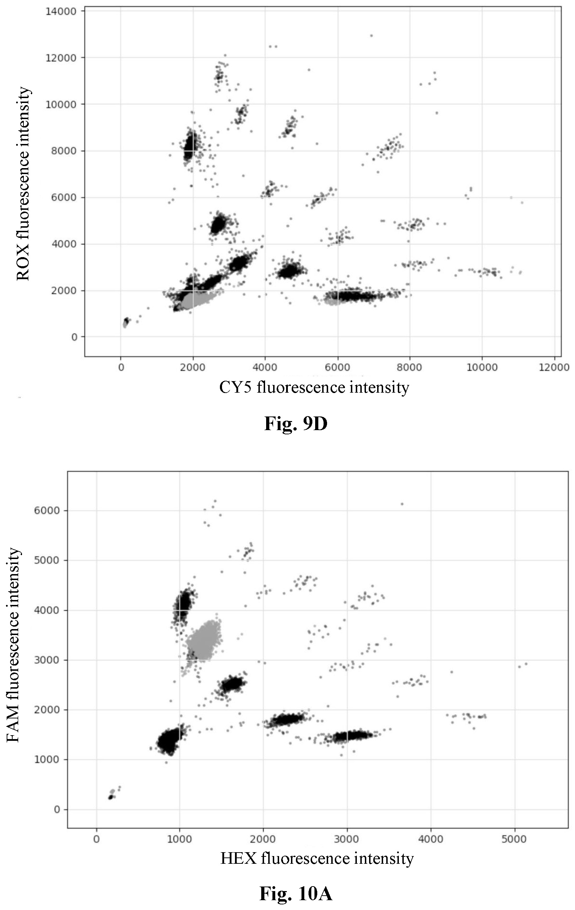

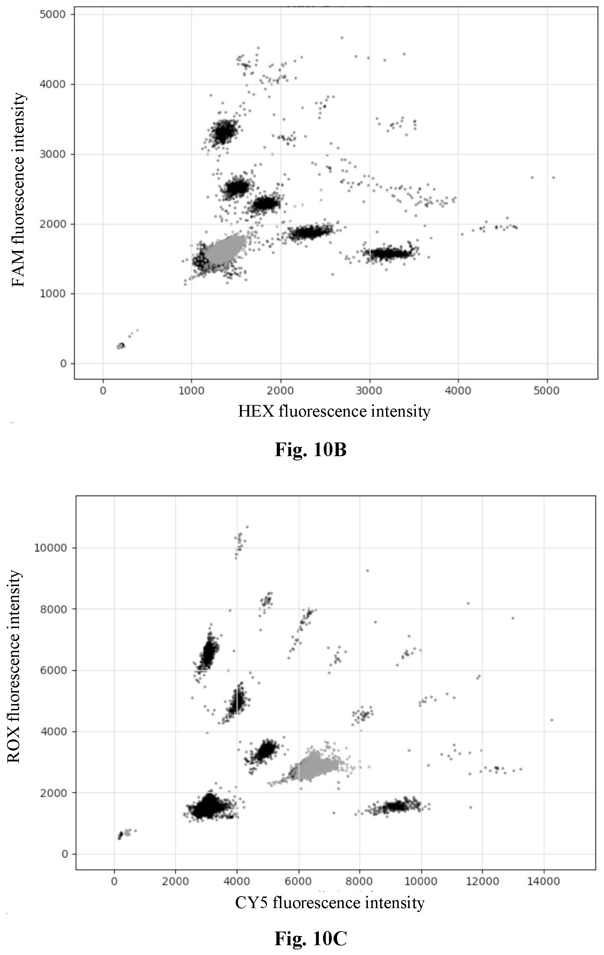

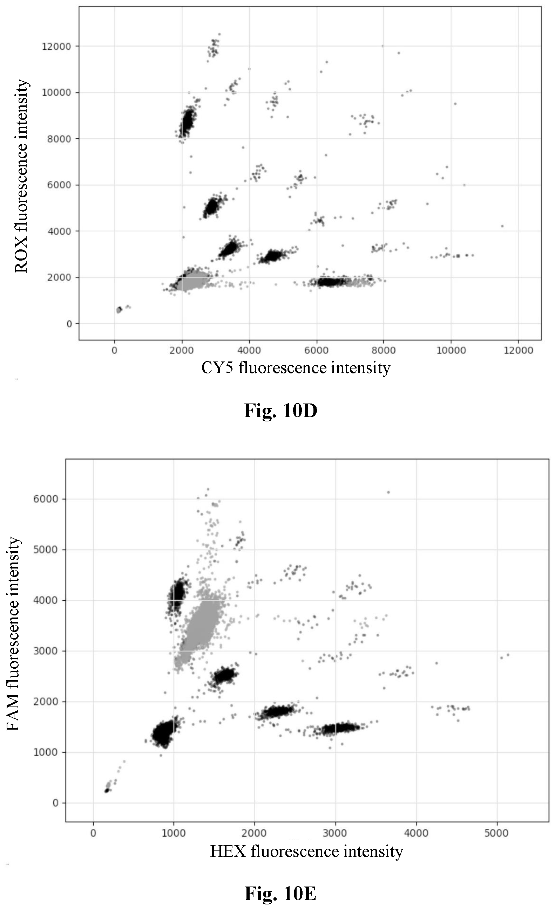

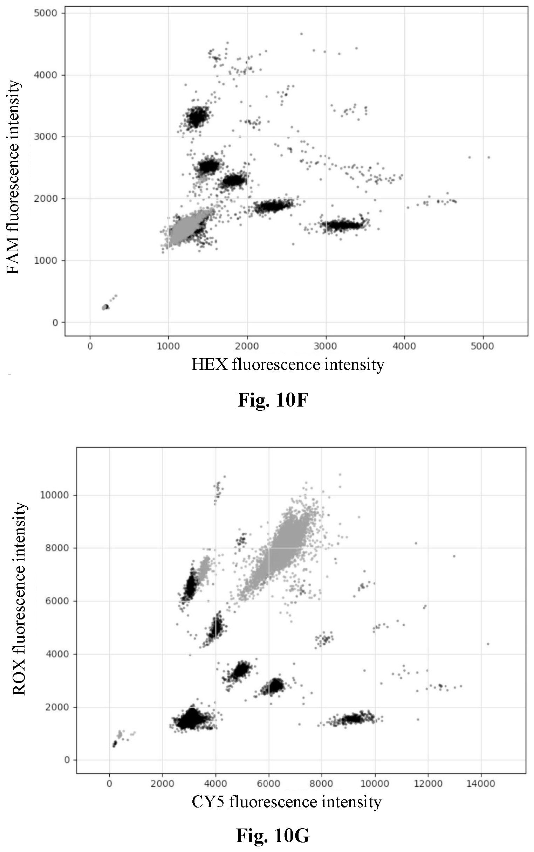

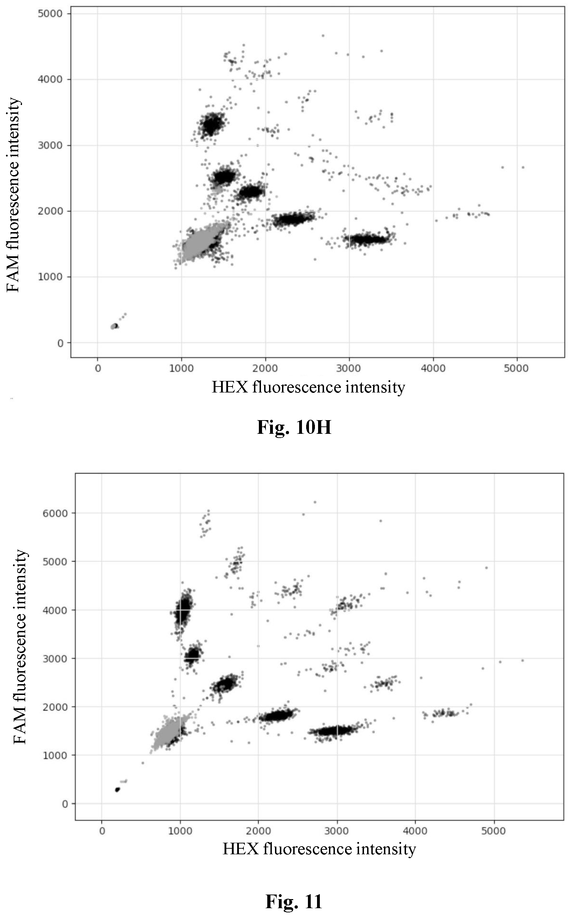

As shown in Fig. 7, the digital PCR result was positive for *Staphylococcus epidermidis.* The patient's sputum smear showed coccal infection, but the specific type of coccus could not be identified. Digital PCR specifically identified the coccus as *Staphylococcus epidermidis.* As shown in Fig. 8, both the digital PCR result and the cerebrospinal fluid smear result were negative, indicating that the results of the two detection methods were consistent. Figs. 9A-9D show that the kit according to this application can detect *Acinetobacter baumannii, Stenotrophomonas maltophilia* (a type of bacillus), *Enterobacter cloacae,* and *Candida albicans* in patient sputum samples; while sputum smear results only detect *Acinetobacter baumannii* or bacilli. The results above show that the smear method can only determine the presence of bacilli based on their morphology under a microscope after staining, but it cannot accurately identify which type of bacilli are causing the infection. Digital PCR, on the other hand, can accurately identify the type of bacilli causing the infection. Figs. 10A-10H show that *Candida albicans, Acinetobacter baumannii,* and *Klebsiella pneumoniae* were detected in both the patient's throat and anal swabs. The patient's anal swabs were tested positive for *Enterococcus faecium.* As shown in Fig. 11, urine culture revealed that the patient had an infection of *C*. *tropicalis.*

### Example 10: Detection of Samples of Strains Carrying Drug-resistance Gene with the Detection Kit

The Samples of strains carrying drug-resistance genes were tested according to the method in Example 1. All strain samples were obtained from the standard strain library. Fig. 15A shows the detection results of *Escherichia coli* strains carrying drug-resistance genes, indicating that the *blaOXA-48* drug-resistance gene was detected in the samples. Fig. 15B shows the detection results of *Stenotrophomonas maltophilia* carrying drug-resistance genes, indicating that the *SmeDEF* drug-resistance gene was detected in all 5 strain samples tested. Fig. 15C shows the detection results of *Klebsiella pneumoniae* carrying drug-resistance genes, indicating that the *SmeDEF* drug-resistance gene was detected in the sample. Fig. 15D shows the detection results of *Staphylococcus aureus* carrying drug-resistance genes, indicating that the *mecA* drug-resistance gene was detected in both strain samples tested. Fig. 15E shows the detection results of *VanA* and *blaCTX-M* genes carrying drug-resistance genes, with the *vanA* drug-resistance gene (FAM fluorescent label) detected in strains #130 and #148, and the *vanB* drug-resistance gene (HEX fluorescent label) detected in strain #135. All three strains could be detected by the *vanA+vanB* mixed primer-probe combination (ROX fluorescent labeling).

### Example 11: Detection of pathogen resistance genes in a sputum sample from a real ICU patient using the detection kit.

Four sputum samples of patients from the First Affiliated Hospital of Soochow University were tested by the method described in Example 1. The results show that the *blaNDM* drug-resistance gene was detected in three of the four samples (Fig. 16).

Only preferred embodiments of this application are described above, and it is not intended to limit the application in any other way. Any person skilled in the art may make changes or modifications to the above-disclosed technical content to create equivalent embodiments. However, any simple modifications, equivalent changes, and alterations made to the above embodiments based on the technical essence of this application, without departing from the scope of the technical solution of this application, shall still fall within the protection scope of the technical solution of this application.

## Claims

1. A combination for specifically detecting P target genes, wherein the combination comprises P pairs of primers for amplifying the P genes and P probes for hybridizing the P genes, the P probes are labeled with N concentrations of M different fluorescent groups, and the types and/or concentrations of fluorescent groups carried by each of the P probes are different;
the P target genes and the fluorescent group meet the following formula: 2≤P≤(M^{N}+M)-X, wherein
M is a positive integer greater than or equal to 2;
N ranges from 1 to 3; and
X ranges from 0 to 19.

2. The combination according to claim 1, wherein M is 2, 3, 4, 5, 6, 7 or 8, N is 1, 2 or 3, and X is 0, 1, 2, 3, 4, 5, 6, 7, 8, 9, 10, 11, 12, 13, 14, 15 or 16.

3. The combination according to claim 1, wherein the N concentrations are achieved by using any two different fluorescent groups in a specific ratio, and the ratio of any two different fluorescent groups A and B is in the range of A:B = 1:(0.20-10).

4. The combination according to any one of claims 1-3, wherein the target gene is a pathogen gene or a drug-resistance gene.

5. The combination according to claim 4, wherein the pathogen is one or more pathogens selected from the group consisting of: *Bacteroides fragilis*, *Staphylococcus epidermidis, Enterococcus faecium*, *Streptococcus pneumoniae, Acinetobacter baumannii, Enterobacter cloacae, Enterococcus faecalis*, *Staphylococcus aureus, Klebsiella pneumoniae, Pseudomonas aeruginosa, Escherichia coli, Staphylococcus capitis, Stenotrophomonas maltophilia, Serratia marcescens, Candida tropicalis, Cryptococcus neoformans*, *Candida krusei, Candida parapsilosis, Candida glabrata, Candida albicans,* human herpesvirus 1, human alpha herpesvirus 1, varicella-zoster virus, *Listeria monocytogenes, Mycobacterium tuberculosis,* human cytomegalovirus, human herpesvirus 5, human herpesvirus 2, human herpesvirus 6, Epstein-Barr virus, human herpesvirus 4, parvovirus B19, primate erythrocytic parvovirus 1, human herpesvirus 7, JC virus, JC polyomavirus, human polyomavirus type 2, pseudorabies virus, porcine herpesvirus type 1, pseudorabies virus (porcine herpesvirus *1), Neisseria meningitidis, Streptococcus agalactiae, Brucella, Brucella melitensis, Brucella melitensis, Haemophilus influenzae, Cryptococcus gattii, Cryptococcus gattii, Aspergillus fumigatus*, *Angiostrongylus cantonensis, Taenia solium, Toxoplasma gondii, Balamuthia mandrillaris,* adenovirus subgroup C type 1, adenovirus subgroup B type 4, human mammary gland Adenovirus B, human polyomavirus Type 1, Merkel cell polyomavirus, human polyomavirus 5, torque teno virus, *Streptococcus intermedius, Fusobacterium, Porphyromonas gingivalis, Neisseria meningitidis, Klebsiella oxytoca, Streptococcus suis, Campylobacter coli, Streptococcus constellatus, Proteus, Salmonella, Prevotella, Rhizopus oryzae, Mucor, Histoplasma capsulatum, Cryptococcus laurentii, Cryptococcus albidus, Aspergillus flavus, Treponema pallidum,* and *Orientia tsutsugamushi;* or
the drug-resistance gene is any one or more selected from the group consisting of: *mecA, mecC, vanA, vanB, MCR-1, blaKPC, blaCTX-M, blaOXA-23, blaNDM-1, blaTEM, blaSHV, blaVIM-1, blaVIM-2, blaIMP-1, blaIMP-4, SmeDEF, Sull, QnrA, QnrB, gyrA, gyrB*, *Aac(6')-Ib-cr, ermB, MefA, aac(3')-VI, aph(3')-VI, ant(2')-Ia, DfrA1, catA1, TetA, TetB,* and *cfr*.

6. The combination according to claim 5, wherein the primers and probes for the pathogen consist of two sets of primers and probes, the first set of primers and probes is: a combination of PCR primers and probes for specifically amplifying *Staphylococcus capitis,* wherein the primers comprise a forward primer represented by SEQ ID NO: 34 and a reverse primer represented by SEQ ID NO: 35, and the probes comprise a probe represented by SEQ ID NO: 36; a combination of PCR primers and probes for specifically amplifying *Acinetobacter baumannii,* wherein the primers comprise a forward primer represented by SEQ ID NO: 13 and a reverse primer represented by SEQ ID NO: 14, and the probes comprise a probe represented by SEQ ID NO: 15; a combination of PCR primers and probes for specifically amplifying *Candida tropicalis,* wherein the primers comprise a forward primer represented by SEQ ID NO: 46 and a reverse primer represented by SEQ ID NO: 47, and the probes comprise a probe represented by SEQ ID NO: 48; a combination of PCR primers and probes for specifically amplifying *Candida krusei,* wherein the primers comprise a forward primer represented by SEQ ID NO: 49 and a reverse primer represented by SEQ ID NO: 50, and the probes comprise a probe represented by SEQ ID NO: 51; a combination of PCR primers and probes for specifically amplifying *Staphylococcus epidermidis,* wherein the primers comprise a forward primer represented by SEQ ID NO: 4 and a reverse primer represented by SEQ ID NO: 5, and the probes comprise a probe represented by SEQ ID NO: 6; a combination of PCR primers and probes for specifically amplifying *Enterococcus faecium*, wherein the primers comprise a forward primer represented by SEQ ID NO: 19 and a reverse primer represented by SEQ ID NO: 20, and the probes comprise a probe represented by SEQ ID NO: 21; a combination of PCR primers and probes for specifically amplifying *Cryptococcus neoformans*, wherein the primers comprise a forward primer represented by SEQ ID NO: 61 and a reverse primer represented by SEQ ID NO: 62, and the probes comprise a probe represented by SEQ ID NO: 63; a combination of PCR primers and probes for specifically amplifying *Stenotrophomonas maltophilia,* wherein the primers comprise a forward primer represented by SEQ ID NO: 37 and a reverse primer represented by SEQ ID NO: 38, and the probes comprise a probe represented by NO:39; a combination of PCR primers and probes for specifically amplifying *Klebsiella pneumoniae,* wherein the primers comprise a forward primer represented by SEQ ID NO: 25 and a reverse primer represented by SEQ ID NO: 26, and the probes comprise a probe represented by SEQ ID NO: 27; a combination of PCR primers and probes for specifically amplifying *Streptococcus pneumoniae,* wherein the primers comprise a forward primer represented by SEQ ID NO: 10 and a reverse primer represented by SEQ ID NO: 11, and the probes comprise a probe represented by SEQ ID NO: 12; a combination of PCR primers and probes for specifically amplifying *Serratia marcescens,* wherein the primers comprise a forward primer represented by SEQ ID NO: 43 and a reverse primer represented by SEQ ID NO: 44, and the probes comprise a probe represented by SEQ ID NO: 45; and
the second set of primers and probes is: a combination of PCR primers and probes for specifically amplifying *Bacteroides fragilis*, wherein the primers comprise a forward primer represented by SEQ ID NO: 1 and a reverse primer represented by SEQ ID NO: 2, and the probes comprise a probe represented by SEQ ID NO: 3; a combination of PCR primers and probes for specifically amplifying *Enterococcus faecalis*, wherein the primers comprise a forward primer represented by SEQ ID NO: 7 and a reverse primer represented by SEQ ID NO: 8, and the probes comprise a probe represented by SEQ ID NO: 9; a combination of PCR primers and probes for specifically amplifying *Enterobacter cloacae,* wherein the primers comprise a forward primer represented by SEQ ID NO: 16 and a reverse primer represented by SEQ ID NO: 17, and the probes comprise a probe represented by SEQ ID NO: 18; a combination of PCR primers and probes for specifically amplifying *Staphylococcus aureus,* wherein the primers comprise a forward primer represented by SEQ ID NO: 22 and a reverse primer represented by SEQ ID NO: 23, and the probes comprise a probe represented by SEQ ID NO: 24; a combination of PCR primers and probes for specifically amplifying *Pseudomonas aeruginosa,* wherein the primers comprise a forward primer represented by SEQ ID NO: 28 and a reverse primer represented by SEQ ID NO: 29, and the probes comprise a probe represented by SEQ ID NO: 30; a combination of PCR primers and probes for specifically amplifying *Escherichia coli,* wherein the primers comprise a forward primer represented by SEQ ID NO: 31 and a reverse primer represented by SEQ ID NO: 32, and the probes comprise a probe represented by SEQ ID NO: 33; a combination of PCR primers and probes for specifically amplifying *Haemophilus influenzae,* wherein the primers comprise a forward primer represented by SEQ ID NO: 40 and a reverse primer represented by SEQ ID NO: 41, and the probes comprise a probe represented by SEQ ID NO: 42; a combination of PCR primers and probes for specifically amplifying *Candida parapsilosis,* wherein the primers comprise a forward primer represented by SEQ ID NO: 52 and a reverse primer represented by SEQ ID NO: 53, and the probes comprise a probe represented by SEQ ID NO: 54; a combination of PCR primers and probes for specifically amplifying *Candida glabrata,* wherein the primers comprise a forward primer represented by SEQ ID NO: 55 and a reverse primer represented by SEQ ID NO: 56, and the probes comprise a probe represented by SEQ ID NO: 57; a combination of PCR primers and probes for specifically amplifying *Candida albicans,* wherein the primers comprise a forward primer represented by SEQ ID NO: 58 and a reverse primer represented by SEQ ID NO: 59, and the probes comprise a probe represented by SEQ ID NO: 60.

7. The combination according to claim 5, wherein the primers and probes for the drug-resistance gene comprise any one, two, or three selected from the following three sets of primers and probes, wherein
the first set of primers and probes is: a combination of PCR primers and probes for specifically amplifying *mecA,* wherein the primers comprise an upstream primer represented by SEQ ID NO: 67 and a downstream primer represented by SEQ ID NO: 68, and the probes comprise a probe represented by SEQ ID NO: 69; a combination of PCR primers and probes for specifically amplifying *vanA,* wherein the primers comprise an upstream primer represented by SEQ ID NO: 73 and a downstream primer represented by SEQ ID NO: 74, and the probes comprise a probe represented by SEQ ID NO: 75; a combination of PCR primers and probes for specifically amplifying *MCR-1*, wherein the primers comprise an upstream primer represented by SEQ ID NO: 79 and a downstream primer represented by SEQ ID NO: 80, and the probes comprise a probe represented by SEQ ID NO: 81; a combination of PCR primers and probes for specifically amplifying *blaKPC,* wherein the primers comprise an upstream primer represented by SEQ ID NO: 82 and a downstream primer represented by SEQ ID NO: 83, and the probes comprise a probe represented by SEQ ID NO: 84; a combination of PCR primers and probes for specifically amplifying *blaNDM-1*, wherein the primers comprise an upstream primer represented by SEQ ID NO: 91 and a downstream primer represented by SEQ ID NO: 92, and the probes comprise a probe represented by SEQ ID NO: 93; a combination of PCR primers and probes for specifically amplifying *blaIMP-1*, wherein the primers comprise an upstream primer represented by SEQ ID NO: 106 and a downstream primer represented by SEQ ID NO: 107, and the probes comprise a probe represented by SEQ ID NO: 108; a combination of PCR primers and probes for specifically amplifying *SmeDEF,* wherein the primers comprise an upstream primer represented by SEQ ID NO: 112 and a downstream primer represented by SEQ ID NO: 113, and the probes comprise a probe represented by SEQ ID NO: 114; a combination of PCR primers and probes for specifically amplifying *Sull,* wherein the primers comprise an upstream primer represented by SEQ ID NO: 115 and a downstream primer represented by SEQ ID NO: 116, and the probes comprise a probe represented by SEQ ID NO: 117; a combination of PCR primers and probes for specifically amplifying *OnrB*, wherein the primers comprise an upstream primer represented by SEQ ID NO: 121 and a downstream primer represented by SEQ ID NO: 122, and the probes comprise a probe represented by SEQ ID NO: 123; a combination of PCR primers and probes for specifically amplifying *gyrB,* wherein the primers comprise an upstream primer represented by SEQ ID NO: 124 and a downstream primer represented by SEQ ID NO: 125, and the probes comprise a probe represented by SEQ ID NO: 126; a combination of PCR primers and probes for specifically amplifying *Aac(6)-Ib-cr,* wherein the primers comprise an upstream primer represented by SEQ ID NO: 130 and a downstream primer represented by SEQ ID NO: 131, and the probes comprise a probe represented by SEQ ID NO: 132;
the second set of primers and probes is: a combination of PCR primers and probes for specifically amplifying *mecC,* wherein the primers comprise an upstream primer represented by SEQ ID NO: 70 and a downstream primer represented by SEQ ID NO: 71, and the probes comprise a probe represented by SEQ ID NO: 72; a combination of PCR primers and probes for specifically amplifying *blaCTX-M*, wherein the primers comprise an upstream primer represented by SEQ ID NO: 85 and a downstream primer represented by SEQ ID NO: 86, and the probes comprise a probe represented by SEQ ID NO: 87; a combination of PCR primers and probes for specifically amplifying *blaTEM,* wherein the primers comprise an upstream primer represented by SEQ ID NO: 94 and a downstream primer represented by SEQ ID NO: 95, and the probes comprise a probe represented by SEQ ID NO: 96; and a combination of PCR primers and probes for specifically amplifying *blaVIM-2,* wherein the primers comprise an upstream primer represented by SEQ ID NO: 103 and a downstream primer represented by SEQ ID NO: 104, and the probes comprise a probe represented by SEQ ID NO: 105; a combination of PCR primers and probes for specifically amplifying *blaVIM-1*, wherein the primers comprise an upstream primer represented by SEQ ID NO: 100 and a downstream primer represented by SEQ ID NO: 101, and the probes comprise a probe represented by SEQ ID NO: 102; a combination of PCR primers and probes for specifically amplifying *gyrA,* wherein the primers comprise an upstream primer represented by SEQ ID NO: 127 and a downstream primer represented by SEQ ID NO: 128, and the probes comprise a probe represented by SEQ ID NO: 129; a combination of PCR primers and probes for specifically amplifying *ermB,* wherein the primers comprise an upstream primer represented by SEQ ID NO: 133 and a downstream primer represented by SEQ ID NO: 134, and the probes comprise a probe represented by SEQ ID NO: 135; a combination of PCR primers and probes for specifically amplifying *aac(3')-VI*, wherein the primers comprise an upstream primer represented by SEQ ID NO: 142 and a downstream primer represented by SEQ ID NO: 143, and the probes comprise a probe represented by SEQ ID NO: 144; a combination of PCR primers and probes for specifically amplifying *cfr*, wherein the primers comprise an upstream primer represented by SEQ ID NO: 139 and a downstream primer represented by SEQ ID NO: 140, and the probes comprise a probe represented by SEQ ID NO: 141; a combination of PCR primers and probes for specifically amplifying *catA1,* wherein the primers comprise an upstream primer represented by SEQ ID NO: 154 and a downstream primer represented by SEQ ID NO: 155, and the probes comprise a probe represented by SEQ ID NO: 156; a combination of PCR primers and probes for specifically amplifying *TetB,* wherein the primers comprise an upstream primer represented by SEQ ID NO: 160 and a downstream primer represented by SEQ ID NO: 161, and the probes comprise a probe represented by SEQ ID NO: 162; and
the third set of primers and probes is: a combination of PCR primers and probes for specifically amplifying *vanB,* wherein the primers comprise an upstream primer represented by SEQ ID NO: 76 and a downstream primer represented by SEQ ID NO: 77, and the probes comprise a probe represented by SEQ ID NO: 78; a combination of PCR primers and probes for specifically amplifying *blaOXA-23,* wherein the primers comprise an upstream primer represented by SEQ ID NO: 88 and a downstream primer represented by SEQ ID NO: 89, and the probes comprise a probe represented by SEQ ID NO: 90; a combination of PCR primers and probes for specifically amplifying *blaSHV,* wherein the primers comprise an upstream primer represented by SEQ ID NO: 97 and a downstream primer represented by SEQ ID NO: 98, and the probes comprise a probe represented by SEQ ID NO: 99; and a combination of PCR primers and probes for specifically amplifying *blaIMP-4*, wherein the primers comprise an upstream primer represented by SEQ ID NO: 109 and a downstream primer represented by SEQ ID NO: 110, and the probes comprise a probe represented by SEQ ID NO: 111; a combination of PCR primers and probes for specifically amplifying *OnrA*, wherein the primers comprise an upstream primer represented by SEQ ID NO: 118 and a downstream primer represented by SEQ ID NO: 119, and the probes comprise a probe represented by SEQ ID NO: 120; a combination of PCR primers and probes for specifically amplifying *TetA,* wherein the primers comprise an upstream primer represented by SEQ ID NO: 157 and a downstream primer represented by SEQ ID NO: 158, and the probes comprise a probe represented by SEQ ID NO: 159; a combination of PCR primers and probes for specifically amplifying *aph(3')-VI*, wherein the primers comprise an upstream primer represented by SEQ ID NO: 145 and a downstream primer represented by SEQ ID NO: 146, and the probes comprise a probe represented by SEQ ID NO: 147; a combination of PCR primers and probes for specifically amplifying *MefA*, wherein the primers comprise an upstream primer represented by SEQ ID NO: 136 and a downstream primer represented by SEQ ID NO: 137, and the probes comprise a probe represented by SEQ ID NO: 138; a combination of PCR primers and probes for specifically amplifying *ant(2")-Ia*, wherein the primers comprise an upstream primer represented by SEQ ID NO: 148 and a downstream primer represented by SEQ ID NO: 149, and the probe comprises a probe represented by SEQ ID NO: 150; a combination of PCR primers and probes for specifically amplifying *DfrA1*, wherein the primers comprise an upstream primer represented by SEQ ID NO: 151 and a downstream primer represented by SEQ ID NO: 152, and the probe comprises a probe represented by SEQ ID NO: 153; a combination of PCR primers and probes for specifically amplifying *B2M,* wherein the primers comprise an upstream primer represented by SEQ ID NO: 163 and a downstream primer represented by SEQ ID NO: 164, and the probe comprises a probe represented by SEQ ID NO: 165.

8. The combination of primers and probes for PCR according to claim 6 or 7, wherein the 5' end of the sequence of the probe is labeled with a fluorescent reporter group and the 3' end of the sequence of the probe is labeled with a fluorescence quenching group; when the probes are in the same reaction system, the fluorescent reporter groups for screening different types of pathogens or drug-resistance genes have different spectral ranges.

9. The combination of primers and probes for PCR according to claim 8, wherein the fluorescent reporter group is selected from FAM, VIC, ROX, Cy5 and/or HEX; and the fluorescence quenching group is selected from BHQ1, BHQ2, BHQ3 and/or Eclipse.

10. The combination of primers and probes for PCR according to claim 6, wherein the probes used and the fluorescent reporter groups labeled at the 5' end and the fluorescence quenching groups labeled at the 3' end are as follows:
the probe for screening *Bacteroides fragilis* is represented by SEQ ID NO: 3, with the 5' end labeled with fluorescent FAM and the 3' end labeled with fluorescent BHQ1; the probe for screening *Staphylococcus epidermidis* is represented by SEQ ID NO: 6, with the 5' end labeled with fluorescent HEX and the 3' end labeled with fluorescent BHQ1; the probe for screening *Enterococcus faecalis* is represented by SEQ ID NO: 9, with the 5' end labeled with fluorescent ROX and the 3' end labeled with fluorescent BHQ2; the probe for screening *Streptococcus pneumoniae* is represented by SEQ ID NO: 12, with the 5' end labeled with fluorescent Cy5 and the 3' end labeled with fluorescent BHQ2; the probe for screening *Acinetobacter baumannii* is represented by SEQ ID NO: 15, with the 5' end labeled with fluorescent FAM/HEX and the 3' end labeled with fluorescent BHQ1/BHQ2; the probe for screening *Enterobacter cloacae* is represented by SEQ ID NO: 18, with the 5' end labeled with fluorescent HEX and the 3' end labeled with fluorescent BHQ1; the probe for screening *Enterococcus faecium* is represented by SEQ ID NO: 21, with the 5' end labeled with fluorescent ROX and the 3' end labeled with fluorescent BHQ2; the probe for screening *Staphylococcus aureus* is represented by SEQ ID NO: 24, with the 5' end labeled with fluorescent ROX/Cy5 and the 3' end labeled with fluorescent BHQ2; the probe for screening *Klebsiella pneumoniae* is represented by SEQ ID NO: 27, with the 5' end labeled with fluorescent ROX/Cy5 and the 3' end labeled with fluorescent BHQ2; the probe for screening *Pseudomonas aeruginosa* is represented by SEQ ID NO: 30, with the 5' end labeled with fluorescent ROX/Cy5 and the 3' end labeled with fluorescent BHQ2; the probe for screening *Escherichia coli* is represented by SEQ ID NO: 33, with the 5' end labeled with fluorescent FAM/HEX and the 3' end labeled with fluorescent BHQ1; the probe for screening *Staphylococcus capitis* is represented by SEQ ID NO: 36, with the 5' end labeled with fluorescent FAM and the 3' end labeled with fluorescent BHQ1; the probe for screening *Stenotrophomonas maltophilia* is represented by SEQ ID NO: 39, with the 5' end labeled with fluorescent ROX/Cy5 and the 3' end labeled with fluorescent BHQ2; the probe for screening *Haemophilus influenzae* is represented by SEQ ID NO: 42, with the 5' end labeled with fluorescent ROX/Cy5 and the 3' end labeled with fluorescent BHQ2; the probe for screening *Serratia marcescens* is represented by SEQ ID NO: 45, with the 5' end labeled with fluorescent Cy5.5 and the 3' end labeled with fluorescent BHQ3; the probe for screening *Candida tropicalis* is represented by SEQ ID NO: 48, with the 5' end labeled with fluorescent FAM/HEX and the 3' end labeled with fluorescent BHQ1; the probe for screening *Candida krusei* is represented by SEQ ID NO: 51, with the 5' end labeled with fluorescent FAM/HEX and the 3' end labeled with fluorescent BHQ1; the probe for screening *Candida parapsilosis* is represented by SEQ ID NO: 54, with the 5' end labeled with fluorescent FAM/HEX and the 3' end labeled with fluorescent BHQ1; the probe for screening *Candida glabrata* is represented by SEQ ID NO:57, with the 5' end labeled with fluorescent FAM/HEX and the 3' end labeled with fluorescent BHQ1; the probe for screening *Candida albicans* is represented by SEQ ID NO: 60, with the 5' end labeled with fluorescent ROX/Cy5 and the 3' end labeled with fluorescent BHQ2; and the probe for screening *Cryptococcus neoformans* is represented by SEQ ID NO: 63, with the 5' end labeled with fluorescent ROX/Cy5 and the 3' end labeled with fluorescent BHQ2.

11. The combination of primers and probes for PCR according to claim7, wherein the probes used and the fluorescent reporter groups labeled at the 5' end and the fluorescence quenching groups labeled at the 3' end are as follows:
the probe for screening the *mecA* gene is represented by SEQ ID NO: 69, with the 5' end labeled with fluorescent FAM and the 3' end labeled with fluorescent BHQ1; the probe for screening the *mecC* gene is represented by SEQ ID NO: 72, with the 5' end labeled with fluorescent FAM and the 3' end labeled with fluorescent BHQ1; the probe for screening the *vanA* gene is represented by SEQ ID NO: 75, with the 5' end labeled with fluorescent FAM/HEX and the 3' end labeled with fluorescent BHQ1; the probe for screening the *vanB* gene is represented by SEQ ID NO: 78, with the 5' end labeled with fluorescent FAM and the 3' end labeled with fluorescent BHQ1; the probe for screening the *MCR-1* gene is represented by SEQ ID NO: 81, with the 5' end labeled with fluorescent FAM/HEX and the 3' end labeled with fluorescent BHQ1; the probe for screening the *blaKPC* gene is represented by SEQ ID NO: 84, with the 5' end labeled with fluorescent FAM/HEX and the 3' end labeled with fluorescent BHQ1; the probe for screening the *blaCTX-M* gene is represented by SEQ ID NO: 87, with the 5' end labeled with fluorescent FAM/HEX and the 3' end labeled with fluorescent BHQ1; the probe for screening the *blaOXA-23* gene is represented by SEQ ID NO: 24, with the 5' end labeled with fluorescent FAM/HEX and the 3' end labeled with fluorescent BHQ1; the probe for screening the *blaNDM-1* gene is represented by SEQ ID NO: 93, with the 5' end labeled with fluorescent HEX and the 3' end labeled with fluorescent BHQ1; the probe for screening the *blaTEM* gene is represented by SEQ ID NO: 96, with the 5' end labeled with fluorescent FAM/HEX and the 3' end labeled with fluorescent BHQ1; the probe for screening the *blaSHV* gene is represented by SEQ ID NO: 99, with the 5' end labeled with fluorescent FAM/HEX and the 3' end labeled with fluorescent BHQ1; the probe for screening the *blaVIM-1* gene is represented by SEQ ID NO: 102, with the 5' end labeled with fluorescent HEX and the 3' end labeled with fluorescent BHQ1; the probe for screening the *blaVIM-2* gene is represented by SEQ ID NO: 105, with the 5' end labeled with fluorescent FAM/HEX and the 3' end labeled with fluorescent BHQ1; the probe for screening the *blaIMP-1* gene is represented by SEQ ID NO: 108, with the 5' end labeled with fluorescent ROX and the 3' end labeled with fluorescent BHQ2; the probe for screening the *blaIMP-4* gene is represented by SEQ ID NO: 111, with the 5' end labeled with fluorescent FAM/HEX and the 3' end labeled with fluorescent BHQ1; the probe for screening the *SmeDEF* gene is represented by SEQ ID NO: 114, with the 5' end labeled with fluorescent ROX/Cy5 and the 3' end labeled with fluorescent BHQ2; the probe for screening the *Sull* gene is represented by SEQ ID NO: 117, with the 5' end labeled with fluorescent ROX/Cy5 and the 3' end labeled with fluorescent BHQ2; the probe for screening the *QnrA* gene is represented by SEQ ID NO: 120, with the 5' end labeled with fluorescent CY5 and the 3' end labeled with fluorescent BHQ2; the probe for screening the *OnrB* gene is represented by SEQ ID NO: 123, with the 5' end labeled with fluorescent ROX/CY5 and the 3' end labeled with fluorescent BHQ2; the probe for screening the *gyrB* gene is represented by SEQ ID NO: 126, with the 5' end labeled with fluorescent Cy5 and the 3' end labeled with fluorescent BHQ2; the probe for screening the *gyrA* gene is represented by SEQ ID NO: 129, with the 5' end labeled with fluorescent ROX and the 3' end labeled with fluorescent BHQ2; the probe for screening the *Aac(6')-Ib-cr* gene is represented by SEQ ID NO: 132, with the 5' end labeled with fluorescent Cy5.5 and the 3' end labeled with fluorescent BHQ3; the probe for screening the *ermB* gene is represented by SEQ ID NO: 135, with the 5' end labeled with fluorescent ROX/Cy5 and the 3' end labeled with fluorescent BHQ2; the probe for screening the *MefA* gene is represented by SEQ ID NO: 138, with the 5' end labeled with fluorescent Cy5 and the 3' end labeled with fluorescent BHQ2; the probe for screening the *cfr* gene is represented by SEQ ID NO: 141, with the 5' end labeled with fluorescent ROX/Cy5 and the 3' end labeled with fluorescent BHQ2; the probe for screening the *aac(3')-VI* gene is represented by SEQ ID NO: 144, with the 5' end labeled with fluorescent ROX/Cy5 and the 3' end labeled with fluorescent BHQ2; the probe for screening the *aph(3')-VI* gene is represented by SEQ ID NO: 147, with the 5' end labeled with fluorescent ROX/Cy5 and the 3' end labeled with fluorescent BHQ2; the probe for screening the *ant(2")-Ia gene* is represented by SEQ ID NO: 150, with the 5' end labeled with fluorescent ROX/Cy5 and the 3' end labeled with fluorescent BHQ2; the probe for screening the *DfrA1* gene is represented by SEQ ID NO: 153, with the 5' end labeled with fluorescent ROX/Cy5 and the 3' end labeled with fluorescent BHQ2; the probe for screening the *catA1* gene is represented by SEQ ID NO: 156, with the 5' end labeled with fluorescent Cy5 and the 3' end labeled with fluorescent BHQ2; the probe for screening the *TetA* gene is represented by SEQ ID NO: 159, with the 5' end labeled with fluorescent ROX and the 3' end labeled with fluorescent BHQ2; the probe for screening the *TetB* gene is represented by SEQ ID NO: 162, with the 5' end labeled with fluorescent Cy5.5 and the 3' end labeled with fluorescent BHQ3; and the probe for screening the *B2M* gene is represented by SEQ ID NO: 165, with the 5' end labeled with fluorescent Cy5.5 and the 3' end labeled with fluorescent BHQ3.

12. The combination according to any one of claims 1-11, which is a combination for digital PCR (dPCR) reaction.

13. A digital PCR reaction premix for specifically detecting a target gene, wherein the digital PCR reaction premix comprises a combination according to any one of claims 1-12.

14. The digital PCR reaction premix according to claim 13, wherein the digital PCR reaction premix further comprises one, two, three, four, five, six, seven or eight components selected from the group consisting of: hot-start Taq enzyme, UDG enzyme, dNTPs, bovine serum albumin, glycerol, betaine, NH₄⁺, and Mg²⁺.

15. A digital PCR microfluidic cartridge, wherein the digital PCR microfluidic cartridge comprises the digital PCR reaction premix according to claim 13 or 14.

16. The microfluidic cartridge according to claim 15, wherein the microfluidic cartridge comprises at least two wells.

17. A kit for detecting a target gene, wherein the kit comprises the digital PCR (dPCR) reaction premix according to claim 13 or 14.

18. The kit according to claim 17, wherein the kit further comprises a positive quality control, a negative quality control, and/or a microdroplet-generating oil.

19. The kit according to claim 18, wherein the kit further comprises the digital PCR microfluidic cartridge according to claim 15.

20. A method for detecting a target gene for non-disease diagnosis purpose, the method comprises the following steps:
(1) releasing the nucleic acids from the sample to be tested;
(2) preparing a digital PCR amplification mixture, wherein the mixture comprises: the nucleic acids of the sample to be tested in step (1), the combination according to any one of claims 1-12, and/or the digital PCR premix according to claim 13 or 14;
(3) preparing microdroplets in a digital PCR microfluidic cartridge and performing PCR amplification reaction;
(4) reading the fluorescence signal after the microdroplet PCR amplification reaction by a digital PCR microfluidic cartridge.

21. The method for detecting a target gene for non-disease diagnosis purpose according to claim 20, wherein the sample to be tested is selected from: whole blood, serum, plasma, throat swab, sputum, urine, feces, cerebrospinal fluid, body fluid, or ascites.
